# EUROPEAN PATENT SPECIFICATION

(11) **EP 3 735 985 B1**
(45) Date of publication and mention of the grant of the patent: **25.08.2021**
(21) Application number: 19305604.1
(22) Date of filing: 10.05.2019
(51) Int. Cl.: A61K 39/12, C12Q 1/70

(54) **A NOVEL ORTHOBUNYAVIRUS IN HUMAN ENCEPHALITIS AND ITS DIAGNOSTIC AND THERAPEUTIC APPLICATIONS**
NEUARTIGER ORTHOBUNYAVIRUS IN DER HUMANEN ENCEPHALITIS UND DESSEN DIAGNOSTISCHE UND THERAPEUTISCHE ANWENDUNGEN
NOUVEL ORTHOBUNYAVIRUS DANS L'ENCÉPHALITE HUMAIN ET SES APPLICATIONS DIAGNOSTIQUES ET THÉRAPEUTIQUES

(43) Date of publication of application: 11.11.2020
(73) Proprietor: Institut Pasteur, 75724 Paris Cédex 15 (FR)
(72) Inventor: ELOIT, Marc, 75006 Paris (FR); PEROT, Philippe, 75014 Paris (FR); SEILHEAN, Danielle, 75019 Paris (FR); DUYCKAERTS, Charles, 94160 Saint Mande (FR); CHRETIEN, Delphine, 93200 Saint Denis (FR)
(74) Representative: Plasseraud IP

(56) References cited:
- US-A1- 2013 323 210
- YADAV PRAGYA D ET AL: "A mini-review of Bunyaviruses recorded in India", INDIAN JOURNAL OF MEDICAL RESEARCH, vol. 145, May 2017 (2017-05), pages 600-609, XP002795364,
- YADAV PRAGYA D ET AL: "Molecular characterization of Umbre virus (Bunyaviridae)", VIROLOGY JOURNAL, BIOMED CENTRAL, LONDON, GB, vol. 5, no. 1, 8 October 2008 (2008-10-08) , page 115, XP021045015, ISSN: 1743-422X
- JAN HELLERT ET AL: "Orthobunyavirus spike architecture and recognition by neutralizing antibodies", NATURE COMMUNICATIONS, vol. 10, no. 1, 20 February 2019 (2019-02-20), XP055637697, DOI: 10.1038/s41467-019-08832-8
- SUKHRALIA SHIVANI ET AL: "From dengue to Zika: the wide spread of mosquito-borne arboviruses", EUROPEAN JOURNAL OF CLINICAL MICROBIOLOGY & INFECTIOUS DISEASES, SPRINGER, WIESBADEN, DE, vol. 38, no. 1, 28 September 2018 (2018-09-28), pages 3-14, XP036664759, ISSN: 0934-9723, DOI: 10.1007/S10096-018-3375-7 [retrieved on 2018-09-28]

### SEQUENCE LISTING

<110> INSTITUT PASTEUR
<120> IDENTIFICATION OF A NOVEL ORTHOBUNYAVIRUS IN HUMAN ENCEPHALITIS AND ITS DIAGNOSTIC AND THERAPEUTIC APPLICATIONS
<130> BEP190068 EP
<160> 89
<170> PatentIn version 3.5
<210> 1
   <211> 772
   <212> DNA
   <213> Moissiacense virus
<220>
   <221> CDS
   <222> (59)..(772)
<400> 1
   aatcattata tctaaatttg ctaaacgcag aactgggtag cactagcatt tatccagg        58
   atg gct gat tct aag ctc aca tac gag gat aca cag gat gtt agt aga       106
   Met Ala Asp Ser Lys Leu Thr Tyr Glu Asp Thr Gln Asp Val Ser Arg
   1               5                   10                  15
   agt act tat aac cca gca gat gaa tac gat acg ttt gtg ggt gtc tac       154
   Ser Thr Tyr Asn Pro Ala Asp Glu Tyr Asp Thr Phe Val Gly Val Tyr
               20                  25                  30
   aga gaa ttt ctc aca att gat aac att aga atc ttc ttc atc aac gct       202
   Arg Glu Phe Leu Thr Ile Asp Asn Ile Arg Ile Phe Phe Ile Asn Ala
           35                  40                  45
   cca aag gcc aaa act gcg atg gct aaa ctc aag acc gag aca gtt gaa       250
   Pro Lys Ala Lys Thr Ala Met Ala Lys Leu Lys Thr Glu Thr Val Glu
       50                  55                  60
   gtt aca ttt gga aca ctc gtt gtc aag ctg gtt aat aac cat aga att       298
   Val Thr Phe Gly Thr Leu Val Val Lys Leu Val Asn Asn His Arg Ile
   65                  70                  75                  80
   gga cag gct cag cgt gag gtt gcg gac gat gag cta act ctt cac cga       346
   Gly Gln Ala Gln Arg Glu Val Ala Asp Asp Glu Leu Thr Leu His Arg
                   85                  90                  95
   ctt tct ggc tac att gca aag tac act ctt cac cta tac aaa aat gca       394
   Leu Ser Gly Tyr Ile Ala Lys Tyr Thr Leu His Leu Tyr Lys Asn Ala
               100                 105                 110
   aaa aaa gca gga gag aga gaa gca ata ata acc aag atc gtt aat cca       442
   Lys Lys Ala Gly Glu Arg Glu Ala Ile Ile Thr Lys Ile Val Asn Pro
           115                 120                 125
   att tct gcc aaa atg ggc ttc caa tgg agt gtt ggg ccg gaa att tat       490
   Ile Ser Ala Lys Met Gly Phe Gln Trp Ser Val Gly Pro Glu Ile Tyr
       130                 135                 140
   ctc tca acg ctg cca gga acc gaa atg ttt cta gat aca ttt aaa atg       538
   Leu Ser Thr Leu Pro Gly Thr Glu Met Phe Leu Asp Thr Phe Lys Met
   145                 150                 155                 160
   ttc cca ttg gca ttc atc ttg tta aga gtt aaa aga gga gag atc aag        586
   Phe Pro Leu Ala Phe Ile Leu Leu Arg Val Lys Arg Gly Glu Ile Lys
                   165                 170                 175
   gtg gaa atg gca aaa aaa gca atg cgg cag aag tat ggc gac aaa gat        634
   Val Glu Met Ala Lys Lys Ala Met Arg Gln Lys Tyr Gly Asp Lys Asp
               180                 185                 190
   gcc agt gaa tgg ctg cag gag gac att gag act gta aaa ggg gca ata        682
   Ala Ser Glu Trp Leu Gln Glu Asp Ile Glu Thr Val Lys Gly Ala Ile
           195                 200                 205
   aaa aca gtt gaa aag ctc aag cca acg ctg aca gga ttg gct gct tca        730
   Lys Thr Val Glu Lys Leu Lys Pro Thr Leu Thr Gly Leu Ala Ala Ser
       210                 215                 220
   atg act gct ttc ctg caa caa ctt ggt atc aac act aag taa                772
   Met Thr Ala Phe Leu Gln Gln Leu Gly Ile Asn Thr Lys
   225                 230                 235
<210> 2
   <211> 237
   <212> PRT
   <213> Moissiacense virus
<400> 2
   Met Ala Asp Ser Lys Leu Thr Tyr Glu Asp Thr Gln Asp Val Ser Arg
   1               5                   10                  15
   Ser Thr Tyr Asn Pro Ala Asp Glu Tyr Asp Thr Phe Val Gly Val Tyr
               20                  25                  30
   Arg Glu Phe Leu Thr Ile Asp Asn Ile Arg Ile Phe Phe Ile Asn Ala
           35                  40                  45
   Pro Lys Ala Lys Thr Ala Met Ala Lys Leu Lys Thr Glu Thr Val Glu
       50                  55                  60
   Val Thr Phe Gly Thr Leu Val Val Lys Leu Val Asn Asn His Arg Ile
   65                  70                  75                  80
   Gly Gln Ala Gln Arg Glu Val Ala Asp Asp Glu Leu Thr Leu His Arg
                   85                  90                  95
   Leu Ser Gly Tyr Ile Ala Lys Tyr Thr Leu His Leu Tyr Lys Asn Ala
               100                 105                 110
   Lys Lys Ala Gly Glu Arg Glu Ala Ile Ile Thr Lys Ile Val Asn Pro
           115                 120                 125
   Ile Ser Ala Lys Met Gly Phe Gln Trp Ser Val Gly Pro Glu Ile Tyr
       130                 135                 140
   Leu Ser Thr Leu Pro Gly Thr Glu Met Phe Leu Asp Thr Phe Lys Met
   145                 150                 155                 160
   Phe Pro Leu Ala Phe Ile Leu Leu Arg Val Lys Arg Gly Glu Ile Lys
                   165                 170                 175
   Val Glu Met Ala Lys Lys Ala Met Arg Gln Lys Tyr Gly Asp Lys Asp
               180                 185                 190
   Ala Ser Glu Trp Leu Gln Glu Asp Ile Glu Thr Val Lys Gly Ala Ile
           195                 200                 205
   Lys Thr Val Glu Lys Leu Lys Pro Thr Leu Thr Gly Leu Ala Ala Ser
       210                 215                 220
   Met Thr Ala Phe Leu Gln Gln Leu Gly Ile Asn Thr Lys
   225                 230                 235
<210> 3
   <211> 4395
   <212> DNA
   <213> Moissiacense virus
<220>
   <221> CDS
   <222> (1)..(4395)
<220>
   <221> variation
   <222> (4114)..(4116)
   <223> aww is att or aaa
<400> 3
   atg gtg atc ttt gca ttg ttg tcg ata cta tta gtt ggc ttc agc aat        48
   Met Val Ile Phe Ala Leu Leu Ser Ile Leu Leu Val Gly Phe Ser Asn
   1               5                   10                  15
   caa gcg act att cga tgt ttc tct ggc ggc tcc aaa atc atg gaa aaa        96
   Gln Ala Thr Ile Arg Cys Phe Ser Gly Gly Ser Lys Ile Met Glu Lys
               20                  25                  30
   aca tcg aat gtc tca ttg agt gaa cta tgc tta aag gat gat gtc agc       144
   Thr Ser Asn Val Ser Leu Ser Glu Leu Cys Leu Lys Asp Asp Val Ser
           35                  40                  45
   cag cta aaa att ata gtt gac cac aca aaa aat gat tca gga tta ttt       192
   Gln Leu Lys Ile Ile Val Asp His Thr Lys Asn Asp Ser Gly Leu Phe
       50                  55                  60
   tca act gcc agc gtc tgg aga aaa tgg ata gtc tca gat tgg acc aca       240
   Ser Thr Ala Ser Val Trp Arg Lys Trp Ile Val Ser Asp Trp Thr Thr
   65                  70                  75                  80
   tgt aac cct aaa aaa atg gct tta gga aca ata tct gtt ata gat atc         288
   Cys Asn Pro Lys Lys Met Ala Leu Gly Thr Ile Ser Val Ile Asp Ile
                   85                  90                  95
   gat aaa aac ttt atc tta aaa agc tcc agc tat ata tgc aat aaa gag         336
   Asp Lys Asn Phe Ile Leu Lys Ser Ser Ser Tyr Ile Cys Asn Lys Glu
               100                 105                 110
   tgc acg ata aca gtg gat aaa gaa aga gcc caa gtt ctg cta caa tca         384
   Cys Thr Ile Thr Val Asp Lys Glu Arg Ala Gln Val Leu Leu Gln Ser
           115                 120                 125
   gat ggc tta aat cac ttt gaa gtt tct gga acc act ttg agt tct gga         432
   Asp Gly Leu Asn His Phe Glu Val Ser Gly Thr Thr Leu Ser Ser Gly
       130                 135                 140
   tgg ttt aaa tcg aca gcc act gtt acc tta gat caa acc tgc gag cat         480
   Trp Phe Lys Ser Thr Ala Thr Val Thr Leu Asp Gln Thr Cys Glu His
   145                 150                 155                 160
   gtt aaa gtg tct tgt ggc cag aaa tca atg caa ttc cat gcc tgc ttc         528
   Val Lys Val Ser Cys Gly Gln Lys Ser Met Gln Phe His Ala Cys Phe
                   165                 170                 175
   aag aat cat atg tca tgc att aga ttc ttg cat aga agt ata atg cca         576
   Lys Asn His Met Ser Cys Ile Arg Phe Leu His Arg Ser Ile Met Pro
               180                 185                 190
   gca agc ttt gca gtt tct atc tgc cag aat ata gag ttg ata tta atg         624
   Ala Ser Phe Ala Val Ser Ile Cys Gln Asn Ile Glu Leu Ile Leu Met
           195                 200                 205
   aca acg ttt aca ttg att ata ttc ttg cta cta agc att tta aca aaa         672
   Thr Thr Phe Thr Leu Ile Ile Phe Leu Leu Leu Ser Ile Leu Thr Lys
       210                 215                 220
   aca tac ata tgc tat att ttg cta ccc ata ttc ata ccc ttt gcg tac         720
   Thr Tyr Ile Cys Tyr Ile Leu Leu Pro Ile Phe Ile Pro Phe Ala Tyr
   225                 230                 235                 240
   gca tat gga ttt gtt tat gat aaa aca tgc aag aag tgc tca tta tgt         768
   Ala Tyr Gly Phe Val Tyr Asp Lys Thr Cys Lys Lys Cys Ser Leu Cys
                   245                 250                 255
   gga tta gct tac cac cct ttc aca aag tgt ggg ata cat tgt gtt tgt         816
   Gly Leu Ala Tyr His Pro Phe Thr Lys Cys Gly Ile His Cys Val Cys
               260                 265                 270
   gga tgt aga ttt gaa aat tca gat gca atg aaa acc cac aga gaa aaa         864
   Gly Cys Arg Phe Glu Asn Ser Asp Ala Met Lys Thr His Arg Glu Lys
           275                 280                 285
   ggg aaa tgt ccg gga ttc aag tca atg aga att gct agg gta ctg tgt         912
   Gly Lys Cys Pro Gly Phe Lys Ser Met Arg Ile Ala Arg Val Leu Cys
       290                 295                 300
   aag gct aaa ggt tca gct ttc gct tta tct gta cta ttg gcc acg ttg         960
   Lys Ala Lys Gly Ser Ala Phe Ala Leu Ser Val Leu Leu Ala Thr Leu
   305                 310                 315                 320
   gca ttg ggc ttc ttg acg cct gta caa ggg cta gtt cta gat aag acc        1008
   Ala Leu Gly Phe Leu Thr Pro Val Gln Gly Leu Val Leu Asp Lys Thr
                   325                 330                 335
   caa att aac tac aca gaa gat caa tta cca gaa tca ttt cag acg atg      1056
   Gln Ile Asn Tyr Thr Glu Asp Gln Leu Pro Glu Ser Phe Gln Thr Met
               340                 345                 350
   caa tca aca ata tct ata ttc aaa ttt gca aca gtc atg aat gca ggt      1104
   Gln Ser Thr Ile Ser Ile Phe Lys Phe Ala Thr Val Met Asn Ala Gly
           355                 360                 365
   tgg act tac tgc atg ttc ctt gtt aca ttg gct tta tat atc ata cac      1152
   Trp Thr Tyr Cys Met Phe Leu Val Thr Leu Ala Leu Tyr Ile Ile His
       370                 375                 380
   aag aag tat gct cac aga tta ctg agc tat tat gta ata cat tgc agg      1200
   Lys Lys Tyr Ala His Arg Leu Leu Ser Tyr Tyr Val Ile His Cys Arg
   385                 390                 395                 400
   gag tgt gac atg ctt cat gac aaa agt ggg ctt aag tat aat ggt gat      1248
   Glu Cys Asp Met Leu His Asp Lys Ser Gly Leu Lys Tyr Asn Gly Asp
                   405                 410                 415
   ttt act aac aaa tgc ggt ttt tgt aca tgt ggg caa gtt gaa gat gca      1296
   Phe Thr Asn Lys Cys Gly Phe Cys Thr Cys Gly Gln Val Glu Asp Ala
               420                 425                 430
   atg ggg ctg acc gtc cat caa ata cga aat cat tgc tct gcc aac tat      1344
   Met Gly Leu Thr Val His Gln Ile Arg Asn His Cys Ser Ala Asn Tyr
           435                 440                 445
   aga act aag atg gtg ctg aac tgg atg atc gtg cta tta tta gct act      1392
   Arg Thr Lys Met Val Leu Asn Trp Met Ile Val Leu Leu Leu Ala Thr
       450                 455                 460
   cta atc aaa gat agc ata atg ctg act gcc gca gca ggg aca gaa ttt      1440
   Leu Ile Lys Asp Ser Ile Met Leu Thr Ala Ala Ala Gly Thr Glu Phe
   465                 470                 475                 480
   gaa aca tgc atc aag aaa gta gaa atg aca ccg gaa tgc aca gga cct      1488
   Glu Thr Cys Ile Lys Lys Val Glu Met Thr Pro Glu Cys Thr Gly Pro
                   485                 490                 495
   ttc atg gaa gtt gga atc tgt gat cac cag cag aag agc cta aga tac      1536
   Phe Met Glu Val Gly Ile Cys Asp His Gln Gln Lys Ser Leu Arg Tyr
               500                 505                 510
   cat gag ata gct aat atg tta ttg ggt aaa ggg aca atc tca aaa tta      1584
   His Glu Ile Ala Asn Met Leu Leu Gly Lys Gly Thr Ile Ser Lys Leu
           515                 520                 525
   gat gtg gat att ata gat gca ttg cct gac agg ata gct gga gac ctc      1632
   Asp Val Asp Ile Ile Asp Ala Leu Pro Asp Arg Ile Ala Gly Asp Leu
       530                 535                 540
   cag ata ata cag tca agc agt gat ttc cac cat caa cag atg ctg gaa      1680
   Gln Ile Ile Gln Ser Ser Ser Asp Phe His His Gln Gln Met Leu Glu
   545                 550                 555                 560
   tat gtg ttt cta ttg cgt cat tgt gat tat tac tca agt ttt gtt tcg      1728
   Tyr Val Phe Leu Leu Arg His Cys Asp Tyr Tyr Ser Ser Phe Val Ser
                   565                 570                 575
   gat aac agc tac tca caa atc ttg tgg caa aca gca gca aag tta aat      1776
   Asp Asn Ser Tyr Ser Gln Ile Leu Trp Gln Thr Ala Ala Lys Leu Asn
               580                 585                 590
   gaa acc aac ctt tgc aaa cat gag aat tca aag aaa tcc tgc aag tgc       1824
   Glu Thr Asn Leu Cys Lys His Glu Asn Ser Lys Lys Ser Cys Lys Cys
           595                 600                 605
   tta aaa tca aat gca aat tgt gga gag tca gaa gac atc aac ata gca       1872
   Leu Lys Ser Asn Ala Asn Cys Gly Glu Ser Glu Asp Ile Asn Ile Ala
       610                 615                 620
   gca cag gca tac tac aaa aat aag gaa tct gag cta gga caa gat cta       1920
   Ala Gln Ala Tyr Tyr Lys Asn Lys Glu Ser Glu Leu Gly Gln Asp Leu
   625                 630                 635                 640
   aca acc ata atg tac ata ctc aaa aaa atg att cca gga tca ggt tat       1968
   Thr Thr Ile Met Tyr Ile Leu Lys Lys Met Ile Pro Gly Ser Gly Tyr
                   645                 650                 655
   agt tat ctg cta aat gca gcc aag cac aac aaa tta gaa ttg ttc aaa       2016
   Ser Tyr Leu Leu Asn Ala Ala Lys His Asn Lys Leu Glu Leu Phe Lys
               660                 665                 670
   gct tac atg act aat att tcc tca aaa tat tca aat aac aaa aaa tta       2064
   Ala Tyr Met Thr Asn Ile Ser Ser Lys Tyr Ser Asn Asn Lys Lys Leu
           675                 680                 685
   aaa gat ttc atg gcg ata ata aat gct ata cta gca aat ctg tca gct       2112
   Lys Asp Phe Met Ala Ile Ile Asn Ala Ile Leu Ala Asn Leu Ser Ala
       690                 695                 700
   ctc tca cat agt agt gaa gaa tca atc tca caa tac aaa cag att gat       2160
   Leu Ser His Ser Ser Glu Glu Ser Ile Ser Gln Tyr Lys Gln Ile Asp
   705                 710                 715                 720
   ttt tct tcc cta gta ggc gaa aca aca gac atg ctc gaa gca aat ttt       2208
   Phe Ser Ser Leu Val Gly Glu Thr Thr Asp Met Leu Glu Ala Asn Phe
                   725                 730                 735
   aat caa acg atc agt aca gac aag ata att tgt gaa aat cct aaa gta       2256
   Asn Gln Thr Ile Ser Thr Asp Lys Ile Ile Cys Glu Asn Pro Lys Val
               740                 745                 750
   atg att tgc att tca ccc agg aca aaa gtt tca tcc ggt gaa ttg tat       2304
   Met Ile Cys Ile Ser Pro Arg Thr Lys Val Ser Ser Gly Glu Leu Tyr
           755                 760                 765
   gtc tgc caa aga tca gga aag tac tac ata ata gat acc ggc aaa ttc       2352
   Val Cys Gln Arg Ser Gly Lys Tyr Tyr Ile Ile Asp Thr Gly Lys Phe
       770                 775                 780
   aag cta tat aaa tta att gac gca gac aat ttg tac tgt gca gca gat       2400
   Lys Leu Tyr Lys Leu Ile Asp Ala Asp Asn Leu Tyr Cys Ala Ala Asp
   785                 790                 795                 800
   gct cag tgt agg tat gaa ttt aga att tta acc aca ggg gaa atg cag       2448
   Ala Gln Cys Arg Tyr Glu Phe Arg Ile Leu Thr Thr Gly Glu Met Gln
                   805                 810                 815
   gca act ttt aaa gat aat tgc aaa gca tca gta cat gtt aac acc cca       2496
   Ala Thr Phe Lys Asp Asn Cys Lys Ala Ser Val His Val Asn Thr Pro
               820                 825                 830
   gga tac caa aat gaa gct ata act cat tgc aaa gtg ctc gcg aat ggt         2544
   Gly Tyr Gln Asn Glu Ala Ile Thr His Cys Lys Val Leu Ala Asn Gly
           835                 840                 845
   aaa tgt aag tat aat aat caa act tca ata gat ata aca ttg tgt gct         2592
   Lys Cys Lys Tyr Asn Asn Gln Thr Ser Ile Asp Ile Thr Leu Cys Ala
       850                 855                 860
   aac aac aga tat tat ccc gaa gca aaa ggc agg atc ttt gga gtt gat         2640
   Asn Asn Arg Tyr Tyr Pro Glu Ala Lys Gly Arg Ile Phe Gly Val Asp
   865                 870                 875                 880
   cca gaa gat cta tgc ttt gat aag aca tgt act aaa caa cag act cca         2688
   Pro Glu Asp Leu Cys Phe Asp Lys Thr Cys Thr Lys Gln Gln Thr Pro
                   885                 890                 895
   tac aac gaa gaa cta atc agt atg tgt gtt tgg gaa aat ccg aca ctt         2736
   Tyr Asn Glu Glu Leu Ile Ser Met Cys Val Trp Glu Asn Pro Thr Leu
               900                 905                 910
   agg cca atc aaa ccc aga act gag gtt tct atg act ttc caa gag tac         2784
   Arg Pro Ile Lys Pro Arg Thr Glu Val Ser Met Thr Phe Gln Glu Tyr
           915                 920                 925
   aaa gat cag tta tta aag aaa atc aac aca gac ctg gtg ctg cac aaa         2832
   Lys Asp Gln Leu Leu Lys Lys Ile Asn Thr Asp Leu Val Leu His Lys
       930                 935                 940
   ttt gtt aga aca caa aac tta cct tat ttc ata ccg caa ttc aag tat         2880
   Phe Val Arg Thr Gln Asn Leu Pro Tyr Phe Ile Pro Gln Phe Lys Tyr
   945                 950                 955                 960
   ata act ata caa gga acg tca aca aca gac ggg att act tcc agt ttt         2928
   Ile Thr Ile Gln Gly Thr Ser Thr Thr Asp Gly Ile Thr Ser Ser Phe
                   965                 970                 975
   ata ttc ttt gaa att cct gca ttg aca gga gca tct gct gga tac aaa         2976
   Ile Phe Phe Glu Ile Pro Ala Leu Thr Gly Ala Ser Ala Gly Tyr Lys
               980                 985                 990
   gtt ata aat agc gaa ggc caa gaa  ttg atg gac atg gta  gtt tat atc       3024
   Val Ile Asn Ser Glu Gly Gln Glu  Leu Met Asp Met Val  Val Tyr Ile
           995                 1000                 1005
   aaa aca  tca aag act agc gca  aag tac aag cgc gtg  tat tat aca         3069
   Lys Thr  Ser Lys Thr Ser Ala  Lys Tyr Lys Arg Val  Tyr Tyr Thr
       1010                 1015                 1020
   ggc ccc  act ata gct ata aat  aca aaa cat caa gag  cac tgc aca         3114
   Gly Pro  Thr Ile Ala Ile Asn  Thr Lys His Gln Glu  His Cys Thr
       1025                 1030                 1035
   ggg aca  tgt cct gaa aga ata  gaa cat gat gaa ggt  tgg gca aca         3159
   Gly Thr  Cys Pro Glu Arg Ile  Glu His Asp Glu Gly  Trp Ala Thr
       1040                 1045                 1050
   ttt tcg  aaa gaa aga acc agc  aat tgg ggc tgt gaa  gaa ttt ggt         3204
   Phe Ser  Lys Glu Arg Thr Ser  Asn Trp Gly Cys Glu  Glu Phe Gly
       1055                 1060                 1065
   tgt cta  gct gtt gag act ggt  tgt tta ttt ggg tca  tgc cag gat         3249
   Cys Leu  Ala Val Glu Thr Gly  Cys Leu Phe Gly Ser  Cys Gln Asp
       1070                 1075                 1080
   gta att  aga aga gaa ctt gat  gtt ttt tca aag ttg  gag gaa gat          3294
   Val Ile  Arg Arg Glu Leu Asp  Val Phe Ser Lys Leu  Glu Glu Asp
       1085                 1090                 1095
   aaa ata  ttg act gaa ctc tgt  ata aca tta agc cat  gac act tac          3339
   Lys Ile  Leu Thr Glu Leu Cys  Ile Thr Leu Ser His  Asp Thr Tyr
       1100                 1105                 1110
   tgc gtg  aca atc gat tct aca  aca cca aca atc aat  gat tac ttt          3384
   Cys Val  Thr Ile Asp Ser Thr  Thr Pro Thr Ile Asn  Asp Tyr Phe
       1115                 1120                 1125
   gaa atc  caa tac aaa aca gta  gat gta caa aca ttg  cca aag atg          3429
   Glu Ile  Gln Tyr Lys Thr Val  Asp Val Gln Thr Leu  Pro Lys Met
       1130                 1135                 1140
   ata gga  gtc act cat cat aag  cta ttg cga ggg cag  ata aac gaa          3474
   Ile Gly  Val Thr His His Lys  Leu Leu Arg Gly Gln  Ile Asn Glu
       1145                 1150                 1155
   tta gga  tca ttt ggc aaa ttt  tgt ggc aac ata caa  gta gtt ggt          3519
   Leu Gly  Ser Phe Gly Lys Phe  Cys Gly Asn Ile Gln  Val Val Gly
       1160                 1165                 1170
   aat caa  aca tat ggg cag gct  gca gta aag ttt gat  tac att tgc          3564
   Asn Gln  Thr Tyr Gly Gln Ala  Ala Val Lys Phe Asp  Tyr Ile Cys
       1175                 1180                 1185
   cat gct  gca caa aga aaa gat  gta gtt ata aga aag  tgc tta gaa          3609
   His Ala  Ala Gln Arg Lys Asp  Val Val Ile Arg Lys  Cys Leu Glu
       1190                 1195                 1200
   aat aat  tac caa agt tgt aag  cta ctg aca cat gac  aca gac ttg          3654
   Asn Asn  Tyr Gln Ser Cys Lys  Leu Leu Thr His Asp  Thr Asp Leu
       1205                 1210                 1215
   ata ctt  gaa gaa agt aag acc  agt gtt gaa gtc ata  aac aat aaa          3699
   Ile Leu  Glu Glu Ser Lys Thr  Ser Val Glu Val Ile  Asn Asn Lys
       1220                 1225                 1230
   agg att  aca gga caa ata gca  cta aag gtc ttg ttg  gga gac ttt          3744
   Arg Ile  Thr Gly Gln Ile Ala  Leu Lys Val Leu Leu  Gly Asp Phe
       1235                 1240                 1245
   aac tac  aag cag tat gct gca  aat gta gag gct gac  ata gaa gca          3789
   Asn Tyr  Lys Gln Tyr Ala Ala  Asn Val Glu Ala Asp  Ile Glu Ala
       1250                 1255                 1260
   aat tgc  gtt ggg tgt aca aat  tgt ata aag gga ata  aca tgt gaa          3834
   Asn Cys  Val Gly Cys Thr Asn  Cys Ile Lys Gly Ile  Thr Cys Glu
       1265                 1270                 1275
   ata aag  gtg aaa aca gaa att  gaa gca tca tgt aag  gta gag ccc          3879
   Ile Lys  Val Lys Thr Glu Ile  Glu Ala Ser Cys Lys  Val Glu Pro
       1280                 1285                 1290
   cca tgc  ccc tct tac aca agt  aga att ata ata aaa  cca ggt aga          3924
   Pro Cys  Pro Ser Tyr Thr Ser  Arg Ile Ile Ile Lys  Pro Gly Arg
       1295                 1300                 1305
   gac acc  cac aca tta ttg atg  aag tgt gac aaa aaa  ata acg act          3969
   Asp Thr  His Thr Leu Leu Met  Lys Cys Asp Lys Lys  Ile Thr Thr
       1310                1315                1320
   ggt gaa  cta gca ata aaa ata  tgt aat atg caa atc  cag gct cat        4014
   Gly Glu  Leu Ala Ile Lys Ile  Cys Asn Met Gln Ile  Gln Ala His
       1325                1330                1335
   ctt tca  aca ata gag agc aat  gac caa cta gaa ttg  tcg act ggg        4059
   Leu Ser  Thr Ile Glu Ser Asn  Asp Gln Leu Glu Leu  Ser Thr Gly
       1340                1345                1350
   gat caa  tca aca tat ata cat  gaa gaa gac cta agg  tgt tca acc        4104
   Asp Gln  Ser Thr Tyr Ile His  Glu Glu Asp Leu Arg  Cys Ser Thr
       1355                1360                1365
   tgg atc  tgc aww gtg aga gac  gaa ggg ata agc ttc  atc ttt aag        4149
   Trp Ile  Cys Xaa Val Arg Asp  Glu Gly Ile Ser Phe  Ile Phe Lys
       1370                1375                1380
   cca tta  aca gac tgg ctt gga  agc ttc aca aag ccg  ata ctt ata        4194
   Pro Leu  Thr Asp Trp Leu Gly  Ser Phe Thr Lys Pro  Ile Leu Ile
       1385                1390                1395
   gct ggc  tgt atg ata att gtt  gca att att ttg att  tat ata tta        4239
   Ala Gly  Cys Met Ile Ile Val  Ala Ile Ile Leu Ile  Tyr Ile Leu
       1400                1405                1410
   atg cct  atg tgt gct aga ctc  aga gat ttg tta aag  aaa aac gag        4284
   Met Pro  Met Cys Ala Arg Leu  Arg Asp Leu Leu Lys  Lys Asn Glu
       1415                1420                1425
   tat gaa  tat cta caa gat aca  aaa gtg att gta aag  ccc aag ggt        4329
   Tyr Glu  Tyr Leu Gln Asp Thr  Lys Val Ile Val Lys  Pro Lys Gly
       1430                1435                1440
   aca gca  agt tca ata ggc ttg  aga tcg aag aaa ata  aat atc caa        4374
   Thr Ala  Ser Ser Ile Gly Leu  Arg Ser Lys Lys Ile  Asn Ile Gln
       1445                1450                1455
   tcg tct  aag gct gtt gat taa                                          4395
   Ser Ser  Lys Ala Val Asp
       1460
<210> 4
   <211> 1464
   <212> PRT
   <213> Moissiacense virus
<220>
   <221> misc_feature
   <222> (1372)..(1372)
   <223> The 'Xaa' at location 1372 stands for Lys or Ile.
<400> 4
   Met Val Ile Phe Ala Leu Leu Ser Ile Leu Leu Val Gly Phe Ser Asn
   1               5                   10                  15
   Gln Ala Thr Ile Arg Cys Phe Ser Gly Gly Ser Lys Ile Met Glu Lys
               20                  25                  30
   Thr Ser Asn Val Ser Leu Ser Glu Leu Cys Leu Lys Asp Asp Val Ser
           35                  40                  45
   Gln Leu Lys Ile Ile Val Asp His Thr Lys Asn Asp Ser Gly Leu Phe
       50                  55                  60
   Ser Thr Ala Ser Val Trp Arg Lys Trp Ile Val Ser Asp Trp Thr Thr
   65                  70                  75                  80
   Cys Asn Pro Lys Lys Met Ala Leu Gly Thr Ile Ser Val Ile Asp Ile
                   85                  90                  95
   Asp Lys Asn Phe Ile Leu Lys Ser Ser Ser Tyr Ile Cys Asn Lys Glu
               100                 105                 110
   Cys Thr Ile Thr Val Asp Lys Glu Arg Ala Gln Val Leu Leu Gln Ser
           115                 120                 125
   Asp Gly Leu Asn His Phe Glu Val Ser Gly Thr Thr Leu Ser Ser Gly
       130                 135                 140
   Trp Phe Lys Ser Thr Ala Thr Val Thr Leu Asp Gln Thr Cys Glu His
   145                 150                 155                 160
   Val Lys Val Ser Cys Gly Gln Lys Ser Met Gln Phe His Ala Cys Phe
                   165                 170                 175
   Lys Asn His Met Ser Cys Ile Arg Phe Leu His Arg Ser Ile Met Pro
               180                 185                 190
   Ala Ser Phe Ala Val Ser Ile Cys Gln Asn Ile Glu Leu Ile Leu Met
           195                 200                 205
   Thr Thr Phe Thr Leu Ile Ile Phe Leu Leu Leu Ser Ile Leu Thr Lys
       210                 215                 220
   Thr Tyr Ile Cys Tyr Ile Leu Leu Pro Ile Phe Ile Pro Phe Ala Tyr
   225                 230                 235                 240
   Ala Tyr Gly Phe Val Tyr Asp Lys Thr Cys Lys Lys Cys Ser Leu Cys
                   245                 250                 255
   Gly Leu Ala Tyr His Pro Phe Thr Lys Cys Gly Ile His Cys Val Cys
               260                 265                 270
   Gly Cys Arg Phe Glu Asn Ser Asp Ala Met Lys Thr His Arg Glu Lys
           275                 280                 285
   Gly Lys Cys Pro Gly Phe Lys Ser Met Arg Ile Ala Arg Val Leu Cys
       290                 295                 300
   Lys Ala Lys Gly Ser Ala Phe Ala Leu Ser Val Leu Leu Ala Thr Leu
   305                 310                 315                 320
   Ala Leu Gly Phe Leu Thr Pro Val Gln Gly Leu Val Leu Asp Lys Thr
                   325                 330                 335
   Gln Ile Asn Tyr Thr Glu Asp Gln Leu Pro Glu Ser Phe Gln Thr Met
               340                 345                 350
   Gln Ser Thr Ile Ser Ile Phe Lys Phe Ala Thr Val Met Asn Ala Gly
           355                 360                 365
   Trp Thr Tyr Cys Met Phe Leu Val Thr Leu Ala Leu Tyr Ile Ile His
       370                 375                 380
   Lys Lys Tyr Ala His Arg Leu Leu Ser Tyr Tyr Val Ile His Cys Arg
   385                 390                 395                 400
   Glu Cys Asp Met Leu His Asp Lys Ser Gly Leu Lys Tyr Asn Gly Asp
                   405                 410                 415
   Phe Thr Asn Lys Cys Gly Phe Cys Thr Cys Gly Gln Val Glu Asp Ala
               420                 425                 430
   Met Gly Leu Thr Val His Gln Ile Arg Asn His Cys Ser Ala Asn Tyr
           435                 440                 445
   Arg Thr Lys Met Val Leu Asn Trp Met Ile Val Leu Leu Leu Ala Thr
       450                 455                 460
   Leu Ile Lys Asp Ser Ile Met Leu Thr Ala Ala Ala Gly Thr Glu Phe
   465                 470                 475                 480
   Glu Thr Cys Ile Lys Lys Val Glu Met Thr Pro Glu Cys Thr Gly Pro
                   485                 490                 495
   Phe Met Glu Val Gly Ile Cys Asp His Gln Gln Lys Ser Leu Arg Tyr
               500                 505                 510
   His Glu Ile Ala Asn Met Leu Leu Gly Lys Gly Thr Ile Ser Lys Leu
           515                 520                 525
   Asp Val Asp Ile Ile Asp Ala Leu Pro Asp Arg Ile Ala Gly Asp Leu
       530                 535                 540
   Gln Ile Ile Gln Ser Ser Ser Asp Phe His His Gln Gln Met Leu Glu
   545 550 555 560
   Tyr Val Phe Leu Leu Arg His Cys Asp Tyr Tyr Ser Ser Phe Val Ser
   565 570 575
   Asp Asn Ser Tyr Ser Gln Ile Leu Trp Gln Thr Ala Ala Lys Leu Asn
   580 585 590
   Glu Thr Asn Leu Cys Lys His Glu Asn Ser Lys Lys Ser Cys Lys Cys
   595 600 605
   Leu Lys Ser Asn Ala Asn Cys Gly Glu Ser Glu Asp Ile Asn Ile Ala
   610 615 620
   Ala Gln Ala Tyr Tyr Lys Asn Lys Glu Ser Glu Leu Gly Gln Asp Leu
   625 630 635 640
   Thr Thr Ile Met Tyr Ile Leu Lys Lys Met Ile Pro Gly Ser Gly Tyr
   645 650 655
   Ser Tyr Leu Leu Asn Ala Ala Lys His Asn Lys Leu Glu Leu Phe Lys
   660 665 670
   Ala Tyr Met Thr Asn Ile Ser Ser Lys Tyr Ser Asn Asn Lys Lys Leu
   675 680 685
   Lys Asp Phe Met Ala Ile Ile Asn Ala Ile Leu Ala Asn Leu Ser Ala
   690 695 700
   Leu Ser His Ser Ser Glu Glu Ser Ile Ser Gln Tyr Lys Gln Ile Asp
   705 710 715 720
   Phe Ser Ser Leu Val Gly Glu Thr Thr Asp Met Leu Glu Ala Asn Phe
   725 730 735
   Asn Gln Thr Ile Ser Thr Asp Lys Ile Ile Cys Glu Asn Pro Lys Val
   740 745 750
   Met Ile Cys Ile Ser Pro Arg Thr Lys Val Ser Ser Gly Glu Leu Tyr
   755 760 765
   Val Cys Gln Arg Ser Gly Lys Tyr Tyr Ile Ile Asp Thr Gly Lys Phe
   770 775 780
   Lys Leu Tyr Lys Leu Ile Asp Ala Asp Asn Leu Tyr Cys Ala Ala Asp
   785 790 795 800
   Ala Gln Cys Arg Tyr Glu Phe Arg Ile Leu Thr Thr Gly Glu Met Gln
   805 810 815
   Ala Thr Phe Lys Asp Asn Cys Lys Ala Ser Val His Val Asn Thr Pro
   820 825 830
   Gly Tyr Gln Asn Glu Ala Ile Thr His Cys Lys Val Leu Ala Asn Gly
   835 840 845
   Lys Cys Lys Tyr Asn Asn Gln Thr Ser Ile Asp Ile Thr Leu Cys Ala
   850 855 860
   Asn Asn Arg Tyr Tyr Pro Glu Ala Lys Gly Arg Ile Phe Gly Val Asp
   865 870 875 880
   Pro Glu Asp Leu Cys Phe Asp Lys Thr Cys Thr Lys Gln Gln Thr Pro
   885 890 895
   Tyr Asn Glu Glu Leu Ile Ser Met Cys Val Trp Glu Asn Pro Thr Leu
   900 905 910
   Arg Pro Ile Lys Pro Arg Thr Glu Val Ser Met Thr Phe Gln Glu Tyr
   915 920 925
   Lys Asp Gln Leu Leu Lys Lys Ile Asn Thr Asp Leu Val Leu His Lys
   930 935 940
   Phe Val Arg Thr Gln Asn Leu Pro Tyr Phe Ile Pro Gln Phe Lys Tyr
   945 950 955 960
   Ile Thr Ile Gln Gly Thr Ser Thr Thr Asp Gly Ile Thr Ser Ser Phe
   965 970 975
   Ile Phe Phe Glu Ile Pro Ala Leu Thr Gly Ala Ser Ala Gly Tyr Lys
   980 985 990
   Val Ile Asn Ser Glu Gly Gln Glu Leu Met Asp Met Val Val Tyr Ile
   995 1000 1005
   Lys Thr Ser Lys Thr Ser Ala Lys Tyr Lys Arg Val Tyr Tyr Thr
   1010 1015 1020
   Gly Pro Thr Ile Ala Ile Asn Thr Lys His Gln Glu His Cys Thr
   1025 1030 1035
   Gly Thr Cys Pro Glu Arg Ile Glu His Asp Glu Gly Trp Ala Thr
   1040 1045 1050
   Phe Ser Lys Glu Arg Thr Ser Asn Trp Gly Cys Glu Glu Phe Gly
   1055 1060 1065
   Cys Leu Ala Val Glu Thr Gly Cys Leu Phe Gly Ser Cys Gln Asp
   1070 1075 1080
   Val Ile Arg Arg Glu Leu Asp Val Phe Ser Lys Leu Glu Glu Asp
   1085 1090 1095
   Lys Ile Leu Thr Glu Leu Cys Ile Thr Leu Ser His Asp Thr Tyr
   1100 1105 1110
   Cys Val Thr Ile Asp Ser Thr Thr Pro Thr Ile Asn Asp Tyr Phe
   1115 1120 1125
   Glu Ile Gln Tyr Lys Thr Val Asp Val Gln Thr Leu Pro Lys Met
   1130 1135 1140
   Ile Gly Val Thr His His Lys Leu Leu Arg Gly Gln Ile Asn Glu
   1145 1150 1155
   Leu Gly Ser Phe Gly Lys Phe Cys Gly Asn Ile Gln Val Val Gly
   1160 1165 1170
   Asn Gln Thr Tyr Gly Gln Ala Ala Val Lys Phe Asp Tyr Ile Cys
   1175 1180 1185
   His Ala Ala Gln Arg Lys Asp Val Val Ile Arg Lys Cys Leu Glu
   1190 1195 1200
   Asn Asn Tyr Gln Ser Cys Lys Leu Leu Thr His Asp Thr Asp Leu
   1205 1210 1215
   Ile Leu Glu Glu Ser Lys Thr Ser Val Glu Val Ile Asn Asn Lys
   1220 1225 1230
   Arg Ile Thr Gly Gln Ile Ala Leu Lys Val Leu Leu Gly Asp Phe
   1235 1240 1245
   Asn Tyr Lys Gln Tyr Ala Ala Asn Val Glu Ala Asp Ile Glu Ala
   1250 1255 1260
   Asn Cys Val Gly Cys Thr Asn Cys Ile Lys Gly Ile Thr Cys Glu
   1265 1270 1275
   Ile Lys Val Lys Thr Glu Ile Glu Ala Ser Cys Lys Val Glu Pro
   1280 1285 1290
   Pro Cys Pro Ser Tyr Thr Ser Arg Ile Ile Ile Lys Pro Gly Arg
   1295 1300 1305
   Asp Thr His Thr Leu Leu Met Lys Cys Asp Lys Lys Ile Thr Thr
   1310 1315 1320
   Gly Glu Leu Ala Ile Lys Ile Cys Asn Met Gln Ile Gln Ala His
   1325 1330 1335
   Leu Ser Thr Ile Glu Ser Asn Asp Gln Leu Glu Leu Ser Thr Gly
   1340 1345 1350
   Asp Gln Ser Thr Tyr Ile His Glu Glu Asp Leu Arg Cys Ser Thr
   1355 1360 1365
   Trp Ile Cys Xaa Val Arg Asp Glu Gly Ile Ser Phe Ile Phe Lys
   1370 1375 1380
   Pro Leu Thr Asp Trp Leu Gly Ser Phe Thr Lys Pro Ile Leu Ile
   1385 1390 1395
   Ala Gly Cys Met Ile Ile Val Ala Ile Ile Leu Ile Tyr Ile Leu
   1400 1405 1410
   Met Pro Met Cys Ala Arg Leu Arg Asp Leu Leu Lys Lys Asn Glu
   1415 1420 1425
   Tyr Glu Tyr Leu Gln Asp Thr Lys Val Ile Val Lys Pro Lys Gly
   1430 1435 1440
   Thr Ala Ser Ser Ile Gly Leu Arg Ser Lys Lys Ile Asn Ile Gln
   1445 1450 1455
   Ser Ser Lys Ala Val Asp
   1460
<210> 5
   <211> 6828
   <212> DNA
   <213> Moissiacense virus
<220>
   <221> CDS
   <222> (1)..(6828)
<400> 5
   atg ctg gac caa gaa aat gag caa gct tta aat aga ctt gca gcc gca 48
   Met Leu Asp Gln Glu Asn Glu Gln Ala Leu Asn Arg Leu Ala Ala Ala
   1 5 10 15
   gtt gcg aga gca aat gag cct gcc gaa ggc aaa gaa gca tat aat gct 96
   Val Ala Arg Ala Asn Glu Pro Ala Glu Gly Lys Glu Ala Tyr Asn Ala
   20 25 30
   ata ctg agt aga agg cat gat tac ttt ggg caa gtt ttt tgt gag acc 144
   Ile Leu Ser Arg Arg His Asp Tyr Phe Gly Gln Val Phe Cys Glu Thr
   35 40 45
   ata ggc ata cca tac agg aac gat gtt cgg cta gag gat atc cta cat 192
   Ile Gly Ile Pro Tyr Arg Asn Asp Val Arg Leu Glu Asp Ile Leu His
   50 55 60
   gaa tgt gac caa aca gtc gac act cta aca aat aag ata cca aac ata 240
   Glu Cys Asp Gln Thr Val Asp Thr Leu Thr Asn Lys Ile Pro Asn Ile
   65 70 75 80
   act cca gac aac ttt gag ttt aga gac aat atc cta act ata ata gat 288
   Thr Pro Asp Asn Phe Glu Phe Arg Asp Asn Ile Leu Thr Ile Ile Asp
   85 90 95
   tat aaa gta gca gtt agt gat gaa agt gct gac tat act cgt gaa aag 336
   Tyr Lys Val Ala Val Ser Asp Glu Ser Ala Asp Tyr Thr Arg Glu Lys
   100 105 110
   tat gaa aat gct gtt gat gcc ata aag cat ttg gtt gac ttt cct att 384
   Tyr Glu Asn Ala Val Asp Ala Ile Lys His Leu Val Asp Phe Pro Ile
   115 120 125
   aga gtg tgt ata gtt agg ata cac cca tac act aga caa gtt act tac 432
   Arg Val Cys Ile Val Arg Ile His Pro Tyr Thr Arg Gln Val Thr Tyr
   130 135 140
   tct gat gac aat ttt atg aga cga ttt gcc cat gtg gta tac cct gtt 480
   Ser Asp Asp Asn Phe Met Arg Arg Phe Ala His Val Val Tyr Pro Val
   145 150 155 160
   gat ttt aca agg tat act caa tta act gaa gaa ctc aag gaa aaa ttt 528
   Asp Phe Thr Arg Tyr Thr Gln Leu Thr Glu Glu Leu Lys Glu Lys Phe
   165 170 175
   gca gat agc cct gaa ttt atg gat atg ata gca tat ggc gat gtc act 576
   Ala Asp Ser Pro Glu Phe Met Asp Met Ile Ala Tyr Gly Asp Val Thr
   180 185 190
   ttt act gca aac tgg tgt caa aat gga tgc cct gag cta tat ggg cat 624
   Phe Thr Ala Asn Trp Cys Gln Asn Gly Cys Pro Glu Leu Tyr Gly His
   195 200 205
   ccg ata ttc aaa gaa ttt tta gaa agc atg cca ttg aaa tac cag aaa 672
   Pro Ile Phe Lys Glu Phe Leu Glu Ser Met Pro Leu Lys Tyr Gln Lys
   210 215 220
   ttg ttt gaa aag tct tta ctg agt tct tgc tat act tct gag aga tgg 720
   Leu Phe Glu Lys Ser Leu Leu Ser Ser Cys Tyr Thr Ser Glu Arg Trp
   225 230 235 240
   gat tcc aat ctt aaa gaa gtc aag ggt gca act aaa gat gag tat gaa 768
   Asp Ser Asn Leu Lys Glu Val Lys Gly Ala Thr Lys Asp Glu Tyr Glu
                   245                 250                 255
   gat ttt ata tct cga tct gca aga gag ctc tat aca aca tct cct gac        816
   Asp Phe Ile Ser Arg Ser Ala Arg Glu Leu Tyr Thr Thr Ser Pro Asp
               260                 265                 270
   tat tca gaa cct agt cga gag gaa att cta aaa ggg tgg gaa gaa atg        864
   Tyr Ser Glu Pro Ser Arg Glu Glu Ile Leu Lys Gly Trp Glu Glu Met
           275                 280                 285
   aca gaa cga gtg aaa gaa caa agg gaa gtg tcc tca aat att aat gac        912
   Thr Glu Arg Val Lys Glu Gln Arg Glu Val Ser Ser Asn Ile Asn Asp
       290                 295                 300
   caa aag cca agt gct cac ttc ata tgg gct gaa aac aat cct gaa cta        960
   Gln Lys Pro Ser Ala His Phe Ile Trp Ala Glu Asn Asn Pro Glu Leu
   305                 310                 315                 320
   ccg act gca aat tct gct aag ata tgt gca att tca aaa ctt tta cag       1008
   Pro Thr Ala Asn Ser Ala Lys Ile Cys Ala Ile Ser Lys Leu Leu Gln
                   325                 330                 335
   tcc att aac ata gaa agc tct ttt gct gag gat ttt aaa aga cta ggc       1056
   Ser Ile Asn Ile Glu Ser Ser Phe Ala Glu Asp Phe Lys Arg Leu Gly
               340                 345                 350
   aaa tta ttc gac ttt tct gaa gat atc cct gga tat gag cga cat tgc       1104
   Lys Leu Phe Asp Phe Ser Glu Asp Ile Pro Gly Tyr Glu Arg His Cys
           355                 360                 365
   gaa caa ctt aaa agg cag gct aga tca aat cca gga caa ata aaa aac       1152
   Glu Gln Leu Lys Arg Gln Ala Arg Ser Asn Pro Gly Gln Ile Lys Asn
       370                 375                 380
   aaa aaa cta gac cca aag aaa att ggg aat gca tta gtt tta tgg gag       1200
   Lys Lys Leu Asp Pro Lys Lys Ile Gly Asn Ala Leu Val Leu Trp Glu
   385                 390                 395                 400
   caa caa ttt tta tta tct gca gat gaa atg ggg aaa gag aat aaa cta       1248
   Gln Gln Phe Leu Leu Ser Ala Asp Glu Met Gly Lys Glu Asn Lys Leu
                   405                 410                 415
   cac tta ctt aaa gac ttc ttt ggc ata ggg aaa cac aaa caa ttt gca       1296
   His Leu Leu Lys Asp Phe Phe Gly Ile Gly Lys His Lys Gln Phe Ala
               420                 425                 430
   aaa aaa ggc tta gat gac act gaa aca gat aaa ccg aca atc tta gat       1344
   Lys Lys Gly Leu Asp Asp Thr Glu Thr Asp Lys Pro Thr Ile Leu Asp
           435                 440                 445
   ttt aat gat aca gaa gtc aaa aga tca gca gag ttg atg atg caa aga       1392
   Phe Asn Asp Thr Glu Val Lys Arg Ser Ala Glu Leu Met Met Gln Arg
       450                 455                 460
   aca aaa ctc tac tta tct ggc agg tcg tca ctc aat aaa gaa cat cct       1440
   Thr Lys Leu Tyr Leu Ser Gly Arg Ser Ser Leu Asn Lys Glu His Pro
   465                 470                 475                 480
   gta gca tca gaa tat cta aat gaa ata aaa aag tgt aat gat gag aca       1488
   Val Ala Ser Glu Tyr Leu Asn Glu Ile Lys Lys Cys Asn Asp Glu Thr
                   485                 490                 495
   cat aat gtt ctg aat gca atc ttt cag aca aaa tat tgg caa tgt atc       1536
   His Asn Val Leu Asn Ala Ile Phe Gln Thr Lys Tyr Trp Gln Cys Ile
               500                 505                 510
   aca gat att agc atg cta atg aaa aat atg ctt tct gtg tcg cag tat        1584
   Thr Asp Ile Ser Met Leu Met Lys Asn Met Leu Ser Val Ser Gln Tyr
           515                 520                 525
   aat aga aac aat act ttc aga att gca aca tgt gct aat aat gca cta        1632
   Asn Arg Asn Asn Thr Phe Arg Ile Ala Thr Cys Ala Asn Asn Ala Leu
       530                 535                 540
   tat gga ctt atc ttt ccg tcg gca gat att aaa aca aag gat gca agc        1680
   Tyr Gly Leu Ile Phe Pro Ser Ala Asp Ile Lys Thr Lys Asp Ala Ser
   545                 550                 555                 560
   att gta tat tgc att att gca att cat aaa gat gag aag tct tta ttc        1728
   Ile Val Tyr Cys Ile Ile Ala Ile His Lys Asp Glu Lys Ser Leu Phe
                   565                 570                 575
   aat cca gga gct cta cat gca act ttc aaa gtg cca ggt ggc ttt tta        1776
   Asn Pro Gly Ala Leu His Ala Thr Phe Lys Val Pro Gly Gly Phe Leu
               580                 585                 590
   tct ata tct aaa tca atg cga ctt gac aag aag cgc tgt cac aga ata        1824
   Ser Ile Ser Lys Ser Met Arg Leu Asp Lys Lys Arg Cys His Arg Ile
           595                 600                 605
   gtt act agt cca ggc ata ttc tta aca att gcc acc atg ttg aaa gga        1872
   Val Thr Ser Pro Gly Ile Phe Leu Thr Ile Ala Thr Met Leu Lys Gly
       610                 615                 620
   gat aat cca act att agt ttg aat gat gtt tta aat ttt agc att ttt        1920
   Asp Asn Pro Thr Ile Ser Leu Asn Asp Val Leu Asn Phe Ser Ile Phe
   625                 630                 635                 640
   gcg agc ttg aac ata act aag agc atg cta tca tta aca gag cct tct        1968
   Ala Ser Leu Asn Ile Thr Lys Ser Met Leu Ser Leu Thr Glu Pro Ser
                   645                 650                 655
   agg tat atg atc atg aac tca tta gca gtc tcc agt cat gta cgc gaa        2016
   Arg Tyr Met Ile Met Asn Ser Leu Ala Val Ser Ser His Val Arg Glu
               660                 665                 670
   tac atc tct gag aaa ttt tcg cca tat act aaa act ctc ttt agt gtc        2064
   Tyr Ile Ser Glu Lys Phe Ser Pro Tyr Thr Lys Thr Leu Phe Ser Val
           675                 680                 685
   cat atg gtt gag aaa ata aaa gca ggc tgt cta aat gca ttc aaa caa        2112
   His Met Val Glu Lys Ile Lys Ala Gly Cys Leu Asn Ala Phe Lys Gln
       690                 695                 700
   aga gaa aaa atc aag act cgc gac ata tac ttg aca gac ata gat ata        2160
   Arg Glu Lys Ile Lys Thr Arg Asp Ile Tyr Leu Thr Asp Ile Asp Ile
   705                 710                 715                 720
   act caa aaa ggg ata caa gat aac aga gac cta tgt agc ata tgg ttt        2208
   Thr Gln Lys Gly Ile Gln Asp Asn Arg Asp Leu Cys Ser Ile Trp Phe
                   725                 730                 735
   gat ggt aat gtc act ttg aaa gaa tat cta aac cag att tat att gtc        2256
   Asp Gly Asn Val Thr Leu Lys Glu Tyr Leu Asn Gln Ile Tyr Ile Val
               740                 745                 750
   ttt tat ctg aat cca aaa gca tta cat gaa aag cat cat gta atg ata        2304
   Phe Tyr Leu Asn Pro Lys Ala Leu His Glu Lys His His Val Met Ile
           755                 760                 765
   gat tta ctc aaa aca gtg ata gaa ata gaa ctc gat caa agg gag aat        2352
   Asp Leu Leu Lys Thr Val Ile Glu Ile Glu Leu Asp Gln Arg Glu Asn
       770                 775                 780
   gtc cct caa cca tgg tct gac gtc cca aaa aag cag act gtc aat ttg        2400
   Val Pro Gln Pro Trp Ser Asp Val Pro Lys Lys Gln Thr Val Asn Leu
   785                 790                 795                 800
   ccc att ctt gtt cat agc cta gca aag aat cta ctt cta gac act tca        2448
   Pro Ile Leu Val His Ser Leu Ala Lys Asn Leu Leu Leu Asp Thr Ser
                   805                 810                 815
   ttc gcg cat ttc tta agg tct aga att gaa agc cgc aat aat ttt aga        2496
   Phe Ala His Phe Leu Arg Ser Arg Ile Glu Ser Arg Asn Asn Phe Arg
               820                 825                 830
   cgc acg ttt gca aca att tca act ttt aca agt agt aag agt tgc tta        2544
   Arg Thr Phe Ala Thr Ile Ser Thr Phe Thr Ser Ser Lys Ser Cys Leu
           835                 840                 845
   aaa aca ggc aat ttc cat gag ttc aag att aag caa tgt caa cgg ctc        2592
   Lys Thr Gly Asn Phe His Glu Phe Lys Ile Lys Gln Cys Gln Arg Leu
       850                 855                 860
   aaa aaa gtg aat aca agt gaa gca aaa aaa cat aga ata gca aat gcc        2640
   Lys Lys Val Asn Thr Ser Glu Ala Lys Lys His Arg Ile Ala Asn Ala
   865                 870                 875                 880
   atg ttt gtt gag gaa caa gac caa cat tta gag gtg gag cat gca aac        2688
   Met Phe Val Glu Glu Gln Asp Gln His Leu Glu Val Glu His Ala Asn
                   885                 890                 895
   tac gaa tta atg agg aag gca atc ccg gat tat gtg gat gtc tta tcg        2736
   Tyr Glu Leu Met Arg Lys Ala Ile Pro Asp Tyr Val Asp Val Leu Ser
               900                 905                 910
   acc aaa gta ttc gat aga tta tat gag cta tat aaa act gga gag att        2784
   Thr Lys Val Phe Asp Arg Leu Tyr Glu Leu Tyr Lys Thr Gly Glu Ile
           915                 920                 925
   gat gac agt cct gtt atc cat cag att ttt gcg acc atg aaa agc cac        2832
   Asp Asp Ser Pro Val Ile His Gln Ile Phe Ala Thr Met Lys Ser His
       930                 935                 940
   act aaa ttc tat ttt act tat ttt aat aaa ggc caa aaa aca gca aaa        2880
   Thr Lys Phe Tyr Phe Thr Tyr Phe Asn Lys Gly Gln Lys Thr Ala Lys
   945                 950                 955                 960
   gac cga gaa ata ttt gta ggg gaa tac gag gcc aaa atg tgt atg tat        2928
   Asp Arg Glu Ile Phe Val Gly Glu Tyr Glu Ala Lys Met Cys Met Tyr
                   965                 970                 975
   ctg gta gaa aga ata tat aaa gag aga tcg aaa gtc aat cca gac gaa        2976
   Leu Val Glu Arg Ile Tyr Lys Glu Arg Ser Lys Val Asn Pro Asp Glu
               980                 985                 990
   atg atc agt gaa cca gga gat ggg  aaa ctc aga gtg cta  gag cgc aaa      3024
   Met Ile Ser Glu Pro Gly Asp Gly  Lys Leu Arg Val Leu  Glu Arg Lys
           995                 1000                1005
   gca gag caa gag ata aga ttc atg gtt gaa aaa atg aaa gca aag      3069
   Ala Glu Gln Glu Ile Arg Phe Met Val Glu Lys Met Lys Ala Lys
       1010                1015                1020
   aat gat gaa ata aat aag gaa atc gag gat gca aaa gac aat tat      3114
   Asn Asp Glu Ile Asn Lys Glu Ile Glu Asp Ala Lys Asp Asn Tyr
       1025                1030                1035
   gtc cta aat tta ggg aaa ata gca gca ctg caa gca caa aaa tat      3159
   Val Leu Asn Leu Gly Lys Ile Ala Ala Leu Gln Ala Gln Lys Tyr
       1040                1045                1050
   aaa gct tta aaa ttg gaa atc aat gca gac atg tca aag tgg agt      3204
   Lys Ala Leu Lys Leu Glu Ile Asn Ala Asp Met Ser Lys Trp Ser
       1055                1060                1065
   gct caa gac gtg ttc ttt aaa tac ttt tgg tct gtc tgt ctt gac      3249
   Ala Gln Asp Val Phe Phe Lys Tyr Phe Trp Ser Val Cys Leu Asp
       1070                1075                1080
   cca att ctc tat cct gaa gaa aaa gaa cac att tta tat ttt ttt      3294
   Pro Ile Leu Tyr Pro Glu Glu Lys Glu His Ile Leu Tyr Phe Phe
       1085                1090                1095
   tgt aac tat atg caa aaa gaa ttg gtt atc cca gat gag gtt atc      3339
   Cys Asn Tyr Met Gln Lys Glu Leu Val Ile Pro Asp Glu Val Ile
       1100                1105                1110
   tgc aat atc cta gat cag agg aaa gca tac cct aat gac ata att      3384
   Cys Asn Ile Leu Asp Gln Arg Lys Ala Tyr Pro Asn Asp Ile Ile
       1115                1120                1125
   agt cag atc aca aat ggc ttg aca acc aac cat ttc aat gta aag      3429
   Ser Gln Ile Thr Asn Gly Leu Thr Thr Asn His Phe Asn Val Lys
       1130                1135                1140
   aga aac tgg cta caa ggc aac ttc aat tac atg tcc agc tat gct      3474
   Arg Asn Trp Leu Gln Gly Asn Phe Asn Tyr Met Ser Ser Tyr Ala
       1145                1150                1155
   cac agt aca gca atg tct gtc ttt aaa gat gtg ata aag cgt gct      3519
   His Ser Thr Ala Met Ser Val Phe Lys Asp Val Ile Lys Arg Ala
       1160                1165                1170
   gca gat tta cta gaa gct gaa gtt ctg att cat tct atg gtt cac      3564
   Ala Asp Leu Leu Glu Ala Glu Val Leu Ile His Ser Met Val His
       1175                1180                1185
   tct gat gat aac cag acc tct gta aca ttg gtc gac aac agg ata      3609
   Ser Asp Asp Asn Gln Thr Ser Val Thr Leu Val Asp Asn Arg Ile
       1190                1195                1200
   tgc cca aag caa ttc aca gca ttt gta atc aat aca ttt gaa aag      3654
   Cys Pro Lys Gln Phe Thr Ala Phe Val Ile Asn Thr Phe Glu Lys
       1205                1210                1215
   atc tgc cta aca ttc ggc tgt caa gct aat atg aaa aaa acc tat      3699
   Ile Cys Leu Thr Phe Gly Cys Gln Ala Asn Met Lys Lys Thr Tyr
       1220                1225                1230
   atc acc aac cat ata aaa gaa ttt gtt tca cta ttc aat tta tat      3744
   Ile Thr Asn His Ile Lys Glu Phe Val Ser Leu Phe Asn Leu Tyr
   1235 1240 1245
   gga gaa cca ttt tca att tat ggc aga ttc aca tta aca tct gtt 3789
   Gly Glu Pro Phe Ser Ile Tyr Gly Arg Phe Thr Leu Thr Ser Val
   1250 1255 1260
   gga gac tgc gca tac atc gga cca tac gaa gat ctc gca agc agg 3834
   Gly Asp Cys Ala Tyr Ile Gly Pro Tyr Glu Asp Leu Ala Ser Arg
   1265 1270 1275
   ata tca gca aca caa acc ggg ata aag cat gga tgc cca gct agc 3879
   Ile Ser Ala Thr Gln Thr Gly Ile Lys His Gly Cys Pro Ala Ser
   1280 1285 1290
   caa gcc tgg tta tcg att act atc gcc caa tgg atg act tac ttg 3924
   Gln Ala Trp Leu Ser Ile Thr Ile Ala Gln Trp Met Thr Tyr Leu
   1295 1300 1305
   aca tat aat atg ttg cca ggc caa caa aat gac ccc tgg cca ata 3969
   Thr Tyr Asn Met Leu Pro Gly Gln Gln Asn Asp Pro Trp Pro Ile
   1310 1315 1320
   ttg gaa atc gat aga aag caa ttg cca ctt gag ttg ggt gga tac 4014
   Leu Glu Ile Asp Arg Lys Gln Leu Pro Leu Glu Leu Gly Gly Tyr
   1325 1330 1335
   atc tct tgt cct cta tcc tta ata gct tta gct ggg cta gaa tcc 4059
   Ile Ser Cys Pro Leu Ser Leu Ile Ala Leu Ala Gly Leu Glu Ser
   1340 1345 1350
   ggg aac att gat ttc ctg atc aaa ttg gta aac aaa tat gtt gac 4104
   Gly Asn Ile Asp Phe Leu Ile Lys Leu Val Asn Lys Tyr Val Asp
   1355 1360 1365
   ccc aga ctt caa cgt gaa cct atc cag gat caa tgc act gat ata 4149
   Pro Arg Leu Gln Arg Glu Pro Ile Gln Asp Gln Cys Thr Asp Ile
   1370 1375 1380
   aaa aac tgg gac att tca agg ctt agc aaa gtt gaa ata ttt aag 4194
   Lys Asn Trp Asp Ile Ser Arg Leu Ser Lys Val Glu Ile Phe Lys
   1385 1390 1395
   cta aaa gtg ctg cgt tat tta gtt tta gat gca gag atg gat aat 4239
   Leu Lys Val Leu Arg Tyr Leu Val Leu Asp Ala Glu Met Asp Asn
   1400 1405 1410
   act gac att atg gga gaa acc agc gaa atg aga gga aga tcc tta 4284
   Thr Asp Ile Met Gly Glu Thr Ser Glu Met Arg Gly Arg Ser Leu
   1415 1420 1425
   cta aca ccc agg aag ttc aca aca gct ggc tcc ttg aga aaa ttg 4329
   Leu Thr Pro Arg Lys Phe Thr Thr Ala Gly Ser Leu Arg Lys Leu
   1430 1435 1440
   gta tct ttt gaa gat ttc caa gcg gtg caa gat act gaa caa ggt 4374
   Val Ser Phe Glu Asp Phe Gln Ala Val Gln Asp Thr Glu Gln Gly
   1445 1450 1455
   gtg gat gtt ata gtt gag tac atg ctg caa aac cca gca ttg ctc 4419
   Val Asp Val Ile Val Glu Tyr Met Leu Gln Asn Pro Ala Leu Leu
   1460 1465 1470
   gtg aca aag gga gaa aca aaa gat gac tat atg aac aca gtg ctc 4464
   Val Thr Lys Gly Glu Thr Lys Asp Asp Tyr Met Asn Thr Val Leu
       1475                1480                1485
   tat aga tat aac tca aag aaa ttt aaa gaa tca tta tcc att cag      4509
   Tyr Arg Tyr Asn Ser Lys Lys Phe Lys Glu Ser Leu Ser Ile Gln
       1490                1495                1500
   aat cct act cag ctc ttt ttg gag caa atc cta ttc tct cac aag      4554
   Asn Pro Thr Gln Leu Phe Leu Glu Gln Ile Leu Phe Ser His Lys
       1505                1510                1515
   cct att gtt gat tat aca ggt atc gca gaa aag ttc tca aat ctt      4599
   Pro Ile Val Asp Tyr Thr Gly Ile Ala Glu Lys Phe Ser Asn Leu
       1520                1525                1530
   aat gat gga acc atg ctt gaa gat aat ccg aca ata aaa ggt agg      4644
   Asn Asp Gly Thr Met Leu Glu Asp Asn Pro Thr Ile Lys Gly Arg
       1535                1540                1545
   atg acc ttt aca gat gct ttc gca atg atc aag aaa gat cta tca      4689
   Met Thr Phe Thr Asp Ala Phe Ala Met Ile Lys Lys Asp Leu Ser
       1550                1555                1560
   aca ctc aaa atc acc aat gag gac ctt gaa att gtg cta aaa ttt      4734
   Thr Leu Lys Ile Thr Asn Glu Asp Leu Glu Ile Val Leu Lys Phe
       1565                1570                1575
   ata att ttg aat gac cct ttg act gtg act gct gcc aac gcc cat      4779
   Ile Ile Leu Asn Asp Pro Leu Thr Val Thr Ala Ala Asn Ala His
       1580                1585                1590
   atc ttg cat gta ata ggt cat tca caa cct agg ctt gga tca aca      4824
   Ile Leu His Val Ile Gly His Ser Gln Pro Arg Leu Gly Ser Thr
       1595                1600                1605
   gct tgt gtt atg cct gaa ttt agg aat ttg aga atg ata aaa cat      4869
   Ala Cys Val Met Pro Glu Phe Arg Asn Leu Arg Met Ile Lys His
       1610                1615                1620
   tca cct gca cta gtc tta aag tgt tat agt aaa aac act ttg ttg      4914
   Ser Pro Ala Leu Val Leu Lys Cys Tyr Ser Lys Asn Thr Leu Leu
       1625                1630                1635
   ctg ccc ggg gtg caa gaa aga gac atg gaa agg gac ctt aac cat      4959
   Leu Pro Gly Val Gln Glu Arg Asp Met Glu Arg Asp Leu Asn His
       1640                1645                1650
   ctc aaa aca ttt att gag act aca aga tta gag cat aaa atg caa      5004
   Leu Lys Thr Phe Ile Glu Thr Thr Arg Leu Glu His Lys Met Gln
       1655                1660                1665
   gat agg atc aag aaa aat gaa gat ctc aaa aaa tcg aag gat atc      5049
   Asp Arg Ile Lys Lys Asn Glu Asp Leu Lys Lys Ser Lys Asp Ile
       1670                1675                1680
   tac ttt gaa atc aaa gag gta act aga ttt tat caa aca tgc tat      5094
   Tyr Phe Glu Ile Lys Glu Val Thr Arg Phe Tyr Gln Thr Cys Tyr
       1685                1690                1695
   gaa tat gtc aaa agt act gat tat aag gta aaa gta ttc ata cta      5139
   Glu Tyr Val Lys Ser Thr Asp Tyr Lys Val Lys Val Phe Ile Leu
       1700                1705                1710
   cca gcc aaa tgt atg act caa aca gat ttt tgc gct gta tta cag          5184
   Pro Ala Lys Cys Met Thr Gln Thr Asp Phe Cys Ala Val Leu Gln
       1715                1720                1725
   ggt tca ttg tta gaa gat aaa aag tgg aaa tta ata caa cac ctc          5229
   Gly Ser Leu Leu Glu Asp Lys Lys Trp Lys Leu Ile Gln His Leu
       1730                1735                1740
   aga cct gtt act gca ggt gga cat aaa gga gag gtt cag ccg gta          5274
   Arg Pro Val Thr Ala Gly Gly His Lys Gly Glu Val Gln Pro Val
       1745                1750                1755
   gtt tcc atg gat ttt aaa cta gca aga gag gcc gta aat tta tta          5319
   Val Ser Met Asp Phe Lys Leu Ala Arg Glu Ala Val Asn Leu Leu
       1760                1765                1770
   tgc ttt ttt act gat tca ttt ata gac tct aga tat aga aga gac          5364
   Cys Phe Phe Thr Asp Ser Phe Ile Asp Ser Arg Tyr Arg Arg Asp
       1775                1780                1785
   ttt ctt cta aaa gcg tgc gta gaa atg gct tat aaa gat agg cct          5409
   Phe Leu Leu Lys Ala Cys Val Glu Met Ala Tyr Lys Asp Arg Pro
       1790                1795                1800
   atg gtt aca ttg ata gaa gaa ata aag aag tca aag aac cga aca          5454
   Met Val Thr Leu Ile Glu Glu Ile Lys Lys Ser Lys Asn Arg Thr
       1805                1810                1815
   tct ttc ttg cca ttc ttg tat aga att gga gag tta act caa gaa          5499
   Ser Phe Leu Pro Phe Leu Tyr Arg Ile Gly Glu Leu Thr Gln Glu
       1820                1825                1830
   gac cta gat gca tat gat gct caa aaa gcg gga ggg aga gta acc          5544
   Asp Leu Asp Ala Tyr Asp Ala Gln Lys Ala Gly Gly Arg Val Thr
       1835                1840                1845
   tgg aat cac tgg caa tct aca aga agc tta aac atg ggg aag ata          5589
   Trp Asn His Trp Gln Ser Thr Arg Ser Leu Asn Met Gly Lys Ile
       1850                1855                1860
   gat ttc caa ata gat aca cat aaa agt cat ctc cat att att gga          5634
   Asp Phe Gln Ile Asp Thr His Lys Ser His Leu His Ile Ile Gly
       1865                1870                1875
   gaa gat caa caa tta gta ata gca gag cta aaa att cct aaa ttg          5679
   Glu Asp Gln Gln Leu Val Ile Ala Glu Leu Lys Ile Pro Lys Leu
       1880                1885                1890
   aca gca aat gct atc act caa gca gga tat aga ctt tta aat gaa          5724
   Thr Ala Asn Ala Ile Thr Gln Ala Gly Tyr Arg Leu Leu Asn Glu
       1895                1900                1905
   aag cat ggg ttt cgg ttt gaa aca atg aat gag gtt tta gtc cag          5769
   Lys His Gly Phe Arg Phe Glu Thr Met Asn Glu Val Leu Val Gln
       1910                1915                1920
   cca aga aaa tat tat ata aca tac cag atg aaa tct aaa aag caa          5814
   Pro Arg Lys Tyr Tyr Ile Thr Tyr Gln Met Lys Ser Lys Lys Gln
       1925                1930                1935
   tat gtc tat aaa atc ttt gac aca gca aca ttg cta tat gaa aga          5859
   Tyr Val Tyr Lys Ile Phe Asp Thr Ala Thr Leu Leu Tyr Glu Arg
       1940                1945                1950
   aga aac  ata aag caa tac aca  ggc cga gaa gca aat  ccc ata gta        5904
   Arg Asn  Ile Lys Gln Tyr Thr  Gly Arg Glu Ala Asn  Pro Ile Val
       1955                 1960                 1965
   gca gtc  tgc gaa gtt gtt cca  atg gag gag gag gaa  act ccc gct        5949
   Ala Val  Cys Glu Val Val Pro  Met Glu Glu Glu Glu  Thr Pro Ala
       1970                 1975                 1980
   tta agc  tta agt gac ata gaa  atg tat aat gct gac  aat gaa tca        5994
   Leu Ser  Leu Ser Asp Ile Glu  Met Tyr Asn Ala Asp  Asn Glu Ser
       1985                 1990                 1995
   gtt agc  att tta aaa act tca  ttc aat gag agt gcg  aag atg aga        6039
   Val Ser  Ile Leu Lys Thr Ser  Phe Asn Glu Ser Ala  Lys Met Arg
       2000                 2005                 2010
   cgt gca  aat ttg tcc aaa atg  caa tat ttt gag act  att ggc gga        6084
   Arg Ala  Asn Leu Ser Lys Met  Gln Tyr Phe Glu Thr  Ile Gly Gly
       2015                 2020                 2025
   tca atg  ttg aaa att cga ttc  atc aat gtc aac gca  cta atg aaa        6129
   Ser Met  Leu Lys Ile Arg Phe  Ile Asn Val Asn Ala  Leu Met Lys
       2030                 2035                 2040
   gac aca  act cta atg tcc ctt  aac tat gac aat ttg  gta aat gtc        6174
   Asp Thr  Thr Leu Met Ser Leu  Asn Tyr Asp Asn Leu  Val Asn Val
       2045                 2050                 2055
   gac ctg  gct aga ttt agc aag  tta ttg gac tgt gac  ggt gat aca        6219
   Asp Leu  Ala Arg Phe Ser Lys  Leu Leu Asp Cys Asp  Gly Asp Thr
       2060                 2065                 2070
   gaa agt  gtt gaa gat gga ata  ttg tgc ttt tct gat  gac cca atg        6264
   Glu Ser  Val Glu Asp Gly Ile  Leu Cys Phe Ser Asp  Asp Pro Met
       2075                 2080                 2085
   gaa caa  acc aca aca gaa gag  att gac gcg atc cct  ctc ttc aaa        6309
   Glu Gln  Thr Thr Thr Glu Glu  Ile Asp Ala Ile Pro  Leu Phe Lys
       2090                 2095                 2100
   gta agc  tac aag ata cct aca  cca gct aga tta aca  tac aag aat        6354
   Val Ser  Tyr Lys Ile Pro Thr  Pro Ala Arg Leu Thr  Tyr Lys Asn
       2105                 2110                 2115
   gcc att  aga gaa ctg tta agg  agg gaa ata aca agg  ttt atg gat        6399
   Ala Ile  Arg Glu Leu Leu Arg  Arg Glu Ile Thr Arg  Phe Met Asp
       2120                 2125                 2130
   gct ttt  acc ttt gtt gga cct  tct ttt gat tca cca  gaa aat atg        6444
   Ala Phe  Thr Phe Val Gly Pro  Ser Phe Asp Ser Pro  Glu Asn Met
       2135                 2140                 2145
   ggg ttc  ata tgc aat ata tgt  tgt ata ata caa ctc  ctc cat aca        6489
   Gly Phe  Ile Cys Asn Ile Cys  Cys Ile Ile Gln Leu  Leu His Thr
       2150                 2155                 2160
   aac gaa  tgg tct act ata atg  cta aat tgt att cat  atc tgc atg        6534
   Asn Glu  Trp Ser Thr Ile Met  Leu Asn Cys Ile His  Ile Cys Met
       2165                 2170                 2175
   att tat  ctg gag aaa gac ata  agg ttt cat aaa ttt  tta cta aat        6579
   Ile Tyr  Leu Glu Lys Asp Ile  Arg Phe His Lys Phe  Leu Leu Asn
       2180                2185                2190
   aga ata  ttc ttt gag gga ttg  aat cct gca tct ggg  aaa ttg gat         6624
   Arg Ile  Phe Phe Glu Gly Leu  Asn Pro Ala Ser Gly  Lys Leu Asp
       2195                2200                2205
   tac aaa  aaa atc aaa gaa ttt  gta atc agc ctg aag  gat aga gag         6669
   Tyr Lys  Lys Ile Lys Glu Phe  Val Ile Ser Leu Lys  Asp Arg Glu
       2210                2215                2220
   atg gct  cct tgg aat gag ata  ttt tca aag ttc aag  caa aaa tgt         6714
   Met Ala  Pro Trp Asn Glu Ile  Phe Ser Lys Phe Lys  Gln Lys Cys
       2225                2230                2235
   gtc aca  ttg cta gac aaa gaa  ata aca aga ata gaa  caa aac aca         6759
   Val Thr  Leu Leu Asp Lys Glu  Ile Thr Arg Ile Glu  Gln Asn Thr
       2240                2245                2250
   aac ttt  gaa aat ata cta gct  tta gat gaa agc aca  gat gat cag         6804
   Asn Phe  Glu Asn Ile Leu Ala  Leu Asp Glu Ser Thr  Asp Asp Gln
       2255                2260                2265
   tct gga  ttc ctc ttg ttt gaa  tag                                      6828
   Ser Gly  Phe Leu Leu Phe Glu
       2270                2275
<210> 6
   <211> 2275
   <212> PRT
   <213> Moissiacense virus
<400> 6
   Met Leu Asp Gln Glu Asn Glu Gln Ala Leu Asn Arg Leu Ala Ala Ala
   1               5                   10                  15
   Val Ala Arg Ala Asn Glu Pro Ala Glu Gly Lys Glu Ala Tyr Asn Ala
               20                  25                  30
   Ile Leu Ser Arg Arg His Asp Tyr Phe Gly Gln Val Phe Cys Glu Thr
           35                  40                  45
   Ile Gly Ile Pro Tyr Arg Asn Asp Val Arg Leu Glu Asp Ile Leu His
       50                  55                  60
   Glu Cys Asp Gln Thr Val Asp Thr Leu Thr Asn Lys Ile Pro Asn Ile
   65                  70                  75                  80
   Thr Pro Asp Asn Phe Glu Phe Arg Asp Asn Ile Leu Thr Ile Ile Asp
                   85                  90                  95
   Tyr Lys Val Ala Val Ser Asp Glu Ser Ala Asp Tyr Thr Arg Glu Lys
               100                 105                 110
   Tyr Glu Asn Ala Val Asp Ala Ile Lys His Leu Val Asp Phe Pro Ile
   115 120 125
   Arg Val Cys Ile Val Arg Ile His Pro Tyr Thr Arg Gln Val Thr Tyr
   130 135 140
   Ser Asp Asp Asn Phe Met Arg Arg Phe Ala His Val Val Tyr Pro Val
   145 150 155 160
   Asp Phe Thr Arg Tyr Thr Gln Leu Thr Glu Glu Leu Lys Glu Lys Phe
   165 170 175
   Ala Asp Ser Pro Glu Phe Met Asp Met Ile Ala Tyr Gly Asp Val Thr
   180 185 190
   Phe Thr Ala Asn Trp Cys Gln Asn Gly Cys Pro Glu Leu Tyr Gly His
   195 200 205
   Pro Ile Phe Lys Glu Phe Leu Glu Ser Met Pro Leu Lys Tyr Gln Lys
   210 215 220
   Leu Phe Glu Lys Ser Leu Leu Ser Ser Cys Tyr Thr Ser Glu Arg Trp
   225 230 235 240
   Asp Ser Asn Leu Lys Glu Val Lys Gly Ala Thr Lys Asp Glu Tyr Glu
   245 250 255
   Asp Phe Ile Ser Arg Ser Ala Arg Glu Leu Tyr Thr Thr Ser Pro Asp
   260 265 270
   Tyr Ser Glu Pro Ser Arg Glu Glu Ile Leu Lys Gly Trp Glu Glu Met
   275 280 285
   Thr Glu Arg Val Lys Glu Gln Arg Glu Val Ser Ser Asn Ile Asn Asp
   290 295 300
   Gln Lys Pro Ser Ala His Phe Ile Trp Ala Glu Asn Asn Pro Glu Leu
   305 310 315 320
   Pro Thr Ala Asn Ser Ala Lys Ile Cys Ala Ile Ser Lys Leu Leu Gln
   325 330 335
   Ser Ile Asn Ile Glu Ser Ser Phe Ala Glu Asp Phe Lys Arg Leu Gly
   340 345 350
   Lys Leu Phe Asp Phe Ser Glu Asp Ile Pro Gly Tyr Glu Arg His Cys
   355 360 365
   Glu Gln Leu Lys Arg Gln Ala Arg Ser Asn Pro Gly Gln Ile Lys Asn
       370                 375                 380
   Lys Lys Leu Asp Pro Lys Lys Ile Gly Asn Ala Leu Val Leu Trp Glu
   385                 390                 395                 400
   Gln Gln Phe Leu Leu Ser Ala Asp Glu Met Gly Lys Glu Asn Lys Leu
                   405                 410                 415
   His Leu Leu Lys Asp Phe Phe Gly Ile Gly Lys His Lys Gln Phe Ala
               420                 425                 430
   Lys Lys Gly Leu Asp Asp Thr Glu Thr Asp Lys Pro Thr Ile Leu Asp
           435                 440                 445
   Phe Asn Asp Thr Glu Val Lys Arg Ser Ala Glu Leu Met Met Gln Arg
       450                 455                 460
   Thr Lys Leu Tyr Leu Ser Gly Arg Ser Ser Leu Asn Lys Glu His Pro
   465                 470                 475                 480
   Val Ala Ser Glu Tyr Leu Asn Glu Ile Lys Lys Cys Asn Asp Glu Thr
                   485                 490                 495
   His Asn Val Leu Asn Ala Ile Phe Gln Thr Lys Tyr Trp Gln Cys Ile
               500                 505                 510
   Thr Asp Ile Ser Met Leu Met Lys Asn Met Leu Ser Val Ser Gln Tyr
           515                 520                 525
   Asn Arg Asn Asn Thr Phe Arg Ile Ala Thr Cys Ala Asn Asn Ala Leu
       530                 535                 540
   Tyr Gly Leu Ile Phe Pro Ser Ala Asp Ile Lys Thr Lys Asp Ala Ser
   545                 550                 555                 560
   Ile Val Tyr Cys Ile Ile Ala Ile His Lys Asp Glu Lys Ser Leu Phe
                   565                 570                 575
   Asn Pro Gly Ala Leu His Ala Thr Phe Lys Val Pro Gly Gly Phe Leu
               580                 585                 590
   Ser Ile Ser Lys Ser Met Arg Leu Asp Lys Lys Arg Cys His Arg Ile
           595                 600                 605
   Val Thr Ser Pro Gly Ile Phe Leu Thr Ile Ala Thr Met Leu Lys Gly
       610                 615                 620
   Asp Asn Pro Thr Ile Ser Leu Asn Asp Val Leu Asn Phe Ser Ile Phe
   625 630 635 640
   Ala Ser Leu Asn Ile Thr Lys Ser Met Leu Ser Leu Thr Glu Pro Ser
   645 650 655
   Arg Tyr Met Ile Met Asn Ser Leu Ala Val Ser Ser His Val Arg Glu
   660 665 670
   Tyr Ile Ser Glu Lys Phe Ser Pro Tyr Thr Lys Thr Leu Phe Ser Val
   675 680 685
   His Met Val Glu Lys Ile Lys Ala Gly Cys Leu Asn Ala Phe Lys Gln
   690 695 700
   Arg Glu Lys Ile Lys Thr Arg Asp Ile Tyr Leu Thr Asp Ile Asp Ile
   705 710 715 720
   Thr Gln Lys Gly Ile Gln Asp Asn Arg Asp Leu Cys Ser Ile Trp Phe
   725 730 735
   Asp Gly Asn Val Thr Leu Lys Glu Tyr Leu Asn Gln Ile Tyr Ile Val
   740 745 750
   Phe Tyr Leu Asn Pro Lys Ala Leu His Glu Lys His His Val Met Ile
   755 760 765
   Asp Leu Leu Lys Thr Val Ile Glu Ile Glu Leu Asp Gln Arg Glu Asn
   770 775 780
   Val Pro Gln Pro Trp Ser Asp Val Pro Lys Lys Gln Thr Val Asn Leu
   785 790 795 800
   Pro Ile Leu Val His Ser Leu Ala Lys Asn Leu Leu Leu Asp Thr Ser
   805 810 815
   Phe Ala His Phe Leu Arg Ser Arg Ile Glu Ser Arg Asn Asn Phe Arg
   820 825 830
   Arg Thr Phe Ala Thr Ile Ser Thr Phe Thr Ser Ser Lys Ser Cys Leu
   835 840 845
   Lys Thr Gly Asn Phe His Glu Phe Lys Ile Lys Gln Cys Gln Arg Leu
   850 855 860
   Lys Lys Val Asn Thr Ser Glu Ala Lys Lys His Arg Ile Ala Asn Ala
   865 870 875 880
   Met Phe Val Glu Glu Gln Asp Gln His Leu Glu Val Glu His Ala Asn
   885 890 895
   Tyr Glu Leu Met Arg Lys Ala Ile Pro Asp Tyr Val Asp Val Leu Ser
   900 905 910
   Thr Lys Val Phe Asp Arg Leu Tyr Glu Leu Tyr Lys Thr Gly Glu Ile
   915 920 925
   Asp Asp Ser Pro Val Ile His Gln Ile Phe Ala Thr Met Lys Ser His
   930 935 940
   Thr Lys Phe Tyr Phe Thr Tyr Phe Asn Lys Gly Gln Lys Thr Ala Lys
   945 950 955 960
   Asp Arg Glu Ile Phe Val Gly Glu Tyr Glu Ala Lys Met Cys Met Tyr
   965 970 975
   Leu Val Glu Arg Ile Tyr Lys Glu Arg Ser Lys Val Asn Pro Asp Glu
   980 985 990
   Met Ile Ser Glu Pro Gly Asp Gly Lys Leu Arg Val Leu Glu Arg Lys
   995 1000 1005
   Ala Glu Gln Glu Ile Arg Phe Met Val Glu Lys Met Lys Ala Lys
   1010 1015 1020
   Asn Asp Glu Ile Asn Lys Glu Ile Glu Asp Ala Lys Asp Asn Tyr
   1025 1030 1035
   Val Leu Asn Leu Gly Lys Ile Ala Ala Leu Gln Ala Gln Lys Tyr
   1040 1045 1050
   Lys Ala Leu Lys Leu Glu Ile Asn Ala Asp Met Ser Lys Trp Ser
   1055 1060 1065
   Ala Gln Asp Val Phe Phe Lys Tyr Phe Trp Ser Val Cys Leu Asp
   1070 1075 1080
   Pro Ile Leu Tyr Pro Glu Glu Lys Glu His Ile Leu Tyr Phe Phe
   1085 1090 1095
   Cys Asn Tyr Met Gln Lys Glu Leu Val Ile Pro Asp Glu Val Ile
   1100 1105 1110
   Cys Asn Ile Leu Asp Gln Arg Lys Ala Tyr Pro Asn Asp Ile Ile
   1115 1120 1125
   Ser Gln Ile Thr Asn Gly Leu Thr Thr Asn His Phe Asn Val Lys
   1130 1135 1140
   Arg Asn Trp Leu Gln Gly Asn Phe Asn Tyr Met Ser Ser Tyr Ala
   1145 1150 1155
   His Ser Thr Ala Met Ser Val Phe Lys Asp Val Ile Lys Arg Ala
   1160 1165 1170
   Ala Asp Leu Leu Glu Ala Glu Val Leu Ile His Ser Met Val His
   1175 1180 1185
   Ser Asp Asp Asn Gln Thr Ser Val Thr Leu Val Asp Asn Arg Ile
   1190 1195 1200
   Cys Pro Lys Gln Phe Thr Ala Phe Val Ile Asn Thr Phe Glu Lys
   1205 1210 1215
   Ile Cys Leu Thr Phe Gly Cys Gln Ala Asn Met Lys Lys Thr Tyr
   1220 1225 1230
   Ile Thr Asn His Ile Lys Glu Phe Val Ser Leu Phe Asn Leu Tyr
   1235 1240 1245
   Gly Glu Pro Phe Ser Ile Tyr Gly Arg Phe Thr Leu Thr Ser Val
   1250 1255 1260
   Gly Asp Cys Ala Tyr Ile Gly Pro Tyr Glu Asp Leu Ala Ser Arg
   1265 1270 1275
   Ile Ser Ala Thr Gln Thr Gly Ile Lys His Gly Cys Pro Ala Ser
   1280 1285 1290
   Gln Ala Trp Leu Ser Ile Thr Ile Ala Gln Trp Met Thr Tyr Leu
   1295 1300 1305
   Thr Tyr Asn Met Leu Pro Gly Gln Gln Asn Asp Pro Trp Pro Ile
   1310 1315 1320
   Leu Glu Ile Asp Arg Lys Gln Leu Pro Leu Glu Leu Gly Gly Tyr
   1325 1330 1335
   Ile Ser Cys Pro Leu Ser Leu Ile Ala Leu Ala Gly Leu Glu Ser
   1340 1345 1350
   Gly Asn Ile Asp Phe Leu Ile Lys Leu Val Asn Lys Tyr Val Asp
   1355 1360 1365
   Pro Arg Leu Gln Arg Glu Pro Ile Gln Asp Gln Cys Thr Asp Ile
   1370 1375 1380
   Lys Asn Trp Asp Ile Ser Arg Leu Ser Lys Val Glu Ile Phe Lys
   1385 1390 1395
   Leu Lys Val Leu Arg Tyr Leu Val Leu Asp Ala Glu Met Asp Asn
   1400 1405 1410
   Thr Asp Ile Met Gly Glu Thr Ser Glu Met Arg Gly Arg Ser Leu
   1415 1420 1425
   Leu Thr Pro Arg Lys Phe Thr Thr Ala Gly Ser Leu Arg Lys Leu
   1430 1435 1440
   Val Ser Phe Glu Asp Phe Gln Ala Val Gln Asp Thr Glu Gln Gly
   1445 1450 1455
   Val Asp Val Ile Val Glu Tyr Met Leu Gln Asn Pro Ala Leu Leu
   1460 1465 1470
   Val Thr Lys Gly Glu Thr Lys Asp Asp Tyr Met Asn Thr Val Leu
   1475 1480 1485
   Tyr Arg Tyr Asn Ser Lys Lys Phe Lys Glu Ser Leu Ser Ile Gln
   1490 1495 1500
   Asn Pro Thr Gln Leu Phe Leu Glu Gln Ile Leu Phe Ser His Lys
   1505 1510 1515
   Pro Ile Val Asp Tyr Thr Gly Ile Ala Glu Lys Phe Ser Asn Leu
   1520 1525 1530
   Asn Asp Gly Thr Met Leu Glu Asp Asn Pro Thr Ile Lys Gly Arg
   1535 1540 1545
   Met Thr Phe Thr Asp Ala Phe Ala Met Ile Lys Lys Asp Leu Ser
   1550 1555 1560
   Thr Leu Lys Ile Thr Asn Glu Asp Leu Glu Ile Val Leu Lys Phe
   1565 1570 1575
   Ile Ile Leu Asn Asp Pro Leu Thr Val Thr Ala Ala Asn Ala His
   1580 1585 1590
   Ile Leu His Val Ile Gly His Ser Gln Pro Arg Leu Gly Ser Thr
       1595                1600                1605
   Ala Cys Val Met Pro Glu Phe Arg Asn Leu Arg Met Ile Lys His
       1610                1615                1620
   Ser Pro Ala Leu Val Leu Lys Cys Tyr Ser Lys Asn Thr Leu Leu
       1625                1630                1635
   Leu Pro Gly Val Gln Glu Arg Asp Met Glu Arg Asp Leu Asn His
       1640                1645                1650
   Leu Lys Thr Phe Ile Glu Thr Thr Arg Leu Glu His Lys Met Gln
       1655                1660                1665
   Asp Arg Ile Lys Lys Asn Glu Asp Leu Lys Lys Ser Lys Asp Ile
       1670                1675                1680
   Tyr Phe Glu Ile Lys Glu Val Thr Arg Phe Tyr Gln Thr Cys Tyr
       1685                1690                1695
   Glu Tyr Val Lys Ser Thr Asp Tyr Lys Val Lys Val Phe Ile Leu
       1700                1705                1710
   Pro Ala Lys Cys Met Thr Gln Thr Asp Phe Cys Ala Val Leu Gln
       1715                1720                1725
   Gly Ser Leu Leu Glu Asp Lys Lys Trp Lys Leu Ile Gln His Leu
       1730                1735                1740
   Arg Pro Val Thr Ala Gly Gly His Lys Gly Glu Val Gln Pro Val
       1745                1750                1755
   Val Ser Met Asp Phe Lys Leu Ala Arg Glu Ala Val Asn Leu Leu
       1760                1765                1770
   Cys Phe Phe Thr Asp Ser Phe Ile Asp Ser Arg Tyr Arg Arg Asp
       1775                1780                1785
   Phe Leu Leu Lys Ala Cys Val Glu Met Ala Tyr Lys Asp Arg Pro
       1790                1795                1800
   Met Val Thr Leu Ile Glu Glu Ile Lys Lys Ser Lys Asn Arg Thr
       1805                1810                1815
   Ser Phe Leu Pro Phe Leu Tyr Arg Ile Gly Glu Leu Thr Gln Glu
       1820                1825                1830
   Asp Leu  Asp Ala Tyr Asp Ala  Gln Lys Ala Gly Gly  Arg Val Thr
       1835                 1840                 1845
   Trp Asn  His Trp Gln Ser Thr  Arg Ser Leu Asn Met  Gly Lys Ile
       1850                 1855                 1860
   Asp Phe  Gln Ile Asp Thr His  Lys Ser His Leu His  Ile Ile Gly
       1865                 1870                 1875
   Glu Asp  Gln Gln Leu Val Ile  Ala Glu Leu Lys Ile  Pro Lys Leu
       1880                 1885                 1890
   Thr Ala  Asn Ala Ile Thr Gln  Ala Gly Tyr Arg Leu  Leu Asn Glu
       1895                 1900                 1905
   Lys His  Gly Phe Arg Phe Glu  Thr Met Asn Glu Val  Leu Val Gln
       1910                 1915                 1920
   Pro Arg  Lys Tyr Tyr Ile Thr  Tyr Gln Met Lys Ser  Lys Lys Gln
       1925                 1930                 1935
   Tyr Val  Tyr Lys Ile Phe Asp  Thr Ala Thr Leu Leu  Tyr Glu Arg
       1940                 1945                 1950
   Arg Asn  Ile Lys Gln Tyr Thr  Gly Arg Glu Ala Asn  Pro Ile Val
       1955                 1960                 1965
   Ala Val  Cys Glu Val Val Pro  Met Glu Glu Glu Glu  Thr Pro Ala
       1970                 1975                 1980
   Leu Ser  Leu Ser Asp Ile Glu  Met Tyr Asn Ala Asp  Asn Glu Ser
       1985                 1990                 1995
   Val Ser  Ile Leu Lys Thr Ser  Phe Asn Glu Ser Ala  Lys Met Arg
       2000                 2005                 2010
   Arg Ala  Asn Leu Ser Lys Met  Gln Tyr Phe Glu Thr  Ile Gly Gly
       2015                 2020                 2025
   Ser Met  Leu Lys Ile Arg Phe  Ile Asn Val Asn Ala  Leu Met Lys
       2030                 2035                 2040
   Asp Thr  Thr Leu Met Ser Leu  Asn Tyr Asp Asn Leu  Val Asn Val
       2045                 2050                 2055
   Asp Leu  Ala Arg Phe Ser Lys  Leu Leu Asp Cys Asp  Gly Asp Thr
   2060 2065 2070
   Glu Ser Val Glu Asp Gly Ile Leu Cys Phe Ser Asp Asp Pro Met
   2075 2080 2085
   Glu Gln Thr Thr Thr Glu Glu Ile Asp Ala Ile Pro Leu Phe Lys
   2090 2095 2100
   Val Ser Tyr Lys Ile Pro Thr Pro Ala Arg Leu Thr Tyr Lys Asn
   2105 2110 2115
   Ala Ile Arg Glu Leu Leu Arg Arg Glu Ile Thr Arg Phe Met Asp
   2120 2125 2130
   Ala Phe Thr Phe Val Gly Pro Ser Phe Asp Ser Pro Glu Asn Met
   2135 2140 2145
   Gly Phe Ile Cys Asn Ile Cys Cys Ile Ile Gln Leu Leu His Thr
   2150 2155 2160
   Asn Glu Trp Ser Thr Ile Met Leu Asn Cys Ile His Ile Cys Met
   2165 2170 2175
   Ile Tyr Leu Glu Lys Asp Ile Arg Phe His Lys Phe Leu Leu Asn
   2180 2185 2190
   Arg Ile Phe Phe Glu Gly Leu Asn Pro Ala Ser Gly Lys Leu Asp
   2195 2200 2205
   Tyr Lys Lys Ile Lys Glu Phe Val Ile Ser Leu Lys Asp Arg Glu
   2210 2215 2220
   Met Ala Pro Trp Asn Glu Ile Phe Ser Lys Phe Lys Gln Lys Cys
   2225 2230 2235
   Val Thr Leu Leu Asp Lys Glu Ile Thr Arg Ile Glu Gln Asn Thr
   2240 2245 2250
   Asn Phe Glu Asn Ile Leu Ala Leu Asp Glu Ser Thr Asp Asp Gln
   2255 2260 2265
   Ser Gly Phe Leu Leu Phe Glu
   2270 2275
<210> 7
   <211> 316
   <212> DNA
   <213> artificial sequence
<220>
   <223> synthetic polynucleotide (S segment target sequence (partial))
<400> 7
   aaacgcagaa ctgggtagca ctagcattta tccaggatgg ctgattctaa gctcacatac        60
   gaggatacac aggatgttag tagaagtact tataacccag cagatgaata cgatacgttt       120
   gtgggtgtct acagagaatt tctcacaatt gataacatta gaatcttctt catcaacgct       180
   ccaaaggcca aaactgcgat ggctaaactc aagaccgaga cagttgaagt tacatttgga       240
   acactcgttg tcaagctggt taataaccat agaattggac aggctcagcg tgaggttgcg       300
   gacgatgagc taactc                                                       316
<210> 8
   <211> 425
   <212> DNA
   <213> artificial sequence
<220>
   <223> synthetic polynucleotide (M segment target sequence)
<400> 8
   acaggacaaa tagcactaaa ggtcttgttg ggagacttta actacaagca gtatgctgca        60
   aatgtagagg ctgacataga agcaaattgc gttgggtgta caaattgtat aaagggaata       120
   acatgtgaaa taaaggtgaa aacagaaatt gaagcatcat gtaaggtaga gcccccatgc       180
   ccctcttaca caagtagaat tataataaaa ccaggtagag acacccacac attattgatg       240
   aagtgtgaca aaaaaataac gactggtgaa ctagcaataa aaatatgtaa tatgcaaatc       300
   caggctcatc tttcaacaat agagagcaat gaccaactag aattgtcgac tggggatcaa       360
   tcaacatata tacatgaaga agacctaagg tgttcaacct ggatctgcaw wgtgagagac       420
   gaagg                                                                   425
<210> 9
   <211> 457
   <212> DNA
   <213> artificial sequence
<220>
   <223> synthetic polynucleotide (L segment target sequence)
<400> 9
   agaattggtt atcccagatg aggttatctg caatatccta gatcagagga aagcataccc 60
   taatgacata attagtcaga tcacaaatgg cttgacaacc aaccatttca atgtaaagag 120
   aaactggcta caaggcaact tcaattacat gtccagctat gctcacagta cagcaatgtc 180
   tgtctttaaa gatgtgataa agcgtgctgc agatttacta gaagctgaag ttctgattca 240
   ttctatggtt cactctgatg ataaccagac ctctgtaaca ttggtcgaca acaggatatg 300
   cccaaagcaa ttcacagcat ttgtaatcaa tacatttgaa aagatctgcc taacattcgg 360
   ctgtcaagct aatatgaaaa aaacctatat caccaaccat ataaaagaat ttgtttcact 420
   attcaattta tatggagaac cattttcaat ttatggc 457
<210> 10
   <211> 20
   <212> DNA
   <213> artificial sequence
<220>
   <223> synthetic oligonucleotide (BunyaCladeS_Fwl)
<400> 10
   aaacgcagaa ctgggtagca 20
<210> 11
   <211> 20
   <212> DNA
   <213> artificial sequence
<220>
   <223> synthetic oligonucleotide (BunyaCladeS_Rvl)
<400> 11
   gagttagctc atcgtccgca 20
<210> 12
   <211> 23
   <212> DNA
   <213> artificial sequence
<220>
   <223> synthetic oligonucleotide (BunyaCladeM_Fwl)
<400> 12
   acaggrcaaa tagcactaaa ggt 23
<210> 13
   <211> 21
   <212> DNA
   <213> artificial sequence
<220>
   <223> synthetic oligonucleotide (BunyaCladeM_Rvl)
<400> 13
   ccttcrtctc tcactttgca g 21
<210> 14
   <211> 24
   <212> DNA
   <213> artificial sequence
<220>
   <223> synthetic oligonucleotide (BunyaCladeL_Fwl)
<400> 14
   agaattggtt atcccagatg aggt 24
<210> 15
   <211> 25
   <212> DNA
   <213> synthetic oligonucleotide (BunyaCladeL_Rvl)
<400> 15
   gccataaatt gaaaatggtt ctcca 25
<210> 16
   <211> 23
   <212> DNA
   <213> artificial sequence
<220>
   <223> synthetic oligonucleotide (BunyaUmbreM_Fw1)
<400> 16
   ggatagtctc agattggacc aca 23
<210> 17
   <211> 23
   <212> DNA
   <213> artificial sequence
<220>
   <223> synthetic oligonucleotide (BunyaUmbreM_Rv1)
<400> 17
   tctgtgggtt ttcattgcat ctg 23
<210> 18
   <211> 22
   <212> DNA
   <213> artificial sequence
<220>
   <223> synthetic oligonucleotide (BunyaUmbreM_Fw2)
<400> 18
   gcttaccacc ctttcacaaa gt 22
<210> 19
   <211> 20
   <212> DNA
   <213> artificial sequence
<220>
   <223> synthetic oligonucleotide (BunyaUmbreM_Rv2)
<400> 19
   gctgcggcag tcagcattat 20
<210> 20
   <211> 22
   <212> DNA
   <213> artificial sequence
<220>
   <223> synthetic oligonucleotide (BunyaUmbreM_Fw3)
<400> 20
   acgaaatcat tgctctgcca ac 22
<210> 21
   <211> 20
   <212> DNA
   <213> artificial sequence
<220>
   <223> synthetic oligonucleotide (BunyaUmbreM_Rv3)
<400> 21
   tgttgtgctt ggctgcattt 20
<210> 22
   <211> 22
   <212> DNA
   <213> artificial sequence
<220>
   <223> synthetic oligonucleotide (BunyaUmbreM_Fw4)
<400> 22
   aaggaatctg agctaggaca ag 22
<210> 23
   <211> 23
   <212> DNA
   <213> artificial sequence
<220>
   <223> synthetic oligonucleotide (BunyaUmbreM_Rv4)
<400> 23
   atctgttgtt agcacacaat gtt 23
<210> 24
   <211> 20
   <212> DNA
   <213> artificial sequence
<220>
   <223> synthetic oligonucleotide (BunyaUmbreM_Fw5)
<400> 24
   ttgcaaagtg ctcgcgaatg 20
<210> 25
   <211> 20
   <212> DNA
   <213> artificial sequence
<220>
   <223> synthetic oligonucleotide (BunyaUmbreM_Rv5)
<400> 25
   agccccaatt gctggttctt 20
<210> 26
   <211> 19
   <212> DNA
   <213> artificial sequence
<220>
   <223> synthetic oligonucleotide (BunyaUmbreM_Fw6)
<400> 26
   gagcactgca cagggacat 19
<210> 27
   <211> 20
   <212> DNA
   <213> artificial sequence
<220>
   <223> syntehtic oligonucleotide (BunyaUmbreM_Rv6)
<400> 27
   cttgtgtaag aggggcatgg 20
<210> 28
   <211> 19
   <212> DNA
   <213> artificial sequence
<220>
   <223> synthetic oligonucleotide (BunyaUmbreM5_Fw)
<400> 28
   gtttgagatt accatcaag 19
<210> 29
   <211> 23
   <212> DNA
   <213> artificial sequence
<220>
   <223> synthetic oligonucleotide (BunyaUmbreM5_Rv)
<400> 29
   tgtggtccaa tctgagacta tcc 23
<210> 30
   <211> 21
   <212> DNA
   <213> artificial sequence
<220>
   <223> synthetic oligonucleotide (BunyaUmbreM3_Fw)
<400> 30
   ctgcaaagtg agagaygaag g 21
<210> 31
   <211> 17
   <212> DNA
   <213> artificial sequence
<220>
   <223> synthetic oligonucleotide (BunyaUmbreM3_Rv)
<400> 31
   catgactata ccattga 17
<210> 32
   <211> 22
   <212> DNA
   <213> artificial sequence
<220>
   <223> synthetic oligonucleotide (BunyaUmbreL3_Fw)
<400> 32
   tcctgcatct gggaaattgg at 22
<210> 33
   <211> 24
   <212> DNA
   <213> artificial sequence
<220>
   <223> synthetic oligonucleotide (BunyaUmbreL3_Rv)
<400> 33
   ctagagagaa tagatgaact cttg 24
<210> 34
   <211> 20
   <212> DNA
   <213> artificial sequence
<220>
   <223> synthetic oligonucleotide (BunyaUmbreL_Fw1)
<400> 34
   acaggaacga tgttcggcta 20
<210> 35
   <211> 21
   <212> DNA
   <213> artificial sequence
<220>
   <223> synthetic oligonucleotide (BunyaUmbreL_Rv1)
<400> 35
   tctttgaata tcggatgccc a 21
<210> 36
   <211> 20
   <212> DNA
   <213> artificial sequence
<220>
   <223> synthetic oligonucleotide (BunyaUmbreL_Fw1bis)
<400> 36
   gttgcgagag caaatgagcc 20
<210> 37
   <211> 22
   <212> DNA
   <213> artificial sequence
<220>
   <223> artificial sequence (BunyaUmbreL_Rvlbis)
<400> 37
   ttctttgaat atcggatgcc ca 22
<210> 38
   <211> 23
   <212> DNA
   <213> artificial sequence
<220>
   <223> synthetic oligonucleotide (BunyaUmbreL_Fw2)
<400> 38
   aagaacaaag ggaagtgtcc tca 23
<210> 39
   <211> 23
   <212> DNA
   <213> artificial sequence
<220>
   <223> synthetic oligonucleotide (BunyaUmbreL_Rv2)
<400> 39
   tctgtttcag tgtcatctaa gcc 23
<210> 40
   <211> 21
   <212> DNA
   <213> artificial sequence
<220>
   <223> synthetic oligonucleotide (BunyaUmbreL_Fw3)
<400> 40
   ctggcaggtc gtcactcaat a 21
<210> 41
   <211> 22
   <212> DNA
   <213> artificial sequence
<220>
   <223> synthetic oligonucleotide (BunyaUmbreL_Rv3)
<400> 41
   aatgcattta gacagcctgc tt 22
<210> 42
   <211> 25
   <212> DNA
   <213> artificial sequence
<220>
   <223> synthtetic oligonucleotide (BunyaUmbreL_Fw4)
<400> 42
   tggtttgatg gtaatgtcac tttga 25
<210> 43
   <211> 21
   <212> DNA
   <213> artificial sequence
<220>
   <223> synthetic oligonucleotide (BunyaUmbreL_Rv4)
<400> 43
   tcgtctggat tgactttcga t 21
<210> 44
   <211> 20
   <212> DNA
   <213> artificial sequence
<220>
   <223> synthetic oligonucleotide (BunyaUmbreL_Fw5)
<400> 44
   tgtaggggaa tacgaggcca 20
<210> 45
   <211> 22
   <212> DNA
   <213> artificial sequence
<220>
   <223> synthetic oligonucleotide (BunyaUmbreL_Rv5)
<400> 45
   agggtatgct ttcctctgat ct 22
<210> 46
   <211> 20
   <212> DNA
   <213> artificial sequence
<220>
   <223> synthetic oligonucleotide (BunyaUmbreL_Fw6)
<400> 46
   ctgcctaaca ttcggctgtc 20
<210> 47
   <211> 20
   <212> DNA
   <213> artificial sequence
<220>
   <223> synthetic oligonucleotide (BunyaUmbreL_Rv6)
<400> 47
   tcttgcaccg cttggaaatc 20
<210> 48
   <211> 22
   <212> DNA
   <213> artificial sequence
<220>
   <223> synthetic oligonucleotide (BunyaUmbreL_Fw6bis)
<400> 48
   actgggacat ttcaaggctt ag 22
<210> 49
   <211> 22
   <212> DNA
   <213> artificial sequence
<220>
   <223> synthetic oligonucleotide (BunyaUmbreL_Rv6bis)
<400> 49
   gagaactttt ctgcgatacc tg 22
<210> 50
   <211> 21
   <212> DNA
   <213> artificial sequence
<220>
   <223> synthetic oligonucleotide (BunyaUmbreL_Fw7)
<400> 50
   actgatcaag gtgtggatgt t 21
<210> 51
   <211> 20
   <212> DNA
   <213> artificial sequence
<220>
   <223> synthetic oligonucleotide (BunyaUmbreL_Rv7)
<400> 51
   cgggcagcaa caaagtgttt 20
<210> 52
   <211> 25
   <212> DNA
   <213> artificial sequence
<220>
   <223> synthetic oligonucleotide (BunyaUmbreL_Fw8)
<400> 52
   gagcataaaa tgcaagatag gatca 25
<210> 53
   <211> 20
   <212> DNA
   <213> artificial sequence
<220>
   <223> synthetic oligonucleotide (BunyaUmbreL_Rv8)
<400> 53
   tcaaaccgaa acccatgctt 20
<210> 54
   <211> 20
   <212> DNA
   <213> artificial sequence
<220>
   <223> synthetic oligonucleotide (BunyaUmbreL_Fw9)
<400> 54
   aagcatgggt ttcggtttga 20
<210> 55
   <211> 21
   <212> DNA
   <213> artificial sequence
<220>
   <223> synthetic oligonucleotide (BunyaUmbreL_Rv9)
<400> 55
   agccaggtcg acatttacca a 21
<210> 56
   <211> 22
   <212> DNA
   <213> artificial sequence
<220>
   <223> synthetic oligonucleotide (BunyaUmbreL_Fw10)
<400> 56
   ttggactgtg acggtgatac ag 22
<210> 57
   <211> 22
   <212> DNA
   <213> artificial sequence
<220>
   <223> synthetic oligonucleotide (BunyaUmbreL_Rv10)
<400> 57
   atccaatttc ccagatgcag ga 22
<210> 58
   <211> 285
   <212> PRT
   <213> Moissiacense virus
<400> 58
   Gln Ala Thr Ile Arg Cys Phe Ser Gly Gly Ser Lys Ile Met Glu Lys
   1               5                   10                  15
   Thr Ser Asn Val Ser Leu Ser Glu Leu Cys Leu Lys Asp Asp Val Ser
               20                  25                  30
   Gln Leu Lys Ile Ile Val Asp His Thr Lys Asn Asp Ser Gly Leu Phe
           35                  40                  45
   Ser Thr Ala Ser Val Trp Arg Lys Trp Ile Val Ser Asp Trp Thr Thr
       50                  55                  60
   Cys Asn Pro Lys Lys Met Ala Leu Gly Thr Ile Ser Val Ile Asp Ile
   65                  70                  75                  80
   Asp Lys Asn Phe Ile Leu Lys Ser Ser Ser Tyr Ile Cys Asn Lys Glu
                   85                  90                  95
   Cys Thr Ile Thr Val Asp Lys Glu Arg Ala Gln Val Leu Leu Gln Ser
               100                 105                 110
   Asp Gly Leu Asn His Phe Glu Val Ser Gly Thr Thr Leu Ser Ser Gly
           115                 120                 125
   Trp Phe Lys Ser Thr Ala Thr Val Thr Leu Asp Gln Thr Cys Glu His
       130                 135                 140
   Val Lys Val Ser Cys Gly Gln Lys Ser Met Gln Phe His Ala Cys Phe
   145                 150                 155                 160
   Lys Asn His Met Ser Cys Ile Arg Phe Leu His Arg Ser Ile Met Pro
                   165                 170                 175
   Ala Ser Phe Ala Val Ser Ile Cys Gln Asn Ile Glu Leu Ile Leu Met
                   180                 185                 190
   Thr Thr Phe Thr Leu Ile Ile Phe Leu Leu Leu Ser Ile Leu Thr Lys
           195                 200                 205
   Thr Tyr Ile Cys Tyr Ile Leu Leu Pro Ile Phe Ile Pro Phe Ala Tyr
       210                 215                 220
   Ala Tyr Gly Phe Val Tyr Asp Lys Thr Cys Lys Lys Cys Ser Leu Cys
   225                 230                 235                 240
   Gly Leu Ala Tyr His Pro Phe Thr Lys Cys Gly Ile His Cys Val Cys
                   245                 250                 255
   Gly Cys Arg Phe Glu Asn Ser Asp Ala Met Lys Thr His Arg Glu Lys
               260                 265                 270
   Gly Lys Cys Pro Gly Phe Lys Ser Met Arg Ile Ala Arg
           275                 280                 285
<210> 59
   <211> 174
   <212> PRT
   <213> Moissiacense virus
<400> 59
   Val Leu Cys Lys Ala Lys Gly Ser Ala Phe Ala Leu Ser Val Leu Leu
   1               5                   10                  15
   Ala Thr Leu Ala Leu Gly Phe Leu Thr Pro Val Gln Gly Leu Val Leu
               20                  25                  30
   Asp Lys Thr Gln Ile Asn Tyr Thr Glu Asp Gln Leu Pro Glu Ser Phe
           35                  40                  45
   Gln Thr Met Gln Ser Thr Ile Ser Ile Phe Lys Phe Ala Thr Val Met
       50                  55                  60
   Asn Ala Gly Trp Thr Tyr Cys Met Phe Leu Val Thr Leu Ala Leu Tyr
   65                  70                  75                  80
   Ile Ile His Lys Lys Tyr Ala His Arg Leu Leu Ser Tyr Tyr Val Ile
                   85                  90                  95
   His Cys Arg Glu Cys Asp Met Leu His Asp Lys Ser Gly Leu Lys Tyr
               100                 105                 110
   Asn Gly Asp Phe Thr Asn Lys Cys Gly Phe Cys Thr Cys Gly Gln Val
           115                 120                 125
   Glu Asp Ala Met Gly Leu Thr Val His Gln Ile Arg Asn His Cys Ser
       130                 135                 140
   Ala Asn Tyr Arg Thr Lys Met Val Leu Asn Trp Met Ile Val Leu Leu
   145                 150                 155                 160
   Leu Ala Thr Leu Ile Lys Asp Ser Ile Met Leu Thr Ala Ala
                   165                 170
<210> 60
   <211> 989
   <212> PRT
   <213> Moissiacense virus
<400> 60
   Ala Gly Thr Glu Phe Glu Thr Cys Ile Lys Lys Val Glu Met Thr Pro
   1               5                   10                  15
   Glu Cys Thr Gly Pro Phe Met Glu Val Gly Ile Cys Asp His Gln Gln
               20                  25                  30
   Lys Ser Leu Arg Tyr His Glu Ile Ala Asn Met Leu Leu Gly Lys Gly
           35                  40                  45
   Thr Ile Ser Lys Leu Asp Val Asp Ile Ile Asp Ala Leu Pro Asp Arg
       50                  55                  60
   Ile Ala Gly Asp Leu Gln Ile Ile Gln Ser Ser Ser Asp Phe His His
   65                  70                  75                  80
   Gln Gln Met Leu Glu Tyr Val Phe Leu Leu Arg His Cys Asp Tyr Tyr
                   85                  90                  95
   Ser Ser Phe Val Ser Asp Asn Ser Tyr Ser Gln Ile Leu Trp Gln Thr
               100                 105                 110
   Ala Ala Lys Leu Asn Glu Thr Asn Leu Cys Lys His Glu Asn Ser Lys
           115                 120                 125
   Lys Ser Cys Lys Cys Leu Lys Ser Asn Ala Asn Cys Gly Glu Ser Glu
       130                 135                 140
   Asp Ile Asn Ile Ala Ala Gln Ala Tyr Tyr Lys Asn Lys Glu Ser Glu
   145                 150                 155                 160
   Leu Gly Gln Asp Leu Thr Thr Ile Met Tyr Ile Leu Lys Lys Met Ile
                   165                 170                 175
   Pro Gly Ser Gly Tyr Ser Tyr Leu Leu Asn Ala Ala Lys His Asn Lys
               180                 185                 190
   Leu Glu Leu Phe Lys Ala Tyr Met Thr Asn Ile Ser Ser Lys Tyr Ser
           195                 200                 205
   Asn Asn Lys Lys Leu Lys Asp Phe Met Ala Ile Ile Asn Ala Ile Leu
       210                 215                 220
   Ala Asn Leu Ser Ala Leu Ser His Ser Ser Glu Glu Ser Ile Ser Gln
   225                 230                 235                 240
   Tyr Lys Gln Ile Asp Phe Ser Ser Leu Val Gly Glu Thr Thr Asp Met
                   245                 250                 255
   Leu Glu Ala Asn Phe Asn Gln Thr Ile Ser Thr Asp Lys Ile Ile Cys
               260                 265                 270
   Glu Asn Pro Lys Val Met Ile Cys Ile Ser Pro Arg Thr Lys Val Ser
           275                 280                 285
   Ser Gly Glu Leu Tyr Val Cys Gln Arg Ser Gly Lys Tyr Tyr Ile Ile
       290                 295                 300
   Asp Thr Gly Lys Phe Lys Leu Tyr Lys Leu Ile Asp Ala Asp Asn Leu
   305                 310                 315                 320
   Tyr Cys Ala Ala Asp Ala Gln Cys Arg Tyr Glu Phe Arg Ile Leu Thr
                   325                 330                 335
   Thr Gly Glu Met Gln Ala Thr Phe Lys Asp Asn Cys Lys Ala Ser Val
               340                 345                 350
   His Val Asn Thr Pro Gly Tyr Gln Asn Glu Ala Ile Thr His Cys Lys
           355                 360                 365
   Val Leu Ala Asn Gly Lys Cys Lys Tyr Asn Asn Gln Thr Ser Ile Asp
       370                 375                 380
   Ile Thr Leu Cys Ala Asn Asn Arg Tyr Tyr Pro Glu Ala Lys Gly Arg
   385                 390                 395                 400
   Ile Phe Gly Val Asp Pro Glu Asp Leu Cys Phe Asp Lys Thr Cys Thr
                   405                 410                 415
   Lys Gln Gln Thr Pro Tyr Asn Glu Glu Leu Ile Ser Met Cys Val Trp
               420                 425                 430
   Glu Asn Pro Thr Leu Arg Pro Ile Lys Pro Arg Thr Glu Val Ser Met
           435                 440                 445
   Thr Phe Gln Glu Tyr Lys Asp Gln Leu Leu Lys Lys Ile Asn Thr Asp
       450                 455                 460
   Leu Val Leu His Lys Phe Val Arg Thr Gln Asn Leu Pro Tyr Phe Ile
   465                 470                 475                 480
   Pro Gln Phe Lys Tyr Ile Thr Ile Gln Gly Thr Ser Thr Thr Asp Gly
                   485                 490                 495
   Ile Thr Ser Ser Phe Ile Phe Phe Glu Ile Pro Ala Leu Thr Gly Ala
               500                 505                 510
   Ser Ala Gly Tyr Lys Val Ile Asn Ser Glu Gly Gln Glu Leu Met Asp
           515                 520                 525
   Met Val Val Tyr Ile Lys Thr Ser Lys Thr Ser Ala Lys Tyr Lys Arg
       530                 535                 540
   Val Tyr Tyr Thr Gly Pro Thr Ile Ala Ile Asn Thr Lys His Gln Glu
   545                 550                 555                 560
   His Cys Thr Gly Thr Cys Pro Glu Arg Ile Glu His Asp Glu Gly Trp
                   565                 570                 575
   Ala Thr Phe Ser Lys Glu Arg Thr Ser Asn Trp Gly Cys Glu Glu Phe
               580                 585                 590
   Gly Cys Leu Ala Val Glu Thr Gly Cys Leu Phe Gly Ser Cys Gln Asp
           595                 600                 605
   Val Ile Arg Arg Glu Leu Asp Val Phe Ser Lys Leu Glu Glu Asp Lys
       610                 615                 620
   Ile Leu Thr Glu Leu Cys Ile Thr Leu Ser His Asp Thr Tyr Cys Val
   625                 630                 635                 640
   Thr Ile Asp Ser Thr Thr Pro Thr Ile Asn Asp Tyr Phe Glu Ile Gln
                   645                 650                 655
   Tyr Lys Thr Val Asp Val Gln Thr Leu Pro Lys Met Ile Gly Val Thr
   660 665 670
   His His Lys Leu Leu Arg Gly Gln Ile Asn Glu Leu Gly Ser Phe Gly
   675 680 685
   Lys Phe Cys Gly Asn Ile Gln Val Val Gly Asn Gln Thr Tyr Gly Gln
   690 695 700
   Ala Ala Val Lys Phe Asp Tyr Ile Cys His Ala Ala Gln Arg Lys Asp
   705 710 715 720
   Val Val Ile Arg Lys Cys Leu Glu Asn Asn Tyr Gln Ser Cys Lys Leu
   725 730 735
   Leu Thr His Asp Thr Asp Leu Ile Leu Glu Glu Ser Lys Thr Ser Val
   740 745 750
   Glu Val Ile Asn Asn Lys Arg Ile Thr Gly Gln Ile Ala Leu Lys Val
   755 760 765
   Leu Leu Gly Asp Phe Asn Tyr Lys Gln Tyr Ala Ala Asn Val Glu Ala
   770 775 780
   Asp Ile Glu Ala Asn Cys Val Gly Cys Thr Asn Cys Ile Lys Gly Ile
   785 790 795 800
   Thr Cys Glu Ile Lys Val Lys Thr Glu Ile Glu Ala Ser Cys Lys Val
   805 810 815
   Glu Pro Pro Cys Pro Ser Tyr Thr Ser Arg Ile Ile Ile Lys Pro Gly
   820 825 830
   Arg Asp Thr His Thr Leu Leu Met Lys Cys Asp Lys Lys Ile Thr Thr
   835 840 845
   Gly Glu Leu Ala Ile Lys Ile Cys Asn Met Gln Ile Gln Ala His Leu
   850 855 860
   Ser Thr Ile Glu Ser Asn Asp Gln Leu Glu Leu Ser Thr Gly Asp Gln
   865 870 875 880
   Ser Thr Tyr Ile His Glu Glu Asp Leu Arg Cys Ser Thr Trp Ile Cys
   885 890 895
   Lys Val Arg Asp Glu Gly Ile Ser Phe Ile Phe Lys Pro Leu Thr Asp
   900 905 910
   Trp Leu Gly Ser Phe Thr Lys Pro Ile Leu Ile Ala Gly Cys Met Ile
   915 920 925
   Ile Val Ala Ile Ile Leu Ile Tyr Ile Leu Met Pro Met Cys Ala Arg
   930 935 940
   Leu Arg Asp Leu Leu Lys Lys Asn Glu Tyr Glu Tyr Leu Gln Asp Thr
   945 950 955 960
   Lys Val Ile Val Lys Pro Lys Gly Thr Ala Ser Ser Ile Gly Leu Arg
   965 970 975
   Ser Lys Lys Ile Asn Ile Gln Ser Ser Lys Ala Val Asp
   980 985
<210> 61
   <211> 920
   <212> PRT
   <213> artificial sequence
<220>
   <223> synthetic polypeptide (Gc protein extracellular domain)
<220>
   <221> VARIANT
   <222> (897)..(897)
   <223> Xaa is Isoleucine or Lysine
<400> 61
   Ala Gly Thr Glu Phe Glu Thr Cys Ile Lys Lys Val Glu Met Thr Pro
   1 5 10 15
   Glu Cys Thr Gly Pro Phe Met Glu Val Gly Ile Cys Asp His Gln Gln
   20 25 30
   Lys Ser Leu Arg Tyr His Glu Ile Ala Asn Met Leu Leu Gly Lys Gly
   35 40 45
   Thr Ile Ser Lys Leu Asp Val Asp Ile Ile Asp Ala Leu Pro Asp Arg
   50 55 60
   Ile Ala Gly Asp Leu Gln Ile Ile Gln Ser Ser Ser Asp Phe His His
   65 70 75 80
   Gln Gln Met Leu Glu Tyr Val Phe Leu Leu Arg His Cys Asp Tyr Tyr
   85 90 95
   Ser Ser Phe Val Ser Asp Asn Ser Tyr Ser Gln Ile Leu Trp Gln Thr
   100 105 110
   Ala Ala Lys Leu Asn Glu Thr Asn Leu Cys Lys His Glu Asn Ser Lys
           115                 120                 125
   Lys Ser Cys Lys Cys Leu Lys Ser Asn Ala Asn Cys Gly Glu Ser Glu
       130                 135                 140
   Asp Ile Asn Ile Ala Ala Gln Ala Tyr Tyr Lys Asn Lys Glu Ser Glu
   145                 150                 155                 160
   Leu Gly Gln Asp Leu Thr Thr Ile Met Tyr Ile Leu Lys Lys Met Ile
                   165                 170                 175
   Pro Gly Ser Gly Tyr Ser Tyr Leu Leu Asn Ala Ala Lys His Asn Lys
               180                 185                 190
   Leu Glu Leu Phe Lys Ala Tyr Met Thr Asn Ile Ser Ser Lys Tyr Ser
           195                 200                 205
   Asn Asn Lys Lys Leu Lys Asp Phe Met Ala Ile Ile Asn Ala Ile Leu
       210                 215                 220
   Ala Asn Leu Ser Ala Leu Ser His Ser Ser Glu Glu Ser Ile Ser Gln
   225                 230                 235                 240
   Tyr Lys Gln Ile Asp Phe Ser Ser Leu Val Gly Glu Thr Thr Asp Met
                   245                 250                 255
   Leu Glu Ala Asn Phe Asn Gln Thr Ile Ser Thr Asp Lys Ile Ile Cys
               260                 265                 270
   Glu Asn Pro Lys Val Met Ile Cys Ile Ser Pro Arg Thr Lys Val Ser
           275                 280                 285
   Ser Gly Glu Leu Tyr Val Cys Gln Arg Ser Gly Lys Tyr Tyr Ile Ile
       290                 295                 300
   Asp Thr Gly Lys Phe Lys Leu Tyr Lys Leu Ile Asp Ala Asp Asn Leu
   305                 310                 315                 320
   Tyr Cys Ala Ala Asp Ala Gln Cys Arg Tyr Glu Phe Arg Ile Leu Thr
                   325                 330                 335
   Thr Gly Glu Met Gln Ala Thr Phe Lys Asp Asn Cys Lys Ala Ser Val
               340                 345                 350
   His Val Asn Thr Pro Gly Tyr Gln Asn Glu Ala Ile Thr His Cys Lys
           355                 360                 365
   Val Leu Ala Asn Gly Lys Cys Lys Tyr Asn Asn Gln Thr Ser Ile Asp
   370 375 380
   Ile Thr Leu Cys Ala Asn Asn Arg Tyr Tyr Pro Glu Ala Lys Gly Arg
   385 390 395 400
   Ile Phe Gly Val Asp Pro Glu Asp Leu Cys Phe Asp Lys Thr Cys Thr
   405 410 415
   Lys Gln Gln Thr Pro Tyr Asn Glu Glu Leu Ile Ser Met Cys Val Trp
   420 425 430
   Glu Asn Pro Thr Leu Arg Pro Ile Lys Pro Arg Thr Glu Val Ser Met
   435 440 445
   Thr Phe Gln Glu Tyr Lys Asp Gln Leu Leu Lys Lys Ile Asn Thr Asp
   450 455 460
   Leu Val Leu His Lys Phe Val Arg Thr Gln Asn Leu Pro Tyr Phe Ile
   465 470 475 480
   Pro Gln Phe Lys Tyr Ile Thr Ile Gln Gly Thr Ser Thr Thr Asp Gly
   485 490 495
   Ile Thr Ser Ser Phe Ile Phe Phe Glu Ile Pro Ala Leu Thr Gly Ala
   500 505 510
   Ser Ala Gly Tyr Lys Val Ile Asn Ser Glu Gly Gln Glu Leu Met Asp
   515 520 525
   Met Val Val Tyr Ile Lys Thr Ser Lys Thr Ser Ala Lys Tyr Lys Arg
   530 535 540
   Val Tyr Tyr Thr Gly Pro Thr Ile Ala Ile Asn Thr Lys His Gln Glu
   545 550 555 560
   His Cys Thr Gly Thr Cys Pro Glu Arg Ile Glu His Asp Glu Gly Trp
   565 570 575
   Ala Thr Phe Ser Lys Glu Arg Thr Ser Asn Trp Gly Cys Glu Glu Phe
   580 585 590
   Gly Cys Leu Ala Val Glu Thr Gly Cys Leu Phe Gly Ser Cys Gln Asp
   595 600 605
   Val Ile Arg Arg Glu Leu Asp Val Phe Ser Lys Leu Glu Glu Asp Lys
   610 615 620
   Ile Leu Thr Glu Leu Cys Ile Thr Leu Ser His Asp Thr Tyr Cys Val
   625 630 635 640
   Thr Ile Asp Ser Thr Thr Pro Thr Ile Asn Asp Tyr Phe Glu Ile Gln
   645 650 655
   Tyr Lys Thr Val Asp Val Gln Thr Leu Pro Lys Met Ile Gly Val Thr
   660 665 670
   His His Lys Leu Leu Arg Gly Gln Ile Asn Glu Leu Gly Ser Phe Gly
   675 680 685
   Lys Phe Cys Gly Asn Ile Gln Val Val Gly Asn Gln Thr Tyr Gly Gln
   690 695 700
   Ala Ala Val Lys Phe Asp Tyr Ile Cys His Ala Ala Gln Arg Lys Asp
   705 710 715 720
   Val Val Ile Arg Lys Cys Leu Glu Asn Asn Tyr Gln Ser Cys Lys Leu
   725 730 735
   Leu Thr His Asp Thr Asp Leu Ile Leu Glu Glu Ser Lys Thr Ser Val
   740 745 750
   Glu Val Ile Asn Asn Lys Arg Ile Thr Gly Gln Ile Ala Leu Lys Val
   755 760 765
   Leu Leu Gly Asp Phe Asn Tyr Lys Gln Tyr Ala Ala Asn Val Glu Ala
   770 775 780
   Asp Ile Glu Ala Asn Cys Val Gly Cys Thr Asn Cys Ile Lys Gly Ile
   785 790 795 800
   Thr Cys Glu Ile Lys Val Lys Thr Glu Ile Glu Ala Ser Cys Lys Val
   805 810 815
   Glu Pro Pro Cys Pro Ser Tyr Thr Ser Arg Ile Ile Ile Lys Pro Gly
   820 825 830
   Arg Asp Thr His Thr Leu Leu Met Lys Cys Asp Lys Lys Ile Thr Thr
   835 840 845
   Gly Glu Leu Ala Ile Lys Ile Cys Asn Met Gln Ile Gln Ala His Leu
   850 855 860
   Ser Thr Ile Glu Ser Asn Asp Gln Leu Glu Leu Ser Thr Gly Asp Gln
   865                 870                 875                 880
   Ser Thr Tyr Ile His Glu Glu Asp Leu Arg Cys Ser Thr Trp Ile Cys
                   885                 890                 895
   Xaa Val Arg Asp Glu Gly Ile Ser Phe Ile Phe Lys Pro Leu Thr Asp
               900                 905                 910
   Trp Leu Gly Ser Phe Thr Lys Pro
           915                 920
<210> 62
   <211> 261
   <212> PRT
   <213> artificial sequence
<220>
   <223> synthetic polypeptide (Gc protein N-terminal alpha helix region)
<400> 62
   Leu Arg Tyr His Glu Ile Ala Asn Met Leu Leu Gly Lys Gly Thr Ile
   1               5                   10                  15
   Ser Lys Leu Asp Val Asp Ile Ile Asp Ala Leu Pro Asp Arg Ile Ala
               20                  25                  30
   Gly Asp Leu Gln Ile Ile Gln Ser Ser Ser Asp Phe His His Gln Gln
           35                  40                  45
   Met Leu Glu Tyr Val Phe Leu Leu Arg His Cys Asp Tyr Tyr Ser Ser
       50                  55                  60
   Phe Val Ser Asp Asn Ser Tyr Ser Gln Ile Leu Trp Gln Thr Ala Ala
   65                  70                  75                  80
   Lys Leu Asn Glu Thr Asn Leu Cys Lys His Glu Asn Ser Lys Lys Ser
                   85                  90                  95
   Cys Lys Cys Leu Lys Ser Asn Ala Asn Cys Gly Glu Ser Glu Asp Ile
               100                 105                 110
   Asn Ile Ala Ala Gln Ala Tyr Tyr Lys Asn Lys Glu Ser Glu Leu Gly
           115                 120                 125
   Gln Asp Leu Thr Thr Ile Met Tyr Ile Leu Lys Lys Met Ile Pro Gly
       130                 135                 140
   Ser Gly Tyr Ser Tyr Leu Leu Asn Ala Ala Lys His Asn Lys Leu Glu
   145                 150                 155                 160
   Leu Phe Lys Ala Tyr Met Thr Asn Ile Ser Ser Lys Tyr Ser Asn Asn
                   165                 170                 175
   Lys Lys Leu Lys Asp Phe Met Ala Ile Ile Asn Ala Ile Leu Ala Asn
               180                 185                 190
   Leu Ser Ala Leu Ser His Ser Ser Glu Glu Ser Ile Ser Gln Tyr Lys
           195                 200                 205
   Gln Ile Asp Phe Ser Ser Leu Val Gly Glu Thr Thr Asp Met Leu Glu
       210                 215                 220
   Ala Asn Phe Asn Gln Thr Ile Ser Thr Asp Lys Ile Ile Cys Glu Asn
   225                 230                 235                 240
   Pro Lys Val Met Ile Cys Ile Ser Pro Arg Thr Lys Val Ser Ser Gly
                   245                 250                 255
   Glu Leu Tyr Val Cys
               260
<210> 63
   <211> 431
   <212> PRT
   <213> artificial sequence
<220>
   <223> synthetic polypeptide (Gc protein N-terminal domain)
<400> 63
   Gly Thr Glu Phe Glu Thr Cys Ile Lys Lys Val Glu Met Thr Pro Glu
   1               5                   10                  15
   Cys Thr Gly Pro Phe Met Glu Val Gly Ile Cys Asp His Gln Gln Lys
               20                  25                  30
   Ser Leu Arg Tyr His Glu Ile Ala Asn Met Leu Leu Gly Lys Gly Thr
           35                  40                  45
   Ile Ser Lys Leu Asp Val Asp Ile Ile Asp Ala Leu Pro Asp Arg Ile
       50                  55                  60
   Ala Gly Asp Leu Gln Ile Ile Gln Ser Ser Ser Asp Phe His His Gln
   65                  70                  75                  80
   Gln Met Leu Glu Tyr Val Phe Leu Leu Arg His Cys Asp Tyr Tyr Ser
                   85                  90                  95
   Ser Phe Val Ser Asp Asn Ser Tyr Ser Gln Ile Leu Trp Gln Thr Ala
               100                 105                 110
   Ala Lys Leu Asn Glu Thr Asn Leu Cys Lys His Glu Asn Ser Lys Lys
           115                 120                 125
   Ser Cys Lys Cys Leu Lys Ser Asn Ala Asn Cys Gly Glu Ser Glu Asp
       130                 135                 140
   Ile Asn Ile Ala Ala Gln Ala Tyr Tyr Lys Asn Lys Glu Ser Glu Leu
   145                 150                 155                 160
   Gly Gln Asp Leu Thr Thr Ile Met Tyr Ile Leu Lys Lys Met Ile Pro
                   165                 170                 175
   Gly Ser Gly Tyr Ser Tyr Leu Leu Asn Ala Ala Lys His Asn Lys Leu
               180                 185                 190
   Glu Leu Phe Lys Ala Tyr Met Thr Asn Ile Ser Ser Lys Tyr Ser Asn
           195                 200                 205
   Asn Lys Lys Leu Lys Asp Phe Met Ala Ile Ile Asn Ala Ile Leu Ala
       210                 215                 220
   Asn Leu Ser Ala Leu Ser His Ser Ser Glu Glu Ser Ile Ser Gln Tyr
   225                 230                 235                 240
   Lys Gln Ile Asp Phe Ser Ser Leu Val Gly Glu Thr Thr Asp Met Leu
                   245                 250                 255
   Glu Ala Asn Phe Asn Gln Thr Ile Ser Thr Asp Lys Ile Ile Cys Glu
               260                 265                 270
   Asn Pro Lys Val Met Ile Cys Ile Ser Pro Arg Thr Lys Val Ser Ser
           275                 280                 285
   Gly Glu Leu Tyr Val Cys Gln Arg Ser Gly Lys Tyr Tyr Ile Ile Asp
       290                 295                 300
   Thr Gly Lys Phe Lys Leu Tyr Lys Leu Ile Asp Ala Asp Asn Leu Tyr
   305                 310                 315                 320
   Cys Ala Ala Asp Ala Gln Cys Arg Tyr Glu Phe Arg Ile Leu Thr Thr
                   325                 330                 335
   Gly Glu Met Gln Ala Thr Phe Lys Asp Asn Cys Lys Ala Ser Val His
                   340                 345                 350
   Val Asn Thr Pro Gly Tyr Gln Asn Glu Ala Ile Thr His Cys Lys Val
           355                 360                 365
   Leu Ala Asn Gly Lys Cys Lys Tyr Asn Asn Gln Thr Ser Ile Asp Ile
       370                 375                 380
   Thr Leu Cys Ala Asn Asn Arg Tyr Tyr Pro Glu Ala Lys Gly Arg Ile
   385                 390                 395                 400
   Phe Gly Val Asp Pro Glu Asp Leu Cys Phe Asp Lys Thr Cys Thr Lys
                   405                 410                 415
   Gln Gln Thr Pro Tyr Asn Glu Glu Leu Ile Ser Met Cys Val Trp
               420                 425                 430
<210> 64
   <211> 254
   <212> PRT
   <213> artificial sequence
<220>
   <223> synthetic polypeptide (fragment of Gc protein N-terminal domain)
<400> 64
   Gly Thr Glu Phe Glu Thr Cys Ile Lys Lys Val Glu Met Thr Pro Glu
   1               5                   10                  15
   Cys Thr Gly Pro Phe Met Glu Val Gly Ile Cys Asp His Gln Gln Lys
               20                  25                  30
   Ser Leu Arg Tyr His Glu Ile Ala Asn Met Leu Leu Gly Lys Gly Thr
           35                  40                  45
   Ile Ser Lys Leu Asp Val Asp Ile Ile Asp Ala Leu Pro Asp Arg Ile
       50                  55                  60
   Ala Gly Asp Leu Gln Ile Ile Gln Ser Ser Ser Asp Phe His His Gln
   65                  70                  75                  80
   Gln Met Leu Glu Tyr Val Phe Leu Leu Arg His Cys Asp Tyr Tyr Ser
                   85                  90                  95
   Ser Phe Val Ser Asp Asn Ser Tyr Ser Gln Ile Leu Trp Gln Thr Ala
               100                 105                 110
   Ala Lys Leu Asn Glu Thr Asn Leu Cys Lys His Glu Asn Ser Lys Lys
           115                 120                 125
   Ser Cys Lys Cys Leu Lys Ser Asn Ala Asn Cys Gly Glu Ser Glu Asp
       130                 135                 140
   Ile Asn Ile Ala Ala Gln Ala Tyr Tyr Lys Asn Lys Glu Ser Glu Leu
   145                 150                 155                 160
   Gly Gln Asp Leu Thr Thr Ile Met Tyr Ile Leu Lys Lys Met Ile Pro
                   165                 170                 175
   Gly Ser Gly Tyr Ser Tyr Leu Leu Asn Ala Ala Lys His Asn Lys Leu
               180                 185                 190
   Glu Leu Phe Lys Ala Tyr Met Thr Asn Ile Ser Ser Lys Tyr Ser Asn
           195                 200                 205
   Asn Lys Lys Leu Lys Asp Phe Met Ala Ile Ile Asn Ala Ile Leu Ala
       210                 215                 220
   Asn Leu Ser Ala Leu Ser His Ser Ser Glu Glu Ser Ile Ser Gln Tyr
   225                 230                 235                 240
   Lys Gln Ile Asp Phe Ser Ser Leu Val Gly Glu Thr Thr Asp
                   245                 250
<210> 65
   <211> 2760
   <212> DNA
   <213> artificial sequence
<220>
   <223> synthetic polynucleotide coding for Gc protein extracellular domain
<400> 65
   gcagggacag aatttgaaac atgcatcaag aaagtagaaa tgacaccgga atgcacagga 60
   cctttcatgg aagttggaat ctgtgatcac cagcagaaga gcctaagata ccatgagata 120
   gctaatatgt tattgggtaa agggacaatc tcaaaattag atgtggatat tatagatgca 180
   ttgcctgaca ggatagctgg agacctccag ataatacagt caagcagtga tttccaccat 240
   caacagatgc tggaatatgt gtttctattg cgtcattgtg attattactc aagttttgtt 300
   tcggataaca gctactcaca aatcttgtgg caaacagcag caaagttaaa tgaaaccaac 360
   ctttgcaaac atgagaattc aaagaaatcc tgcaagtgct taaaatcaaa tgcaaattgt 420
   ggagagtcag aagacatcaa catagcagca caggcatact acaaaaataa ggaatctgag 480
   ctaggacaag atctaacaac cataatgtac atactcaaaa aaatgattcc aggatcaggt 540
   tatagttatc tgctaaatgc agccaagcac aacaaattag aattgttcaa agcttacatg 600
   actaatattt cctcaaaata ttcaaataac aaaaaattaa aagatttcat ggcgataata 660
   aatgctatac tagcaaatct gtcagctctc tcacatagta gtgaagaatc aatctcacaa 720
   tacaaacaga ttgatttttc ttccctagta ggcgaaacaa cagacatgct cgaagcaaat 780
   tttaatcaaa cgatcagtac agacaagata atttgtgaaa atcctaaagt aatgatttgc 840
   atttcaccca ggacaaaagt ttcatccggt gaattgtatg tctgccaaag atcaggaaag 900
   tactacataa tagataccgg caaattcaag ctatataaat taattgacgc agacaatttg 960
   tactgtgcag cagatgctca gtgtaggtat gaatttagaa ttttaaccac aggggaaatg 1020
   caggcaactt ttaaagataa ttgcaaagca tcagtacatg ttaacacccc aggataccaa 1080
   aatgaagcta taactcattg caaagtgctc gcgaatggta aatgtaagta taataatcaa 1140
   acttcaatag atataacatt gtgtgctaac aacagatatt atcccgaagc aaaaggcagg 1200
   atctttggag ttgatccaga agatctatgc tttgataaga catgtactaa acaacagact 1260
   ccatacaacg aagaactaat cagtatgtgt gtttgggaaa atccgacact taggccaatc 1320
   aaacccagaa ctgaggtttc tatgactttc caagagtaca aagatcagtt attaaagaaa 1380
   atcaacacag acctggtgct gcacaaattt gttagaacac aaaacttacc ttatttcata 1440
   ccgcaattca agtatataac tatacaagga acgtcaacaa cagacgggat tacttccagt 1500
   tttatattct ttgaaattcc tgcattgaca ggagcatctg ctggatacaa agttataaat 1560
   agcgaaggcc aagaattgat ggacatggta gtttatatca aaacatcaaa gactagcgca 1620
   aagtacaagc gcgtgtatta tacaggcccc actatagcta taaatacaaa acatcaagag 1680
   cactgcacag ggacatgtcc tgaaagaata gaacatgatg aaggttgggc aacattttcg 1740
   aaagaaagaa ccagcaattg gggctgtgaa gaatttggtt gtctagctgt tgagactggt 1800
   tgtttatttg ggtcatgcca ggatgtaatt agaagagaac ttgatgtttt ttcaaagttg 1860
   gaggaagata aaatattgac tgaactctgt ataacattaa gccatgacac ttactgcgtg 1920
   acaatcgatt ctacaacacc aacaatcaat gattactttg aaatccaata caaaacagta 1980
   gatgtacaaa cattgccaaa gatgatagga gtcactcatc ataagctatt gcgagggcag 2040
   ataaacgaat taggatcatt tggcaaattt tgtggcaaca tacaagtagt tggtaatcaa 2100
   acatatgggc aggctgcagt aaagtttgat tacatttgcc atgctgcaca aagaaaagat 2160
   gtagttataa gaaagtgctt agaaaataat taccaaagtt gtaagctact gacacatgac 2220
   acagacttga tacttgaaga aagtaagacc agtgttgaag tcataaacaa taaaaggatt 2280
   acaggacaaa tagcactaaa ggtcttgttg ggagacttta actacaagca gtatgctgca 2340
   aatgtagagg ctgacataga agcaaattgc gttgggtgta caaattgtat aaagggaata 2400
   acatgtgaaa taaaggtgaa aacagaaatt gaagcatcat gtaaggtaga gcccccatgc 2460
   ccctcttaca caagtagaat tataataaaa ccaggtagag acacccacac attattgatg 2520
   aagtgtgaca aaaaaataac gactggtgaa ctagcaataa aaatatgtaa tatgcaaatc 2580
   caggctcatc tttcaacaat agagagcaat gaccaactag aattgtcgac tggggatcaa 2640
   tcaacatata tacatgaaga agacctaagg tgttcaacct ggatctgcaw wgtgagagac 2700
   gaagggataa gcttcatctt taagccatta acagactggc ttggaagctt cacaaagccg 2760
<210> 66
   <211> 783
   <212> DNA
   <213> artificial sequence
<220>
   <223> synthetic polynucleotide coding fo Gc protein N-terminal alpha-helix region
<400> 66
   ctaagatacc atgagatagc taatatgtta ttgggtaaag ggacaatctc aaaattagat 60
   gtggatatta tagatgcatt gcctgacagg atagctggag acctccagat aatacagtca 120
   agcagtgatt tccaccatca acagatgctg gaatatgtgt ttctattgcg tcattgtgat 180
   tattactcaa gttttgtttc ggataacagc tactcacaaa tcttgtggca aacagcagca 240
   aagttaaatg aaaccaacct ttgcaaacat gagaattcaa agaaatcctg caagtgctta 300
   aaatcaaatg caaattgtgg agagtcagaa gacatcaaca tagcagcaca ggcatactac 360
   aaaaataagg aatctgagct aggacaagat ctaacaacca taatgtacat actcaaaaaa 420
   atgattccag gatcaggtta tagttatctg ctaaatgcag ccaagcacaa caaattagaa 480
   ttgttcaaag cttacatgac taatatttcc tcaaaatatt caaataacaa aaaattaaaa 540
   gatttcatgg cgataataaa tgctatacta gcaaatctgt cagctctctc acatagtagt 600
   gaagaatcaa tctcacaata caaacagatt gatttttctt ccctagtagg cgaaacaaca 660
   gacatgctcg aagcaaattt taatcaaacg atcagtacag acaagataat ttgtgaaaat 720
   cctaaagtaa tgatttgcat ttcacccagg acaaaagttt catccggtga attgtatgtc 780
   tgc 783
<210> 67
   <211> 1293
   <212> DNA
   <213> artificial sequence
<220>
   <223> synthetic polynucleotide coding for Gc prootein N-terminal domain
<400> 67
   gggacagaat ttgaaacatg catcaagaaa gtagaaatga caccggaatg cacaggacct      60
   ttcatggaag ttggaatctg tgatcaccag cagaagagcc taagatacca tgagatagct     120
   aatatgttat tgggtaaagg gacaatctca aaattagatg tggatattat agatgcattg     180
   cctgacagga tagctggaga cctccagata atacagtcaa gcagtgattt ccaccatcaa     240
   cagatgctgg aatatgtgtt tctattgcgt cattgtgatt attactcaag ttttgtttcg     300
   gataacagct actcacaaat cttgtggcaa acagcagcaa agttaaatga aaccaacctt     360
   tgcaaacatg agaattcaaa gaaatcctgc aagtgcttaa aatcaaatgc aaattgtgga     420
   gagtcagaag acatcaacat agcagcacag gcatactaca aaaataagga atctgagcta     480
   ggacaagatc taacaaccat aatgtacata ctcaaaaaaa tgattccagg atcaggttat     540
   agttatctgc taaatgcagc caagcacaac aaattagaat tgttcaaagc ttacatgact     600
   aatatttcct caaaatattc aaataacaaa aaattaaaag atttcatggc gataataaat     660
   gctatactag caaatctgtc agctctctca catagtagtg aagaatcaat ctcacaatac     720
   aaacagattg atttttcttc cctagtaggc gaaacaacag acatgctcga agcaaatttt     780
   aatcaaacga tcagtacaga caagataatt tgtgaaaatc ctaaagtaat gatttgcatt     840
   tcacccagga caaaagtttc atccggtgaa ttgtatgtct gccaaagatc aggaaagtac     900
   tacataatag ataccggcaa attcaagcta tataaattaa ttgacgcaga caatttgtac     960
   tgtgcagcag atgctcagtg taggtatgaa tttagaattt taaccacagg ggaaatgcag    1020
   gcaactttta aagataattg caaagcatca gtacatgtta acaccccagg ataccaaaat    1080
   gaagctataa ctcattgcaa agtgctcgcg aatggtaaat gtaagtataa taatcaaact    1140
   tcaatagata taacattgtg tgctaacaac agatattatc ccgaagcaaa aggcaggatc    1200
   tttggagttg atccagaaga tctatgcttt gataagacat gtactaaaca acagactcca    1260
   tacaacgaag aactaatcag tatgtgtgtt tgg                                 1293
<210> 68
   <211> 762
   <212> DNA
   <213> artificial sequence
<220>
   <223> synthetic polynucleotide coding for fragment of Gc protein N-terminal domain
<400> 68
   gggacagaat ttgaaacatg catcaagaaa gtagaaatga caccggaatg cacaggacct 60
   ttcatggaag ttggaatctg tgatcaccag cagaagagcc taagatacca tgagatagct 120
   aatatgttat tgggtaaagg gacaatctca aaattagatg tggatattat agatgcattg 180
   cctgacagga tagctggaga cctccagata atacagtcaa gcagtgattt ccaccatcaa 240
   cagatgctgg aatatgtgtt tctattgcgt cattgtgatt attactcaag ttttgtttcg 300
   gataacagct actcacaaat cttgtggcaa acagcagcaa agttaaatga aaccaacctt 360
   tgcaaacatg agaattcaaa gaaatcctgc aagtgcttaa aatcaaatgc aaattgtgga 420
   gagtcagaag acatcaacat agcagcacag gcatactaca aaaataagga atctgagcta 480
   ggacaagatc taacaaccat aatgtacata ctcaaaaaaa tgattccagg atcaggttat 540
   agttatctgc taaatgcagc caagcacaac aaattagaat tgttcaaagc ttacatgact 600
   aatatttcct caaaatattc aaataacaaa aaattaaaag atttcatggc gataataaat 660
   gctatactag caaatctgtc agctctctca catagtagtg aagaatcaat ctcacaatac 720
   aaacagattg atttttcttc cctagtaggc gaaacaacag ac 762
<210> 69
   <211> 19
   <212> DNA
   <213> artificial sequence
<220>
   <223> synthetic oligonucleotide (Koongol virus L segment forward primer)
<400> 69
   gacccaatac tgtaaacag 19
<210> 70
   <211> 18
   <212> DNA
   <213> artificial sequence
<220>
   <223> synthetic oligonucleotide (Koongol virus L segment reverse primer)
<400> 70
   cgtcagaatg caccattg 18
<210> 71
   <211> 240
   <212> DNA
   <213> Moissiacense virus
<220>
   <221> CDS
   <222> (1)..(240)
<400> 71
   atg tta gta gaa gta ctt ata acc cag cag atg aat acg ata cgt ttg        48
   Met Leu Val Glu Val Leu Ile Thr Gln Gln Met Asn Thr Ile Arg Leu
   1               5                   10                  15
   tgg gtg tct aca gag aat ttc tca caa ttg ata aca tta gaa tct tct        96
   Trp Val Ser Thr Glu Asn Phe Ser Gln Leu Ile Thr Leu Glu Ser Ser
               20                  25                  30
   tca tca acg ctc caa agg cca aaa ctg cga tgg cta aac tca aga ccg       144
   Ser Ser Thr Leu Gln Arg Pro Lys Leu Arg Trp Leu Asn Ser Arg Pro
           35                  40                  45
   aga cag ttg aag tta cat ttg gaa cac tcg ttg tca agc tgg tta ata       192
   Arg Gln Leu Lys Leu His Leu Glu His Ser Leu Ser Ser Trp Leu Ile
       50                  55                  60
   acc ata gaa ttg gac agg ctc agc gtg agg ttg cgg acg atg agc taa       240
   Thr Ile Glu Leu Asp Arg Leu Ser Val Arg Leu Arg Thr Met Ser
   65                  70                  75
<210> 72
   <211> 79
   <212> PRT
   <213> Moissiacense virus
<400> 72
   Met Leu Val Glu Val Leu Ile Thr Gln Gln Met Asn Thr Ile Arg Leu
   1               5                   10                  15
   Trp Val Ser Thr Glu Asn Phe Ser Gln Leu Ile Thr Leu Glu Ser Ser
               20                  25                  30
   Ser Ser Thr Leu Gln Arg Pro Lys Leu Arg Trp Leu Asn Ser Arg Pro
           35                  40                  45
   Arg Gln Leu Lys Leu His Leu Glu His Ser Leu Ser Ser Trp Leu Ile
       50                  55                  60
   Thr Ile Glu Leu Asp Arg Leu Ser Val Arg Leu Arg Thr Met Ser
   65                  70                  75
<210> 73
   <211> 714
   <212> DNA
   <213> moissiacense virus
<400> 73
   atggctgatt ctaagctcac atacgaggat acacaggatg ttagtagaag tacttataac 60
   ccagcagatg aatacgatac gtttgtgggt gtctacagag aatttctcac aattgataac 120
   attagaatct tcttcatcaa cgctccaaag gccaaaactg cgatggctaa actcaagacc 180
   gagacagttg aagttacatt tggaacactc gttgtcaagc tggttaataa ccatagaatt 240
   ggacaggctc agcgtgaggt tgcggacgat gagctaactc ttcaccgact ttctggctac 300
   attgcaaagt acactcttca cctatacaaa aatgcaaaaa aagcaggaga gagagaagca 360
   ataataacca agatcgttaa tccaatttct gccaaaatgg gcttccaatg gagtgttggg 420
   ccggaaattt atctctcaac gctgccagga accgaaatgt ttctagatac atttaaaatg 480
   ttcccattgg cattcatctt gttaagagtt aaaagaggag agatcaaggt ggaaatggca 540
   aaaaaagcaa tgcggcagaa gtatggcgac aaagatgcca gtgaatggct gcaggaggac 600
   attgagactg taaaaggggc aataaaaaca gttgaaaagc tcaagccaac gctgacagga 660
   ttggctgctt caatgactgc tttcctgcaa caacttggta tcaacactaa gtaa 714
<210> 74
   <211> 24
   <212> RNA
   <213> artificial sequence
<220>
   <223> oligonucleotide primer (RNA equivalent of Cal/Bwa group Fw primer)
<400> 74
   gcaaauggau uugauccuga ugcag 24
<210> 75
   <211> 25
   <212> RNA
   <213> artificial sequence
<220>
   <223> oligonucleotide primer (RNA equivalent of Cal/Bwa group RV primer)
<400> 75
   uuguuccugu uugcuggaaa augau 25
<210> 76
   <211> 25
   <212> RNA
   <213> artificial sequence
<220>
   <223> Moissiacense virus genomic sequence
<400> 76
   agaaguacuu auaacccagc agaug 25
<210> 77
   <211> 25
   <212> RNA
   <213> artificial sequence
<220>
   <223> Moissiacense virus genomic sequence
<400> 77
   cgcugagccu guccaauucu auggu 25
<210> 78
   <211> 26
   <212> RNA
   <213> artificial sequence
<220>
   <223> oligonucleotide primer (RNA equivalent of Bunyamwera group Fw primer)
<400> 78
   cugcuaacac cagcaguacu uuugac 26
<210> 79
   <211> 28
   <212> RNA
   <213> artificial sequence
<220>
   <223> synthetic oligonucleotide (RNA equivalent of Bunyamwera group Rv
   primer)
<400> 79
   uggaggguaa gaccaucguc aggaacug 28
<210> 80
   <211> 26
   <212> RNA
   <213> artificial sequence
<220>
   <223> Moissiacense virus genomic sequence
<400> 80
   aggauguuag uagaaguacu uauaac 26
<210> 81
   <211> 28
   <212> RNA
   <213> artificial sequence
<220>
   <223> Moissiacense virus sequence
<400> 81
   ugaagaguua gcucaucguc cgcaaccu 28
<210> 82
   <211> 29
   <212> RNA
   <213> artificial sequence
<220>
   <223> synthetic oligonucleotide(RNA equivalent of Wyeomyia Fw primer)
<400> 82
   augucugaaa uuguauuuga ugauauugg 29
<210> 83
   <211> 21
   <212> RNA
   <213> artificial sequence
<220>
   <223> synthetic oligonucleotide(RNA equivalent of Wyeomyia Rv primer)
<400> 83
   uauuucgauu ccccggaaag u 21
<210> 84
   <211> 29
   <212> RNA
   <213> artificial sequence
<220>
   <223> Moissiacense virus genomic sequence
<400> 84
   gauucuaagc ucacauacga ggauacaca 29
<210> 85
   <211> 21
   <212> RNA
   <213> artificial sequence
<220>
   <223> Moissiacense virus genomic sequence
<400> 85
   gcugagccug uccaauucua u 21
<210> 86
   <211> 22
   <212> RNA
   <213> artificial sequence
<220>
   <223> synthetic oligonucleotide (RNA equivalent of Oropouche Fw primer)
<400> 86
   ggcccauggu ugaccuuacu uu 22
<210> 87
   <211> 21
   <212> RNA
   <213> artificial sequence
<220>
   <223> RNA equivalent (RNA equivalent of Oropouche Rv primer)
<400> 87
   accaaaggga agaaagugaa u 21
<210> 88
   <211> 22
   <212> RNA
   <213> artificial sequence
<220>
   <223> Moissiacense virus genomic sequence
<400> 88
   ccgagacagu ugaaguuaca uu 22
<210> 89
   <211> 21
   <212> RNA
   <213> artificial sequence
<220>
   <223> Moissiacense virus genomic sequence
<400> 89
   gccaauggga acauuuuaaa u 21

## Description

### FIELD OF THE INVENTION

The invention relates to methods of diagnosis or detection of Moissiacense virus, a novel orthobunyavirus causing human encephalitis, comprising determining the presence of at least one nucleic acid or protein of said virus or antibodies thereto, in a biological sample. The invention also relates to the various diagnostic agents derived from the viral nucleic acids or proteins, in particular nucleic acid primers and probes, antigens and antibodies, and their use for the diagnosis of Moissiacense virus infection and associated disease, in particular encephalitis. The invention further relates to antigens derived from the viral proteins as vaccine for the prevention of Moissiacense virus infection and associated disease, in particular encephalitis.

### BACKGROUND OF THE INVENTION

Human encephalitis is a potentially fatal disease of the central nervous system that is frequently associated with neurological sequelae. The incidence is about 1.5 - 7 cases / year / 100,000 inhabitants. In 20-50% of cases, encephalitis with known etiology is related to infection most often of viral origin (Glaser et al., Clin. Infect. Dis. Off. Publ. Infect. Dis. Soc. Am., 2006, 43, 1565-77). The most frequently implicated viruses belong to the *Herpesviridae* family (Herpes Simplex virus, Varicella Zoster virus and others), but also arboviruses depending on the geographical area, in particular Japanese encephalitis virus (JEV) in Asia and Tick-borne encephalitis virus (TBEV) in Europe.

Encephalitis is one of the most challenging syndromes as its etiology remains unknown in more than one third of the cases, even in countries benefiting from high-performance diagnostic technologies (Venkatesan et al., Clin. Infect. Dis. Off. Publ. Infect. Dis. Soc. Am., 2013, 57, 1114-28).

Indeed, new pathogens provoking encephalitis are recurrently identified (Mailles et al., Clin. Microbiol. Infect. Off. Publ. Eur. Soc. Clin. Microbiol. Infect. Dis., 2017, 23, 607-13). Diagnosis of encephalitis is also challenging because of the geographical spread of neurotropic arboviruses favored by climate change and human migration. In addition, this spread exposes human or animal populations devoid of specific immunity against these new arboviruses (Tabachnick WJ., Annu. Rev. Virol., 2016, 3, 125-45). A recent example is the Usutu virus, a flavivirus of the JEV complex responsible for human encephalitis, which has recently expanded in Europe (Ashraf et al., Viruses, 2015, 7, 219-38).

Etiological diagnosis of encephalitis is essential for appropriate management of patients (Nath A., JAMA Neurol., 2015, 72, 143-144). It is, however, particularly difficult as encephalitis can have autoimmune as well as viral origin (Ashraf et al., Viruses, 2015, 7, 219-38). Inaccurate diagnosis is a main issue as immunosuppressive treatments, in the case of undiagnosed infectious encephalitis, can be deleterious for the patient. In addition, the possibility of an infection underlying an autoimmune reaction renders the situation even more complex (Schein et al., Infection, 2017, 45, 545-9). In this context, identification of new pathogens responsible for encephalitis is critical for improving the scope of targeted diagnostic tests and diagnostic efficiency.

### SUMMARY OF THE INVENTION

The inventors have used untargeted metagenomics to search for pathogens in a fatal case of encephalitis of unknown origin. This approach led to the identification and the characterization of Moissiacense virus, a novel orthobunyavirus related to viruses of the Koongol group, a clade that had never previously been identified in vertebrates. It is of note that standard approach using PCR consensus assays targeting a broad range of orthobunyaviruses would have missed Moissiacense virus due to sequence variation. Viral sequences of the same clade were found in *Culex pipiens* mosquitoes captured in Southern France, which suggests that a novel arbovirus clade with neurotropic potential is present in Europe. Thus, Moissiacense virus should be added to serological tests and to targeted tests in routine diagnosis of brain tissue from encephalitis cases with unidentified etiology.

Therefore, in the various embodiments, the invention relates to methods of diagnosis or detection of Moissiacense virus, a novel orthobunyavirus causing human encephalitis, comprising determining the presence of at least one nucleic acid or protein of said virus or antibodies thereto, in a biological sample. The invention also relates to the various diagnostic agents derived from the viral nucleic acids or proteins, in particular nucleic acid primers and probes, antigens and antibodies, and their use for the diagnosis of Moissiacense virus infection and associated disease, in particular encephalitis. The invention further relates to antigens derived from the viral proteins as vaccine for the prevention of Moissiacense virus infection and associated disease, in particular encephalitis.

### DETAILED DESCRIPTION OF THE INVENTION

### Methods of diagnosis or detection of Moissiacense virus

The invention relates to methods of diagnosis or detection of Moissiacense virus, comprising determining the presence of at least one nucleic acid or protein of said virus or of antibodies against said virus, in a biological sample.

Moissiacense virus is a new neurotropic arbovirus belonging to orthobunayviruses, a genus of viruses within the family *Peribunyaviridae* in the order *Bunyavirales.* Orthobunyaviruses are spherical enveloped virus (80 nm to 120 nm in diameter) comprising a segmented Negative-stranded RNA linear genome encapsulated in a ribonucleocapsid. The L segment (about 6.9kb) encodes the viral RNA dependent RNA Polymerase or L Protein. The M segment (about 4.5kb) encodes a polyprotein which is cleaved by host protease into Gn, NSm and Gc proteins. The S segment (about 1kb) encodes the Nucleocapsid protein (N protein) and a non-structural protein (NSs Protein), by leaky scanning. The type species or prototype of orthobunyavirus is Bunyamwera (BUNV). As used herein, a bunyavirus refers to an orthobunyavirus. Unless otherwise specified herein, "Moissiacense virus", refers to the Moissiacense virus group or species which includes in particular, Moissiacense virus, a new virus identified by the inventors of the present application, as well as the known, Umbre and Little Sussex viruses. The Moissiacense virus group is a subset of the Koongol virus group or clade which does not include Koongol virus. The sequences disclosed herein are those of the newly identified Moissiacense virus. All the sequences disclosed herein are in the 5' to 3' orientation. According to the standard practice in the field of bunyavirus (negative-sense single stranded RNA virus), viral nucleotide sequences are disclosed as the positive strand or sense strand coding sequences of the S, M, and L segments, in the DNA form. However, the present invention encompasses the RNA equivalent of the DNA sequences herein disclosed as well as their complement (i.e., reverse complement) sequences.

A sample or biological sample refers to any material obtained or derived from living or dead individual (human or animal) that may contain a Moissiacense virus or components thereof such as nucleic acids, proteins or fragments thereof, or antibodies against said Moissiacense virus appropriate for detection by the methods of the invention. Biological sample also includes any material derived or obtained from an arthropod vector that may contain a Moissiacense virus or components thereof such as nucleic acids, proteins or fragments thereof. The source of the sample may be any solid tissue as from fresh, frozen and/or preserved organ or tissue sample or biopsy or aspirate; any body fluid such as whole-blood or any blood constituent (serum, plasma), cerebral spinal fluid (CSF), peritoneal, pleural, synovial or amniotic fluid, urine, lymph and mucosal secretions. The sample may also be primary or cultured cells. The source of the sample can also be a whole vector specimen, a portion thereof or egg(s) thereof. The sample can be derived from any vector capable of transmitting Moissiacense virus, in particular a mosquitoe such as *Culex sp* or *Aedes sp.,* or others. Samples include also processed samples that have been treated to disrupt tissue or cell structure, thus releasing intracellular components into a solution which may further contain reagents (buffers, salts, detergents, enzymes and the like) which are used to prepare, using standard methods, a biological sample for analysis. In particular, processed samples include samples that have been treated by standard methods used for the isolation of nucleic acids or proteins from biological samples.

In some embodiments of the methods of the invention, the sample is a clinical sample (*i.e.,* a sample from living or dead individual (human or animal) suspected of having a Moissiacense virus). Preferably, the sample is brain biopsy, cerebral spinal fluid (CSF), whole-blood, plasma or serum. In some other embodiments, the biological sample is an arthropod vector specimen.

### Nucleic acid detection

In some aspects, the method of diagnosis or detection of Moissiacense virus comprises determining the presence of at least one nucleic acid of said virus in a sample.

In some embodiments, the viral nucleic acid is selected from the group consisting of:
- a first nucleic acid (S segment) comprising a sequence having at least 80% identity with SEQ ID NO: 1 and further including a coding sequence for a Nucleocapsid (N) protein, or its complement;
- a second nucleic acid (M segment) comprising a sequence having at least 80% identity with SEQ ID NO: 3 and further including a coding sequence for a polyprotein M, or its complement; and
- a third nucleic acid (L segment) comprising a sequence having at least 80% identity with SEQ ID NO: 5 and further including a coding sequence for a polymerase (L) protein, or its complement.

The first nucleic acid corresponds to the S segment which codes for the Nucleocapsid (N) protein or Nucleoprotein and for the nonstructural protein NSs. The N protein coding sequence or ORF is from positions 59 to 772 of SEQ ID NO: 1 and has the sequence SEQ ID NO: 73. The NSs protein coding sequence or ORF is from positions 96 to 335 of SEQ ID NO: 1 and has the sequence SEQ ID NO: 71.

The Nucleocapsid (N) protein refers to the protein of SEQ ID NO: 2 or a functional variant thereof. The protein of SEQ ID NO: 2 and its functional variants have the general properties of a bunyavirus nucleocapsid protein, taking as reference Bunyamwera (BUNV-N: GenBank NP_047213.1). The bunyavirus N protein is the major component of the viral nucleocapsid. This protein is thought to interact with the L protein, virus RNA and/or other N proteins.

The NSs protein refers to the protein of SEQ ID NO: 72 or a functional variant thereof. The protein of SEQ ID NO: 72 and its functional variants have the general properties of a bunyavirus NSs protein, taking as reference Bunyamwera (BUNV-NSs: GenBank NP_047214.1). The bunyavirus NSs protein plays a role in the escape of host innate immune response by promoting the degradation of host EIF2AK2/PKR and inhibiting host transcription. Cytoplasmic NSs interacts with host FBXW11 to degrade PKR whereas nuclear pool binds to host FBXO3 to target TFIIH subunit GTF2H1 for proteasomal degradation.

The second nucleic acid corresponds to the M segment which codes for the two virion glycoproteins, Gn (G2) and Gc (G1), and a nonstructural protein NSm, in the form of a polyprotein precursor (polyprotein M or M polyprotein) which is cotranslationally cleaved. The gene order is 5'Gn-NSm-Gc-3'. The polyprotein M coding sequence or ORF is from positions 1 to 4395 of SEQ ID NO: 3.

The polyprotein M refers to the protein precursor of SEQ ID NO: 4 which is cotranslationally cleaved into the two virion glycoproteins, Gn (G2) and Gc (G1), and nonstructural protein NSm, or a functional variant thereof. Gn is from positions 17 to 301 of SEQ ID NO: 4 and has the sequence SEQ ID NO: 58; NSm is from positions 302 to 475 of SEQ ID NO: 4 and has the sequence SEQ ID NO: 59, and Gc is from positions 476 to 1464 of SEQ ID NO: 4 and has the sequence SEQ ID NO: 60. The polyprotein of SEQ ID NO: 4, the derived Gn (G2), Gc (G1), and NSm proteins and their functional variants have the general properties of bunyavirus M polyprotein, Gn, Gc and NSm proteins, taking as reference Bunyamwera (BUNV-M: Uniprot P04505-1 or GenBank NP_047212.1). Using BUNV polyprotein M (1433 aa) as reference sequence, Gn is from positions 17 to 302, NSm is from positions 303 to 477, and Gc is from positions 478 to 1433. Glycoprotein N (Gn) and Glycoprotein C (Gc) interact with each other and are present at the surface of the virion. They are able to attach the virion to a cell receptor and to promote fusion of membranes after endocytosis of the virion. Non-structural protein M (NSm) plays a role in virion budding at Golgi tubes and in subcellular location of Glycoprotein C.

The third nucleic acid corresponds to the L segment which codes for the RNA-dependent or RNA-directed RNA polymerase (L protein) also named as large structural protein, Replicase, Transcriptase or RdRp. The L protein coding sequence or ORF is from positions 1 to 6828 of SEQ ID NO: 5.

The polymerase (L) protein refers to the protein of SEQ ID NO: 6 or a functional variant thereof. The protein of SEQ ID NO: 6 and its functional variants have the general properties of a bunyavirus L protein, taking as reference Bunyamwera (BUNV-L: UniProt P20470-1). The bunyavirus RNA-dependent RNA polymerase (RdRp or L protein) is responsible for replication and transcription of the viral RNA genome using antigenomic RNA as an intermediate. During transcription, it synthesizes subgenomic RNAs and assures their capping by a cap-snatching mechanism. These short capped RNAs are then used as primers for viral transcription. Bunyavirus RdRp have eight conserved motifs (PreA:F, A, H, B, C, D and E) in their central region or core polymerase domain (positions 951 to 1220 of BUN-L). The core polymerase domain of Moissiacense virus RdRP including the eight conserved motifs is from positions 954 to 1246 of SEQ ID NO: 6.

The percent amino acid or nucleotide sequence identity is defined as the percent of amino acid residues or nucleotides in a Compared Sequence that are identical to the Reference Sequence after aligning the sequences and introducing gaps if necessary, to achieve the maximum sequence identity. Alignment for purposes of determining percent amino acid or nucleotide sequence identity can be achieved in various ways known to a person of skill in the art, for instance using publicly available computer software such as the GCG (Genetics Computer Group, Program Manual for the GCG Package, Version 7, Madison, Wisconsin) pileup program, or any of sequence comparison algorithms such as BLAST (Altschul et al., J. Mol. Biol., 1990, 215, 403-10), FASTA or CLUSTALW.

Individual refers to a mammal, including a human or a domestic animal, preferably a human.

As used herein "the diagnosis of Moissiacense virus infection" includes the diagnosis of any Moissiacense virus associated disease, in particular encephalitis.

The terms "a", "an", and "the" include plural referents, unless the context clearly indicates otherwise. For example "a target" as used herein is understood to represent one or more targets. As such, the term "a" (or "an"), "one or more" or "at least one" can be used interchangeably herein.

In some embodiments, the S segment comprises a sequence having at least 85% or more identity with SEQ ID NO: 1; the M -segment comprises a sequence having at least 85% or more identity with SEQ ID NO: 3; and/or the L segment comprises a sequence having at least 85% or more identity with SEQ ID NO: 5.

In some embodiments, the nucleocapsid (N) protein has at least 90% identity with SEQ ID NO: 2; the polyprotein M has at least 90% identity with SEQ ID NO: 4 and comprises the Gn protein from positions 17 to 301 (SEQ ID NO: 58), the NSm from positions 302 to 475 (SEQ ID NO: 59) and the Gc protein from positions 476 to 1464 (SEQ ID NO : 60); and/or the L polyprotein has at least 90% identity with SEQ ID NO: 6 including at least 90% identity on the core polymerase domain (positions 954 to 1246 of SEQ ID NO : 6).

The method of the invention is used for the detection or diagnosis of any virus of the Moissiacense group or species, in particular Moissiacense virus, Umbre virus, Little Sussex virus or any variant thereof. In some preferred embodiments, said virus is Moissiacense virus or any variant thereof.

The viral nucleic acid or target nucleic acid may be genomic RNA (viral RNA or vRNA), complementary RNA (cRNA) or viral mRNA. Genomic RNA or viral RNA (vRNA) is negative-sense RNA comprising the reverse complement of one of the above-mentioned coding sequences, in the RNA form. Complementary RNA (cRNA) is positive-sense RNA comprising one of the above-mentioned coding sequences, in the RNA form. Viral mRNA comprises one of the above-mentioned coding sequences, in the RNA form. In some preferred embodiments of the methods of the invention, the viral nucleic acid is viral RNA (*i.e.* Moissiacense virus genomic RNA).

The diagnosis or detection method of the invention comprises the detection of a target sequence of the viral nucleic acid. The target sequence refers to the particular nucleotide sequence of the viral nucleic acid that is to be detected by any appropriate mean such as hybridization with an oligonucleotide probe, amplification with a pair of oligonucleotide primers, sequencing, in particular high-throughput sequencing, or a combination thereof. For example, an amplification product (amplicon) of the target sequence obtained by amplification of viral nucleic acid from the sample may be detected by using a DNA binding dye or labelled primer, or hybridization assay using a labelled oligonucleotide probe. As used herein, the detection of a target sequence of the viral nucleic acid refers to the direct detection and the detection following another step performed on the viral nucleic acid such as a reverse transcription step and an amplification of said viral nucleic acid.

The target sequence includes the sequence to which oligonucleotide probe hybridizes during the nucleic acid detection process and the sequences to which oligonucleotide primers hybridize during the nucleic acid amplification process. The target sequence that is detected directly using an oligonucleotide probe consists usually from a lower limit of 15 to 20 consecutive nucleotides to an upper limit of 45 to 60 consecutive nucleotides of one of the above-mentioned viral nucleic acid sequences. The target sequence that is amplified and detected consists usually of at least 50 consecutive nucleotides, preferably at least 100 or 150 consecutive nucleotides of one of the above-mentioned viral nucleic acid sequences. Preferably, the target sequence consists of 150 to 600 consecutive nucleotides, more preferably 300 to 500 consecutive nucleotides of one of the above-mentioned viral nucleic acid sequences. The target nucleic acid being originally single-stranded, the term target sequence will also refer to the sequence complementary to the target sequence as present in the viral nucleic acid.

The target sequence is a unique sequence which is specific for Moissiacense virus. Thus, the detection of the target sequence indicates the presence of Moissiacense virus in the sample. Therefore, the detection of the target sequence in a sample from the individual is indicative of whether the individual is suffering from Moissiacense virus infection and in particular from a disease caused by Moissiacense virus such as encephalitis.

By comparing the L, M and S segment sequences of Moissiacense virus which are disclosed in the present application with that of other bunyavirus species or other members of the order *Bunyavirales* which are available in the sequence databases, one skilled in the art can easily identify a unique target sequence which is specific for Moissiacense virus using standard nucleotide sequence analysis softwares that are well-known in the art.

In some embodiments, the method of the invention comprises the detection of at least one target sequence selected from the group consisting of:
a) a sequence SEQ ID NO: 7 situated in the region from positions 23 to position 338 of the S segment sequence SEQ ID NO: 1;
b) a sequence SEQ ID NO: 8 situated in the region from positions 3706 to 4130 of the M segment sequence SEQ ID NO: 3;
c) a sequence SEQ ID NO: 9 situated in the region from positions 3312 to 3768 of the L segment sequence SEQ ID NO: 5;
d) a sequence of at least 15 nucleotides, preferably at least 50, 100, 150, 200 or 300 nucleotides from any one of SEQ ID NO: 7 to 9; and
e) a sequence comprising any one of SEQ ID NO: 7 to 9 and up to 40 consecutive nucleotides, preferably up to 35, 30, 25 or 20 (2, 3, 4, 5, 6, 7, 8, 9, 10, 11, 12, 13, 14 ,15, 16, 17, 18, 19 and 20), more preferably up to 15 (2, 3, 4, 5, 6, 7, 8, 9, 10, 11, 12, 13, 14 ,15), up to 10 (2, 3, 4, 5, 6, 7, 8, 9, 10) or up to 5 (2, 3, 4, 5) consecutive nucleotides of 5' and/or 3' flanking sequence from said S, M or L segment sequence.

The target sequences listed above are species specific. They allow the detection of viruses of the Moissiacense group or species including at least Moissiacence, Umbre and Little Sussex viruses. The target sequence consists of or is included, comprised in or contained in the target region. In some preferred embodiment, the target sequence consists of the target region as defined above.

Nucleic acid amplification techniques include target amplification systems, probe amplification systems and signal amplification. Target amplification systems include with no limitations: Polymerase Chain Reaction (PCR) including RT-PCR, nested PCR and multiplex PCR; Nucleic Acid Sequence-Based Amplification (NASBA), Transcription Mediated Amplification (TMA), Strand Displacement Amplification (SDA) and isothermal amplification such as reverse-transcription loop-mediated isothermal amplification (RT-LAMP). Target nucleic acid amplification is performed using any of the various natural or engineered enzymes available for this purpose. For example, PCR can be performed using any thermostable polymerase such as with no limitations: Taq, Vent, Pfu, Tth and variants thereof. Signal amplification includes Branched DNA probes (bDNA) and Hybrid capture assay using anti-DNA-RNA hybrid antibody conjugated to a label such as phosphatase alkaline. Probe amplification includes Ligase Chain Reaction (LCR) and Cleavase Invader (FEN-1 DNA polymerase (cleavase)). Hybridization is preferably sequence-specific hybridization, i.e., hybridization with a nucleic acid probe that specifically hybridizes to a target sequence specific for Moissiacense virus.

Since the viral nucleic acid is RNA, the method of the invention comprise a reverse transcription reaction prior to or concomitantly with the amplification reaction of the target sequence. The reverse transcription reaction is performed using any reverse transcriptase (RT). RT are well-known in the art and include for example Avian Myeloblastosis Virus (AMV) and Moloney Murine Leukemia virus (MMLV) RT. The Reverse transcription and DNA amplification can be performed in the same reaction mixture comprising the DNA polymerase and RT enzymes.

The amplification product is detected using any of the various methods available for this purpose which are well-known in the art. For example, the detection method is fluorescence, bioluminescence, colorimetry or immunoenzymatic detection. The detection may be semi-quantitative or quantitative. The detection may also be real-time detection, wherein the signal resulting from the presence of the amplification product is measured during the course of the nucleic acid amplification reaction to monitor the accumulation of specific amplification products. Fluorescence may use DNA intercaling dyes or fluorescent molecular beacon probes. Immunoassays include Enzyme-linked immunosorbent assays (ELISA) and lateral flow.

In some preferred embodiments, the method of the invention comprises:
- subjecting said sample to a nucleic acid amplification reaction using at least one pair of oligonucleotide primers for amplifying a target sequence of said viral nucleic acid, and
- detecting the presence of an amplification product for said target sequence.

The target amplification reaction is performed using any nucleic acid target amplification techniques as defined above. In some preferred embodiments, target amplification is performed by RT-PCR techniques using suitable enzymes for that purpose as defined above. The amplification product includes monomers and concatemers of the target sequence. The amplification product may be detected using any appropriate means available for that purpose as disclosed above such as by using a labelled primer or probe or a DNA binding dye. In the second step of the method of the invention, the detection of the presence or absence of the amplification product determines the presence or absence of a Moissiacence virus in the sample, and thereby whether or not the individual is infected with Moissiacense virus.

Oligonucleotide refers to a polymer of 5 to 100 nucleotides, preferably from a lower limit of 15 to 20 nucleotides to an upper limit of 45 to 60 nucleotides, wherein the polymer comprise ribonucleotides, deoxyribonucleotides, modified nucleotides or mixtures thereof and may further include modified internucleotide linkages and/or modified 5' and/or 3' termini. For example, the oligonucleotide can be DNA, RNA, PNA or mixed, and may comprise locked nucleic acids (LNA). Oligonucleotides are usually synthesized using any of a variety of well-known enzymatic or chemical methods. Oligonucleotide includes a probe or primer. The oligonucleotide probe or primer according to the invention is substantially complementary to the target sequence. Substantially complementary means that the oligonucleotide is at least 80% identical, preferably at least 85%, 90%, 95%, 96%, 97% 98% or 99% identical to the target sequence. The oligonucleotide may comprise additional sequences (not complementary to the target sequence) at its 5' end. In some embodiments the oligonucleotide comprises a sequence of at least 5, preferably 10 to 15 consecutive nucleotides which is 100% identical to the target sequence. In some more preferred embodiments, the oligonucleotide sequence is 100% identical to the target sequence.

The oligonucleotides which function as probes are capable of hybridizing specifically to the target sequence. They advantageously include a label to detect the target nucleic acid. The oligonucleotides which function as primers are capable of annealing specifically to the target sequence and can be further extended in the presence of a nucleic acid polymerase to specifically amplify the target sequence. At least one oligonucleotide may also function as a probe and further includes a detectable label to detect a target nucleic acid or an amplicon thereof.

The label is any moiety that can be detected directly or indirectly by the production of a detectable signal such as luminescent (radioluminescent, chemiluminescent, bioluminescent, fluorescent or phosphorescent), radioactive, colorimetric, magnetic signal or others. Directly detectable labels include radioisotopes and fluorophores. Indirectly detectable labels are detected by labelling with additional reagents that enable the detection. Indirectly detectable labels include, for example, chemiluminescent agents, enzymes that produce visible or coloured reaction products. Indirectly detectable labels also include ligand-detectable ligand binding partners, for example based on avidin or streptavidin/biotin complex, such as with no limitations a biotinylated oligonucleotide (for nucleic acid detection) or antibody (for protein detection) and streptavidin conjugated with a label (fluorophore or enzyme).

The oligonucleotides are designed by comparing the L, M and S segment sequences of the Moissiacense virus which are disclosed in the present application with that of other bunyaviruses or other members of the order *Bunyavirales* which are available in sequence databases, using general principles for designing amplification primers or probes that are well-known in the art. The design of the primers may also include specific principles for designing primers for use in specific amplification methods. Appropriate softwares for designing oligonucleotide primers or probes are available in the art. The sequences of the oligonucleotide primers or probe are specific for the target sequence of the Moissiacense virus. This means that they are chosen to hybridize specifically to the target sequence of the Moissiacense virus but not to related target sequences of other bunyaviruses or other members of the order *Bunyavirales* which infect humans or animals, under the hybridization conditions which are used for the methods of the invention. It is within the skills of one of ordinary skill in the art to choose the sequences of the oligonucleotide probe or primes pair and adapt the hybridization conditions (Temperature, salt concentration) of said oligonucleotide probe or primer pair to permit only hybridization with nucleic acid of Moissiacense virus.

In some embodiments of the above method, the oligonucleotide probe or primers are selected from the group consisting of: the sequences of 5 to 100 nucleotides, preferably 15 to 60 nucleotides having 80% to 100% identity, preferably at least 85%, 90% 95%, 96 %, 97%, 98% or 99% identity with any of SEQ ID NO: 1, 3, 5, and 7 to 9 or with a sequence comprising any of SEQ ID NO: 7 to 9 and up to 40 consecutive nucleotides, preferably up to 35, 30, 25 or 20 (2, 3, 4, 5, 6, 7, 8, 9, 10, 11, 12, 13, 14 ,15, 16, 17, 18, 19 and 20), more preferably up to 15 (2, 3, 4, 5, 6, 7, 8, 9, 10, 11, 12, 13, 14 ,15), up to 10 (2, 3, 4, 5, 6, 7, 8, 9, 10) or up to 5 (2, 3, 4, 5) consecutive nucleotides of 5' and/or 3' flanking sequence from said S, M or L segment sequence, and their complement.

In some preferred embodiments, the oligonucleotide probe or primers are selected from the group consisting of the sequences SEQ ID NO: 10 to 57. The primer pairs are advantageously selected from the group consisting of: SEQ ID NO: 10-11, 12-13, 14-15, 16-17, 18-19, 20-21, 22-23, 24-25, 26-27, 28-29, 30-31, 32-33, 34-35, 36-37, 38-39, 40-41, 42-43, 44-45, 46-47, 48-49, 50-51, 52-53, 54-55, and 56-57. The primer pairs SEQ ID NO: 10-11, 12-13, and/or 14-15 are advantageously used for the detection of any virus of the Moissiacense group, in particular Moissiacense virus, Umbre virus, Little Sussex virus or any variant thereof.

### Antigen or antibody detection

In some aspects, the method of detection or diagnosis of Moissiacense virus comprises determining the presence of at least one protein of said virus or antibodies thereto in a sample.

The detection or diagnosis is generally performed by immunoassay using an antigen from virus Moissiacense or antibody thereto. Immunoassays are well-known techniques for protein or antibody detection which rely on the detection of antigen-antibody complexes using an appropriate label. The method of the invention may use any immunoassay such as with no limitations, immunoblotting, immunoprecipitation, ELISA, immunocytochemistry or immunohistochemistry, and immunofluorescence like flow cytometry assay, and FACS. The method of the invention may use any appropriate label used in immunoassays such as enzymes, biotin, fluorescent dyes/proteins or others.

In some embodiments, the antigen from Moissiacense virus comprises the amino acid sequence of the N, Gn or Gc protein or a fragment thereof. A fragment refers to a functional fragment, which means a fragment of at least 10 amino acids that is bound by antibodies against Moissiacense virus. In some embodiments, the fragment comprises at least 50, 100, 200, 300 or more amino acids.

In some preferred embodiments, the antigen comprises the N protein amino acid sequence or a fragment thereof, preferably a sequence having 90% to 100% identity with SEQ ID NO: 2, or a fragment of at least 10 amino acids thereof which is bound by antibodies against Moissiacense virus.

In some other preferred embodiments, the antigen comprises an extracellular fragment of Gc protein, preferably chosen from:
- a sequence SEQ ID NO: 61 comprising the extracellular domain (positions 476 to 1395 on the polyprotein M of SEQ ID NO: 4);
- a sequence SEQ ID NO: 62 comprising the N-terminal alpha-helix (positions 510 to 770 on the polyprotein M of SEQ ID NO: 4);
- a sequence SEQ ID NO: 63 comprising the N-terminal domain (positions 477 to 907 on the polyprotein M of SEQ ID NO: 4), and
- a sequence of at least 50 amino acids from any one of SEQ ID NO: 61 to 63, in particular SEQ ID NO: 64 (positions 477 to 730 of SEQ ID NO: 4).

An antibody refers to an antibody against a protein of Moissiacense virus, preferably the N, Gn or Gc protein, more preferably an antibody which binds an extracellular epitope of the Gn or Gc protein that is exposed on the surface of virion particles. The antibody according to the invention recognizes specifically said viral protein, in particular Gn or Gc protein on the plasma membrane of infected cells. Such recognition can be determined by standard immunofluorescence assay like flow cytometry assay. The antibody has a relatively high affinity to its epitope of said viral protein, but do not substantially recognize and bind to peptides other than the one(s) of interest. As used herein, the term "relatively high affinity" means a binding affinity between the antibody and the protein of interest of at least 10⁻⁶ M, and preferably of at least about 10⁻⁷ M and even more preferably 10⁻⁸ M to 10⁻¹⁰ M. Determination of such affinity is preferably conducted under standard competitive binding immunoassay conditions which is common knowledge to the person of ordinary skill in the art. The term "antibody" is meant to encompass a whole antibody or antigen binding fragment thereof, an aggregate, polymer, derivative, or conjugate of antibody. The antibody may be monoclonal, polyclonal, non-recombinant or recombinant, chimeric or humanized.

The antigen or antibody may include an appropriate label for antibody or antigen detection, for example in the form of a fusion protein or conjugate. In some embodiments, the antigen is fused with an enzyme such as luciferase.

The antigen of the invention is prepared by the conventional techniques known to those skilled in the art, in particular by expression from an appropriate recombinant expression vector in a suitable cell system (eukaryotic including mammalian and insect cells or prokaryotic). In particular embodiments, the vector comprises any one of SEQ ID NO: 65 to 68 and 73 or a fragment thereof

The antibody of the invention can be produced by the conventional techniques known to those skilled in the art. For example, they may be produced by immunization of an animal with an antigen from virus Moissiacense, in particular a recombinant antigen as define above.

The antigen and antibody are specific for Moissiacense virus and usually do not exhibit substantial cross-reactivity with other pathogens that infect human or animal individuals, under the conditions for antigen-antibody complex formation that are used in the methods of the invention. It is within the skill of a person having ordinary skill in the art to prepare specific antigen and antibodies and adapt the conditions of the method to detect the specific binding of the diagnostic agent (antigen or antibody) to its antibody or antigen counterpart from Moissiacense virus.

In some embodiments, the method of detection or diagnosis of Moissiacense virus comprises the step of:
- incubating an antigen from Moissiacense virus or antibody thereto with the biological sample to form a mixture; and
- detecting antigen-antibody complexes in the mixture.

The sample for anti-Moissiacense virus antibody detection is preferably body fluid from the individual, in particular serum. The sample for viral protein detection may be tissue sample, in particular biopsied tissue such as brain biopsy, or body fluid such as cerebral spinal fluid (CSF), whole-blood or serum.

The antigen or antibody is preferably labeled and the antigen-antibody complexes are detected by measuring the signal from the label by any appropriate means available for that purpose as disclosed above.

In some embodiments, antibody detection is performed by luciferase immunoprecipitation using an antigen fused with luciferase, preferably an antigen comprising SEQ ID NO: 61 or 62.

In some embodiments, antibody detection is performed by ELISA using an antigen comprising SEQ ID NO: 63 or 64.

In some embodiments, the detecting step comprises the determination of the amount of bound antibody or bound antigen in the mixture, and optionally, comparing the amount of bound antibody or bound antigen in the mixture with at least one predetermined value.

The detection of the viral nucleic acid target sequence, protein or antibody thereto in a sample from the individual using the methods of the invention is indicative of whether the individual is suffering from Moissiacense virus infection and in particular from a disease caused by Moissiacense virus such as encephalitis.

Therefore, the above methods of the invention are useful for the diagnosis of Moissiacense virus infection in an individual, in particular the diagnosis of the disease caused by Moissiacense virus, ranging from febrile illness to central nervous system involvement such as encephalitis.

In some embodiments, the above methods comprise the step of deducing therefrom whether the individual is suffering from Moissiacense virus infection and in particular from a disease caused by Moissiacense virus such as encephalitis.

In some embodiments in connection with this aspect of the invention, the above methods comprise a further step of administering an appropriate treatment to the individual depending on whether or not the individual is diagnosed with Moissiacense virus infection and in particular with a disease caused by Moissiacense virus such as encephalitis.

The above methods of the invention are in particular useful for the differential diagnosis of encephalitis, in particular human encephalitis. In some embodiments, in connection with this aspect of the invention, the same biological sample from an individual may be subjected, simultaneously or separately, to a method of detection or diagnosis of at least another pathogen, in particular at least another encephalitis-causing agent such as encephalitis virus. Encephalitis virus includes in particular Herpesvirus such as HSV and VZV, enteroviruses, polyomaviruses, astroviruses, measles virus, mumps virus, and various arboviruses such as without limitation, West Nile, JEV and TBEV.

The above methods of the invention are also useful for the detection of Moissiacense virus in an arthropod vector population to study virus Moissiacense dissemination across geographic areas for epidemiological surveys.

In some preferred embodiments, the above methods of the invention comprise assaying several samples simultaneously, and/or assaying different target sequences, antigens or antibodies simultaneously on the same sample, in parallel in separate reaction mixtures, or in the same reaction mixture and detected independently. In some advantageous embodiments, the methods comprise assaying multiple samples, targets, antigens, and/or antibodies simultaneously in a high-throughput process.

### Kits for the diagnosis or detection of Moissiacense virus

Another aspect of the invention is a kit for the diagnosis or detection of Moissiacense virus, comprising at least one oligonucleotide probe or primer, antigen or antibody thereto for the detection of Moissiacense virus nucleic acid, antibody or protein, as defined above, preferably further including a detectable label. In particular, the kit comprises at least one pair of oligonucleotide primers for amplifying said target; preferably wherein at least one oligonucleotide of the pair may comprise a detectable label.

In some embodiments, the kit is for the differential diagnosis of encephalitis, in particular human encephalitis. In some particular embodiments, in connection with this aspect of the invention, the kit further comprises at least one oligonucleotide probe or primer, antigen or antibody thereto for the detection of least another encephalitis-causing agent such as encephalitis virus, as defined above. The encephalitis virus is in particular chosen from Herpesvirus such as HSV and VZV, enteroviruses, polyomaviruses, astroviruses, measles virus, mumps virus, and various arboviruses such as without limitation, West Nile, JEV and TBEV. The at least one oligonucleotide probe or primer, antigen or antibody for the detection of said other(s) encephalitis-causing agent(s) preferably further includes a detectable label. In particular, the kit comprises at least one pair of oligonucleotide primers for amplifying a target sequence of the nucleic acid of at least another encephalitis-causing agent such as encephalitis virus as defined above; preferably wherein at least one oligonucleotide of the pair may comprise a detectable label.

The kit optionally comprises reagents for the amplification of the target sequence and/or the detection of the amplification product or antigen/antibody complex. Reagents available for this purpose are well-known in the art and include the DNA polymerases and RT enzymes described above, buffers or substrates for the enzymes, detergents, enhancing agents, nucleic acid binding dyes and probes, preferably labelled probes, secondary antibody conjugated to a label, avidin/streptavidin conjugated to a label. In some preferred embodiments of the kit of the invention, the primers, probe, antigen or antibody, and optional reagents are in lyophilised form to allow ambient storage. The components of the kits are packaged together into any of the various containers suitable for nucleic acid amplification or antigen/antibody complex detection such as plates, slides, wells, dishes, beads, particles, cups, strands, chips, strips and others. The kit optionally includes instructions for performing at least one specific embodiment of the method of the invention. In some advantageous embodiments, the kit comprises micro-well plates or microtubes, preferably in a dried format, *i.e.,* wherein the wells of the plates or microtubes comprise a dried composition containing at least the primers, probe, antigen or antibody, and preferably further comprising all the reagents for the amplification of the target sequence and/or the detection of the amplification product amplification or antigen/antibody complex. In some other advantageous embodiments, the primers, probe, antigen or antibody and optional reagents are included into any of the devices available for nucleic acid amplification or immunoassay including devices further integrating nucleic acid extraction and/or amplification product detection capacities such as microfluidic devices or other devices.

Another aspect of the invention, relates to an oligonucleotide probe or primer, antigen or antibody thereto for the detection of Moissiacense virus nucleic acid, antibody or protein, as defined above. The invention also relates to the use of an oligonucleotide probe or primer, antigen or antibody thereto for the detection of Moissiacense virus nucleic acid, antibody or protein as defined above, as diagnostic agent for the *in vitro* detection or diagnosis of Moissiacense virus. The invention encompasses combinations of the above diagnostic agents for the detection of Moissiacense virus with other diagnostic agents, in particular oligonucleotide probe(s) or primer(s), antigen(s) or antibodies thereto, for the detection of other encephalitis-causing agent(s) as defined above and their use for the differential diagnosis of encephalitis, in particular human encephalitis.

### Immunogenic or vaccine compositions

Another aspect of the invention, relates to an immunogenic or vaccine pharmaceutical composition comprising, as active substance an antigen from Moissiacense virus as defined above, in association with at least one pharmaceutically acceptable vehicle.

The pharmaceutical vehicles are those appropriate to the planned route of administration, which are well known in the art.

The pharmaceutical composition may further comprise a carrier and/or adjuvant. Non-limitative examples of carriers suitable for use in the composition of the invention include uni- or multi-lamellar liposomes, ISCOMS, virosomes, viral pseudo-particules, saponin micelles, saccharid (poly(lactide-co-glycolide)) or gold microspheres, and nanoparticules. Non-limitative examples of adjuvants suitable for use in the composition of the invention include: CpG oligodeoxynucleotide, Freund adjuvant, polyI:C (polyinosine-polycytidylic acid), oil emulsion, mineral substances, bacterial extracts, saponin, aluminium salts, monophosphoryl-lipid A and squalene.

The pharmaceutical composition comprises a therapeutically effective amount of the antigen sufficient to induce a protective immune response against Moissiacense virus infection in the individual to whom it is administered. The pharmaceutically effective dose depends upon the composition used, the route of administration, the type of mammal (human or animal) being treated, the physical characteristics of the specific mammal under consideration, concurrent medication, and other factors, that those skilled in the medical arts will recognize.

The invention provides also an antigen according to the invention for use as a medicament.

The invention provides also an antigen or pharmaceutical composition according to the invention for use in the prevention of Moissiacense virus infection and associated disease, in particular encephalitis.

The invention provides also a method for preventing treating Moissiacense virus infection and associated disease, in particular encephalitis in an individual, comprising: administering a therapeutically effective amount of the pharmaceutical composition according to the invention to the individual.

The pharmaceutical composition of the present invention is generally administered according to known procedures, at dosages and for periods of time effective to induce a beneficial effect in the individual. The administration may be by injection.

The practice of the present invention will employ, unless otherwise indicated, conventional techniques which are within the skill of the art. Such techniques are explained fully in the literature.

The invention will now be exemplified with the following examples, which are not limitative, with reference to the attached drawings in which:

### FIGURE LEGENDS

**Figure 1****:** Brain MRI of a 21-year-old man with Moissiacense virus encephalitis. Axial A) FLAIR and B) Diffusion-weighted images, reveal subtle hyper intensity of both putamina (thick arrows), frontal, insular and posterior cingulate cortices (thin arrows).

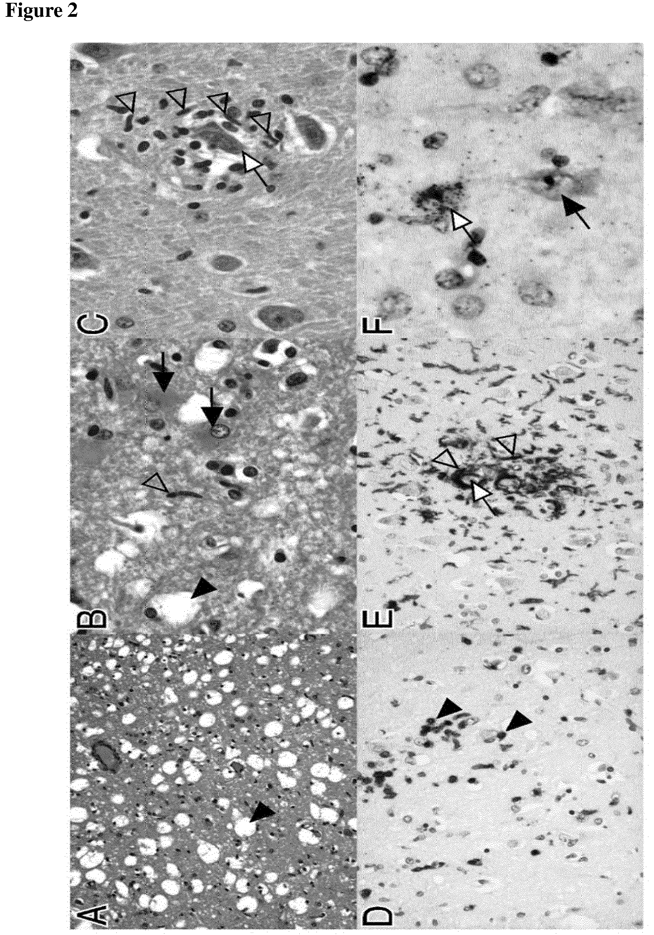

**Figure 2****: A-C,** hematoxylin and eosin staining. The cerebral cortex harbored severe lesions: spongiosis (solid arrowhead in A and B), astrocytic gliosis (solid arrows in B), marked microglial activation (open arrowhead in B), neuronophagia corresponding to a neuron (white arrow in C) surrounded by activated microglial cells (open arrowheads in C). **D,** a CD3 immunostaining highlighted a T cell inflammatory infiltrate. **E,** an IBA1 immunostaining showed a lesion of neuronophagia with microglial cells (open arrowheads) surrounding a neuron (white arrow). **F,** red signal of the Moissiacense virus ISH probe is observed in a pyramidal neuron of the cerebral cortex whereas a neighbor neuron is negative (solid arrow).
**Figure 3****:** Phylogenetic analysis. **A**) S-segment, **B**) M-segment, **C**) L-segment and **D**) PCR-targeted region on the L-segment. Moissiacense virus and the closest relative viral sequences are indicated in red. Serogroups are indicated in black on the right side of each panel. The details of the methods used for the phylogenetic analysis and the list of accession numbers are available in the material and methods section.
**Figure 4****:** RT-PCR assay on the patient's brain sample using primer pairs specific for Moissiacense virus species S, L and M segment sequences.
**Figure 5****:** Comparison of multiplex pan-orthobunyaviruses PCR primers from Lambert AJ & Lanciotti RS (J. Clin. Microbiol., 2009, 47, 2398-404) with Moissiacense virus sequence (SEQ ID NO: 74 to 89). Disagreements between the consensus primers and Moissiacense virus sequences are highlighted.

### EXAMPLES

### 1. Material and Methods

### - Metagenomics analysis

Total RNA was extracted from post-mortem brain using a bead based tissue homogenizer (Bertin) and the Trizol procedure (Thermo Fisher Scientific), followed by RNA purification on RNeasy column including a DNAse treatment (Qiagen). NGS library was prepared with the SMARTer Stranded Total RNA-Seq Kit - Pico Input Mammalian (Takara Bio) and sequenced on a NextSeq500 instrument (Illumina), generating approximately 116 million reads. These reads were trimmed with AlienTrimmer (Criscuolo et al., A, Genomics, 2013, 102, 500-6; version 0.4.0, options -k 10 -m 5 -150 -p 80 -c 012 -q 20), and then assembled with MegaHi² (Li et al., Bioinforma Oxf. Engl., 2015, 31, 1674-6; version 1.1.2, with default options). At the end of the assembly step, reads which did not contributed to any contig were kept along with contigs. A search for viral sequences was performed with DIAMOND (Buchfink et al., Nat. Methods, 2015, 12, 59-60) on the protein version of RVDB database (Bigot et al., F1000Research, 2019, 8, 530. Matches with viral sequences were then searched in NCBI NR protein and NCBI NT databases to avoid false positive. This led to the identification of 168 sequences (22 contigs and 146 singletons) belonging to the *Peribunyaviridae* family, covering approximately 55% of the closest reference virus genome represented by Umbre virus. No other viral sequence was identified. A search for bacteria or other non-viral pathogen sequences was performed with Kraken2 (Wood et al., Genome Biol., 2014, 15, R46), Centrifuge (Kim D et al., Genome Res [Internet] 2016 [cited 2018 Nov 30]; Available from: http://genome.cshlp.org/content/early/2016/11/16/gr.210641.116) and Methaphlan2 (Truong et al., Nat. Methods., 2015, 12, 902-3) leading to no other viral or bacterial hit. Full CDS of the S, L and M segments were obtained by a combination of RT-PCR and RACE analysis (S segment (SEQ ID NO: 1); M segment (SEQ ID NO: 3); L segment SEQ ID NO: 5).

### - Phylogenetic analysis

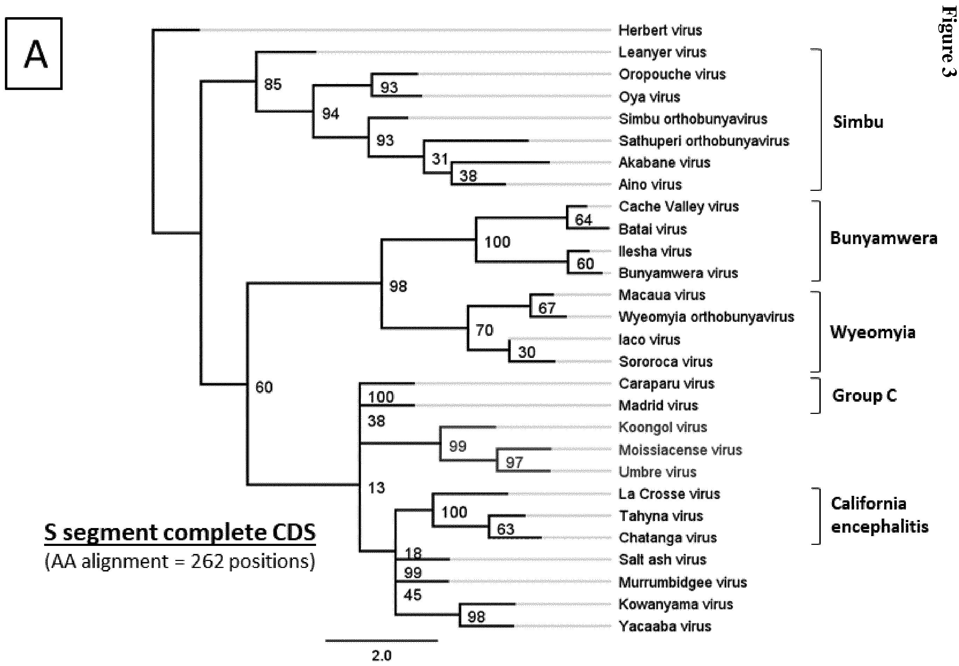

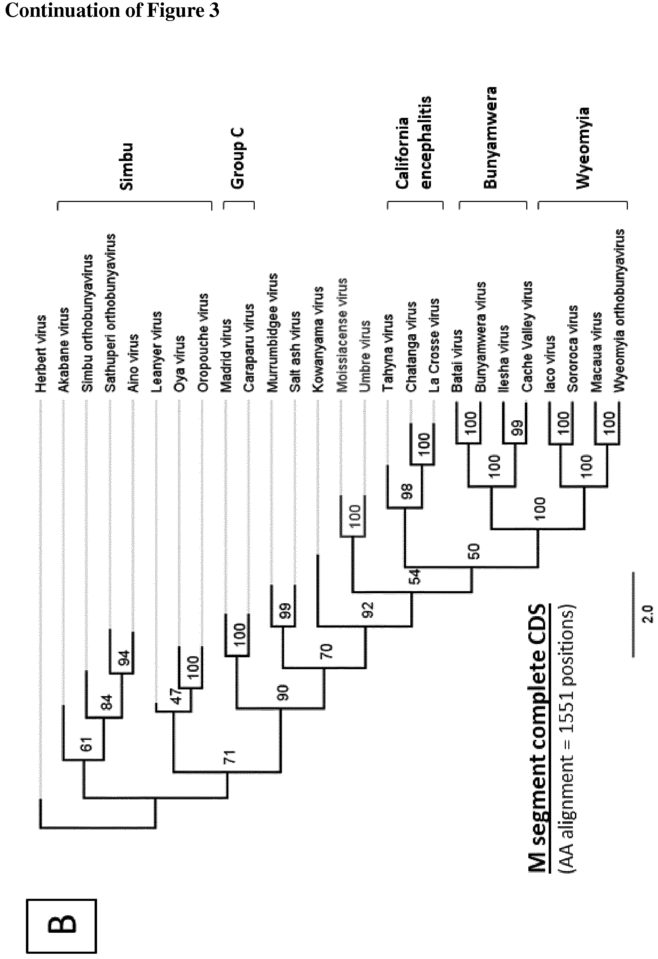

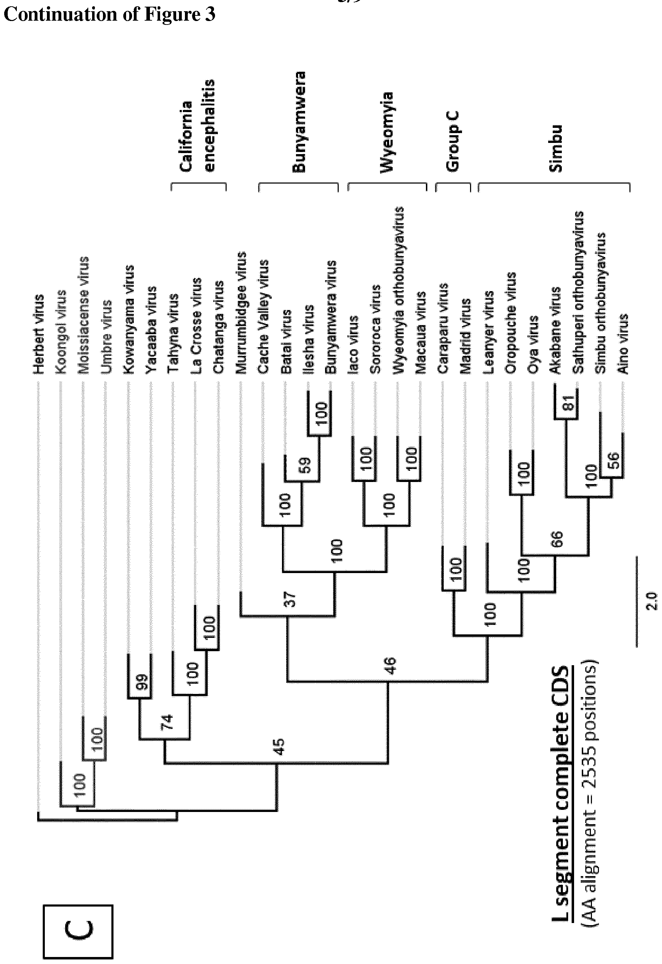

Alignments of amino acids sequences were done with MAFFT v7.388 (Katoh et al., Nucleic Acids Res., 2002, 30, 3059-66; Katoh et al., Mol. Biol. Evol., 2013, 30, 772-80) and manually adjusted when needed. Identity matrices are given in **Tables I to IV.** RAxML phylogeny with 100 bootstrap replications was performed in Geneious 11.1.5 (Kearse et al., Bioinforma Oxf Engl., 2012, 28, 1647-9) after initial model selection done in MEGA7 (Kumar et al., Mol. Biol. Evol., 2016, 33, 1870-4). Only complete CDS sequences were kept for phylogenetic analysis of the S, M and L segments (**Figure 3A****, B, C**). The M segment of Koongol virus was removed from the analysis because of the presence of a large deletion (> 300 AA) in the NSn and Gc part of the sequence, which most probably reflects a cell culture passaging artifact as demonstrated for Maguari orthobunyavirus (Pollitt et al., Virology, 2006, 348, 224-32). The M segment of Yacabba virus, and the L segments of Little Sussex virus and Salt Ash virus, were not included in the analysis because of partial CDS sequences. No sequence was available for Little Sussex virus M and S segments. Herbert virus (Family: *Peribunyaviridae;* Genus: ***Herbevirus***) was used as outgroup. **Accession numbers: S segment:** Aino virus (NC_018460.1), Akabane virus (NC_009896.1), Batai virus (JX846604.1), Bunyamwera virus (NC_001927.1), Cache Valley virus (KC436108.1), Caraparu virus (KF254789.1), Chatanga virus (EU479697.1), Herbert herbevirus (JQ659258.1), Iaco virus (JN572067.1), Ilesha virus (KF234073.1), Koongol virus (KP792667.1), Kowanyama virus (KT820204.1), La Crosse virus (NC_004110.1), Leanyer virus (HM627177.1), Macaua virus (JN572070.1), Madrid virus (NC_034498.1), Murrumbidgee virus (NC_022597.1), Oropouche virus (NC_005777.1), Oya virus (JX983192.1), Salt ash virus (KF234258.1), Sathuperi orthobunyavirus (NC_018462.1), Simbu orthobunyavirus (NC_018477.1), Sororoca virus (JN572073.1), Tahyna virus (HM036217.1), Umbre virus (KP792685.2), Wyeomyia orthobunyavirus (JN572082.1), Yacaaba virus (KT820210.1). **M segment:** Aino virus (NC_018459.1), Akabane virus (NC_009895.1), Batai virus (JX846605.1), Bunyamwera virus (NC_001926.1), Cache Valley virus (KC436107.1), Caraparu virus (KF254788.1), Chatanga virus (EU621834.1), Herbert herbevirus (JQ659257.1), Iaco virus (JN572066.1), Ilesha virus (KF234074.1), Kowanyama virus (KT820203.1), La Crosse virus (NC_004109.1), Leanyer virus (HM627176.1), Macaua virus (JN572069.1), Madrid virus (KF254780.1), Murrumbidgee virus (NC_022596.1), Oropouche virus (NC_005775.1), Oya virus (JX983193.1), Salt ash virus (KF234257.1), Sathuperi orthobunyavirus (NC_018466.1), Simbu orthobunyavirus (NC_018478.1), Sororoca virus (JN572072.1), Tahyna virus (HM036218.1), Umbre virus (KP792686.1), Wyeomyia orthobunyavirus (JN572081.1). **L segment:** Aino virus (NC_018465.1), Akabane virus (NC_009894.1), Batai virus (JX846606.1), Bunyamwera virus (NC_001925.1), Cache Valley virus (KC436106.1), Caraparu virus (KF254793.1), Chatanga virus (EU616903.1), Herbert herbevirus (NC_038714.1), Iaco virus (JN572065.1), Ilesha virus (KF234075.1), Koongol virus (KP792669.1), Kowanyama virus (KT820202.1), La Crosse virus (NC_004108.1), Leanyer virus (HM627178.1), Little Sussex virus (KT720483.1), Macaua virus (JN572068.1), Madrid virus (KF254779.1), Murrumbidgee virus (KF234253.1), Oropouche virus (NC_005776.1), Oya virus (JX983194.1), Salt ash virus (KF234256.1), Sathuperi orthobunyavirus (NC_018461.1), Simbu orthobunyavirus (HE795108.1), Sororoca virus (JN572071.1), Tahyna virus (HM036219.1), Umbre virus (KP792687.1), Wyeomyia orthobunyavirus (JN572080.1), Yacaaba virus (KT820208.1).

**Moissiacense virus:** S segment (SEQ ID NO: 2); M segment (SEQ ID NO: 4); L segment SEQ ID NO: 6.

### - Immunohistochemistry

Formalin-fixed paraffin-embedded tissue sections were cut. Immunostainings were performed in a Ventana Ultra stainer (Roche) with diaminobenzidine as a brown chromogen. Primary antibodies were a rabbit monoclonal anti-CD3 (clone 2GV6, prediluted, Roche) and a polyclonal rabbit anti IBA1 (dilution 1/500, Wako).

### - In-situ hybridization

ViewRNA ISH Tissue Assay Kit 2-plex (Thermo Fisher Scientific) was used with a cocktail of 95 custom bDNA probes targeting 3 segments of Moissiacense virus (15 for S, 40 for M, 40 for L) and labeled in red. A mix of control probes targeting human GAPDH, ACTB and PPI transcripts were labeled in blue.

### - Mosquito capture

Approximately 4,000 *Culex pipiens* female mosquitoes were captured in 2015 in the French Rhône Delta region as described in Eiden et al. 2018 (Eiden M, Gil P, Ziegler U, et al. Infect Genet Evol J Mol Epidemiol Evol Genet Infect Dis 2018;61:151-4) and 133 pools of approximately 30 individuals were made. In addition, around 100 *Culex pipiens,* 30 *Culex Sp.,* 100 *Aedes caspius,* 50 *Aedes detritus,* 10 *Aedes vexans,* 10 *Aedes Sp.,* and 20 *Anopheles* mosquitoes were captured in 2017 in Camargue and Languedoc (Southern France) and species-specific pools of 5-20 individuals were made.

### - RT-PCR

Reverse transcription reactions were performed using the SuperScript IV First-Strand Synthesis System (Thermo Fischer Scientific). PCR were done in SYBR Green format with 45 cycles of amplification. Primer pairs used for detection of Moissiacense virus were: L segment AGAATTGGTTATCCCAGATGAGGT (forward; SEQ ID NO: 14) and GCCATAAATTGAAAATGGTTCTCCA (reverse; SEQ ID NO: 15); M segment ACAGGRCAAATAGCACTAAAGGT (forward; SEQ ID NO: 12) and CCTTCRTCTCTCACTTTGCAG (reverse; SEQ ID NO: 13); S segment AAACGCAGAACTGGGTAGCA (forward; SEQ ID NO: 10) and GAGTTAGCTCATCGTCCGCA (reverse; SEQ ID NO: 11). The primer pair used for detection of the Koongol virus L segment was GACCCAATACTGTAAACAG (forward; SEQ ID NO: 69) and CGTCAGAATGCACCATTG (reverse; SEQ ID NO: 70).

### - CSF analysis

RNA extracts from 193 CSFs collected in patients (attending both University hospitals of Montpellier and Nimes, two towns close to the Camargue region) presenting with a meningitis or meningo-encephalitis during the course of a regional arbovirus surveillance program during the peak mosquito activity period from 1^{st} May to 31 November 2016 and 2017 were tested by RT-PCR using primers specific for the S segment of Moissiacense virus.

### 2. Case report

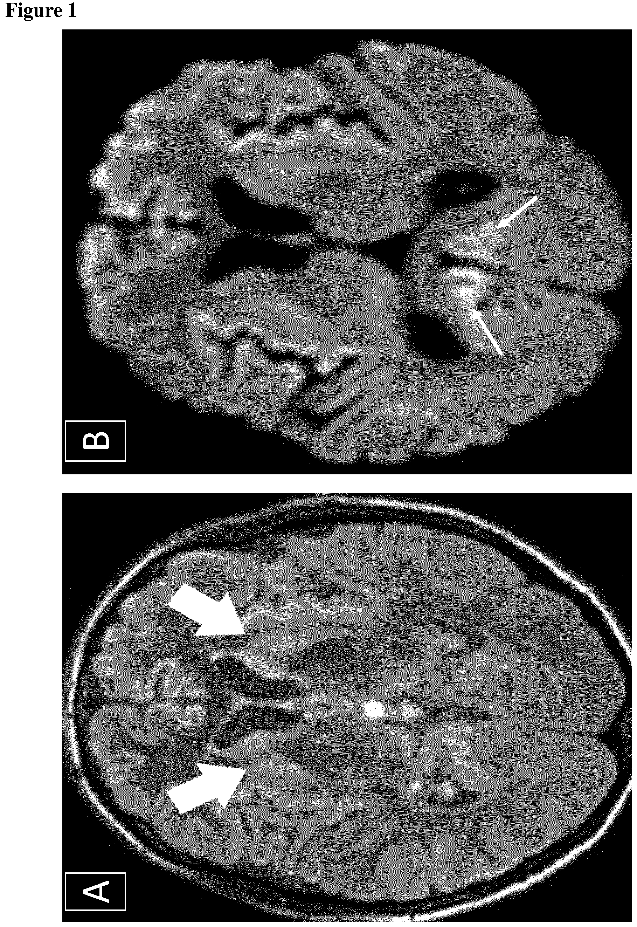

A 21-year-old man was referred to our institution in November 2013 for a catatonic syndrome. His past condition included Bruton's syndrome that has been treated since early childhood by repeated infusion of intravenous immunoglobulins together with an anti-infectious prophylaxis by valaciclovir and sulfamethoxazole/trimethoprim. He also suffered from Crohn's disease, which was treated with mesalazine and azathioprine. The patient made recreational use of cocaine and cannabis. One month ago after a party he quickly developed a syndrome without any clear neurological signs or fever, but lost 10 kg of body weight in a few days. He was first admitted in the department of psychiatry then transferred to neurology after a bulbar syndrome was diagnosed. MRI showed a diffuse cortical, insular and posterior cingulate T2 flair and diffusion hypersignal. High signal intensity was also found in the caudate nucleus and putamen bilaterally (**Figure 1**). Repeated lumbar punctures revealed 0.8 WBCs/mm³. No oligoclonal bands were detected. Serological tests for Borrelia and VDRL, TPHA, and polyomavirus were negative. PCR for enteroviruses, herpesviruses, and Whipple's disease were negative. In serum the following tests were negative or non-informative: HIV, hepatitis B, C, E, thyroglobulin antibody (Ab), Thyroperoxidase Ab, Anti-Ri/La/Hu Abs, anti-VGCC Ab, anti-endomysial Ab and celiac panel, anti-NMDA receptor, anti-GABA, anti-ANNA-1, anti-ANNA-2, anti-gangliosides, anti-AQP4, and anti-MOG. Ionogram, ceruloplasmin and urine copper level were normal. Of note, CRP was increased 30-fold but erythrocyte sedimentation rate was normal. Anti-GAD65 was 6-fold higher, ASAT and ALAT were 2-fold higher. A lymphopenia at 500 G/L was observed. 14-3-3 protein detection was positive. Whole body CT-scan and FDG-PET/CT scan were unremarkable. EEG suggested diffuse encephalopathy without generalized periodic sharp wave pattern. Overall, a Creutzfeldt-Jakob disease (either iatrogenic or variant) was suspected and the patient died in December 2013 despite treatment by ceftriaxone. Postmortem brain examination was performed. On gross examination, the external aspect was unremarkable. Microscopic examination showed marked spongiosis and astrogliosis in several cortical areas (**Figure 2**). Nevertheless, microglial activation was more severe than in most Creutzfeldt-Jakob diseases, forming microglial nodules, and was associated with lymphocytic perivascular cuffs (**Figure 2**). Moreover, PrP 12F10 immunohistochemistry was negative. The diagnosis of Creutzfeldt-Jakob disease was definitively ruled out by the negativity of PrP in Western blot.

### 3. Results

### - Identification and characterization of a novel orthobunyavirus from a brain sample

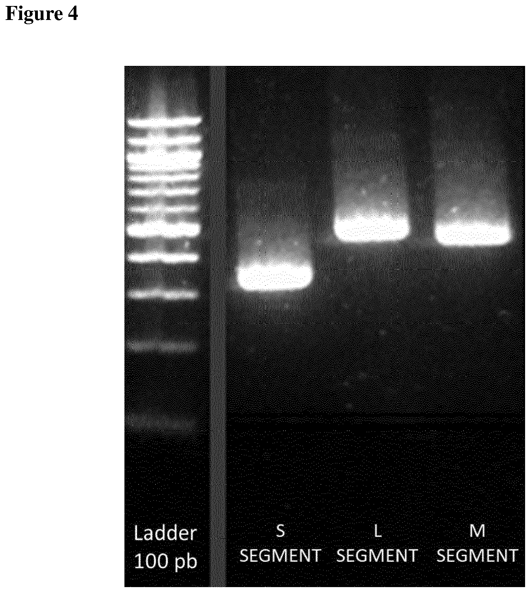

Next-Generation Sequencing identified sequences of an orthobunyavirus in a post-mortem brain sample of a 21-year-old patient suffering from Bruton's disease. The full coding genome was obtained: SEQ ID NO: 5 (L segment), SEQ ID NO: 3 (M segment) and SEQ ID NO: 1 (S segment). This novel virus, named Moissiacense virus in reference to the French region where the patient was living, is a member of a currently unnamed clade or serogroup (**Figure 3A****, B, C**), and is closely related to Umbre virus (97.6% amino acid sequence identity (AA Id) and 88% nucleotide sequence identity (nt Id) for L segment; 94.3% AA Id and 86% nt Id for M segment; 97.5% AA Id and 92% nt Id for S segment) and to a lesser extend to Koongol virus (70.4% AA Id and 68% nt Id for L segment; 55.0% AA Id and 66% nt Id for S segment; no data for M segment) (**Tables I to III**). Moissiacense virus also appears to be very closely related to Little Sussex virus (100.0% AA Id for the 96 AA PCR amplicon on L segment; 88% nt Id on the 5800 nt partial CDS sequence of L segment (GenBank accession number KT720483.1) (**Table IV**). PCR primer pairs were designed for the specific detection of Moissiacense virus, Umbre virus and Little Sussex virus (Moissiacense virus species-specific primers): SEQ ID NO: 10-11 (S segment); SEQ ID NO: 12-13 (M segment): SEQ ID NO: 14-15 (L segment). Moissiacense virus S, L and M segments were detected by RT-PCR on the patient's brain sample using the Moissiacense virus species-specific primers (**Figure 4**). *C. pipiens* samples containing related virus sequences were negative by PCR with the Moissiacense virus species-specific primer. These results demonstrate the specificity of the designed PCR primers for Moissiacense virus species (Moissiacense virus, Umbre virus and Little Sussex virus). Determination of viral titer in the patient's brain sample by RT-qPCR indicated approximately 10⁷ to 10⁸ genome copies/gram of tissue.

### - Moissiacense virus infects brain neurons

In the absence of commercially available antibody specific to the Moissiacense group of viruses, *in-situ* hybridization probes were designed to detect the S, M and L segments of Moissiacense virus. ISH performed on sections of the patient's cerebral cortex evidenced viral sequences in the cell body and the apical neurite of pyramidal neurons (**Figure 2F**), while no glial cells or lymphocytes were positive for the same sequences. These data indicate that Moissiacense virus infects and replicates in cortical neurons.

### - Detection and prevalence of related viral sequences in Culex pipiens captured in France

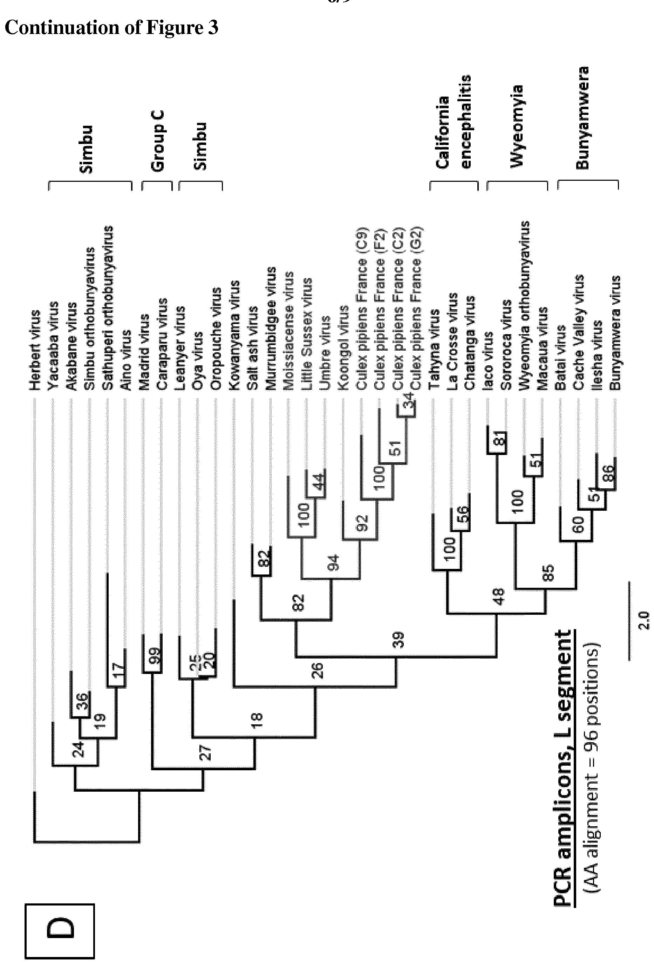

All 133 pools of *Culex pipiens* mosquitoes captured in 2015 in Camargue were negative for PCRs targeting the S, M and L segments of Moissiacense virus. However, 4 out of the 133 pools were positive for Koongol L-segment by RT-PCR (samples C2, C9, F2 and G2). PCR performed after omitting the reverse transcription step was negative, supporting the detection of viral RNA rather than endogenous viral DNA. Sanger sequence analysis of the viral amplicons from mosquitoes confirmed the proximity between Koongol virus (82.1-83.2% AA Id), Little Sussex, Umbre and Moissiacense viruses (75.8-76.8% AA Id) (**Figure 3D** **and Table IV**). Of the mosquitoes captured in 2017, none were positive, which might reflect the lower number of mosquitoes tested. Based on the number of positive pools and the size of the pools from the 2015 captures, the prevalence of Koongol-like sequences in *Culex pipiens* was estimated by statistical prediction models to be approximately 0.1% (variances ranging from 10⁻⁷ to 10⁻³ depending on the model used), assuming either unknown or perfect sensitivity and specificity values of the PCR (Cowling et al., Prev. Vet. Med., 1999, 39, 211-25).

### - Absence of detection of Moissiacense virus in CSF

Of the 193 CSFs from meningo-encephalitis cases tested by RT-PCR, none was positive for the Moissiacense S segment. CSF from the index patient was not available for testing.

### 4. Discussion

The inventors have used untargeted metagenomics to search for pathogens in a fatal case of encephalitis of unknown origin. This approach led to the identification and the characterization of Moissiacense virus, a novel orthobunyavirus related to viruses of the Koongol group. The amount of Moissiacense virus genomic sequences in the brain biopsy was very high (around 10⁸ genome copies/gram) and no other sequence related to virus or bacteria was found by NGS. *In-situ* hybridization demonstrated that the virus infected neuronal cells, supporting the role of Moissiacense virus as the cause of encephalitis for this patient.

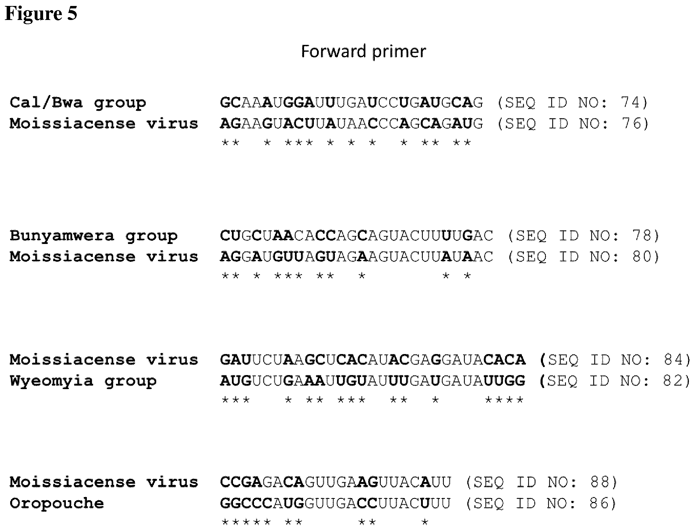

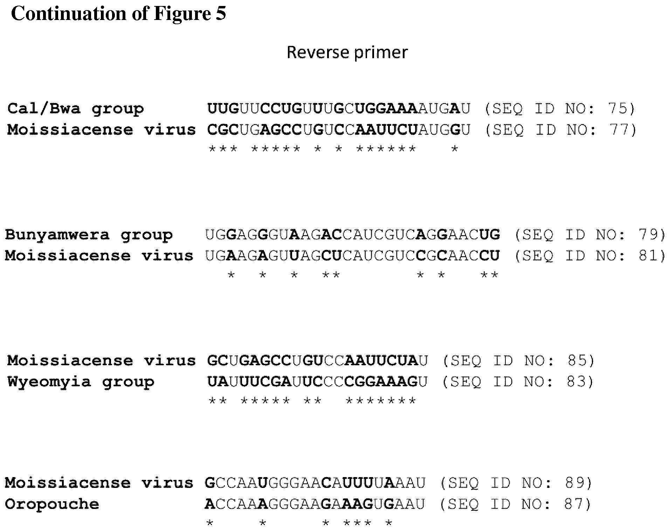

The work is original because it deals with the damaged brain tissue obtained from a brain biopsy or autopsy and analyzed by confirmed neuropathologists and the analysis is done without a priori, by methods of very deep sequencing (High Troughput sequencing or NGS), followed by a comparison of the sequences with viral and general nucleotide and protein data bases. The originality of the work is strengthened by the fact that PCR consensus assays targeting a broad range of orthobunyaviruses (Lambert AJ, Lanciotti RS, J. Clin. Microbiol., 2009, 47, 2398-404) would have missed Moissiacense virus due to sequence variation (**Figure 5**).

Moissiacense virus is related to Umbre virus, Little Sussex virus and Koongol virus. Within the genus *Orthobunyavirus,* these viruses belong to the Koongol group of viruses, a group that is yet to be classified by the ICTV but that is distinct from other *Orthobunyavirus* groups such as the California encephalitis, Bunyamwera, Simbu and Wyeomyia groups. Within the order *Bunyavirales,* members of the California serogroup (genus *Orthobunyavirus,* family *Peribunyaviridae*) and the Rift Valley Fever virus (genus *Phlebovirus,* family *Phenuiviridae*) can be responsible for human encephalitis. Regarding viruses related to the Koongol group, however, their pathogenic potential remains unknown.

Based on the species demarcation criteria of the International Committee on Taxonomy of Viruses (ICTV) for orthobunyaviruses, Moissiacense virus, Umbre virus and Little Sussex virus would belong to the same species (>90% identical AA identity; ICTV report, 30 Nov 2018 (available from: https://talk.ictvonline.org/ictv-reports/ictv_9th_report/). Previously, Umbre, Little Sussex and Koongol viruses have been identified in *Culex sp.* in India, Australia and New Guinea (Dandawate et al., Indian J. Med. Res., 1969, 57, 1420-6; Doherty et al., Aust. J. Exp. Biol. Med. Sci., 1979, 57, 509-20; Doherty et al., Aust. J. Exp. Biol. Med. Sci., 1963, 41, 17-39; Karabatsos, edited by N. International catalogue of arboviruses, including certain other viruses of vertebrates [Internet]. 3rd ed. San Antonio, Texas: American Society of Tropical Medicine and Hygiene for The Subcommittee on Information Exchange of the American Committee on Arthropod-borne Viruses; 1985. Available from: https://catalyst.library.jhu.edu/catalog/bib_66192).

Serological surveys based on hemagglutination assay have suggested that members of the Koongol group could infect several mammals, but this has not been confirmed with a more specific technique such as seroneutralization (Shchetinin et al., Viruses, 2015, 7, 5987-6008).

Thus, to the knowledge of the Inventors, this is the first time that infection of a vertebrate has been shown for a virus from the Koongol group, in a European country. Viral sequences of the Koongol group were found in *Culex pipiens* mosquitoes captured in Southern France, which suggests that a novel arbovirus clade with neurotropic potential is circulating in Europe.

Moissiacense virus was lethal in an immunocompromised patient who had never traveled abroad and can be viewed as a sentinel. However, its clinical presentation in the general population may range, as for many other arboviruses, from asymptomatic or mild infectious syndromes to various neurological disorders including milder encephalitis, as for West Nile virus or Eastern equine encephalitis virus (Salimi et al., J. Am. Soc. Exp. Neurother., 2016, 13, 514-34).

In an attempt to diagnose other Moissiacense virus infection, the inventors have analyzed 193 CSFs taken from suspicious cases of meningo-encephalitis in South of France. No virus was detected. This is nevertheless not fully informative as, unfortunately, no CSF from the index patient was available for testing. It is indeed well known that other major arboviruses, like JEV, are hardly detectable in the CSF after the onset of the disease (Dubot-Pérès et al., Lancet Infect. Dis., 2015, 15, 1376-7; Touch et al., Trop. Med. Int. Health TM IH, 2009, 14, 1365-73). In line with this, negative PCR-testing of the CSF was also recorded for Cache Valley virus, another orthobunyavirus identified recently in the brain of an immunodeficient patient (Wilson et al., Ann. Neurol., 2017, 82, 105-14).

Diagnosis of orthobunyavirus encephalitis is often based on antibody testing (Miller A et al., Hosp. Pediatr., 2012, 2, 235-242). Serological assays based on the Moissiacense virus antigen(s) disclosed in the present application would provide insight into the seroprevalence and expected burden of this new virus in human.

In conclusion, the convergence of virological and epidemiological data shows that the inventors have identified a new neurotropic arbovirus in Europe, belonging to the orthobunyaviruses. Another close species was identified in *Culex pipiens* mosquitoes from the same region. Thus, members of the Moissiacense group of viruses, in particular Moissiacense virus, should be added to targeted tests in routine diagnosis of brain tissue from encephalitis cases with unidentified etiology.

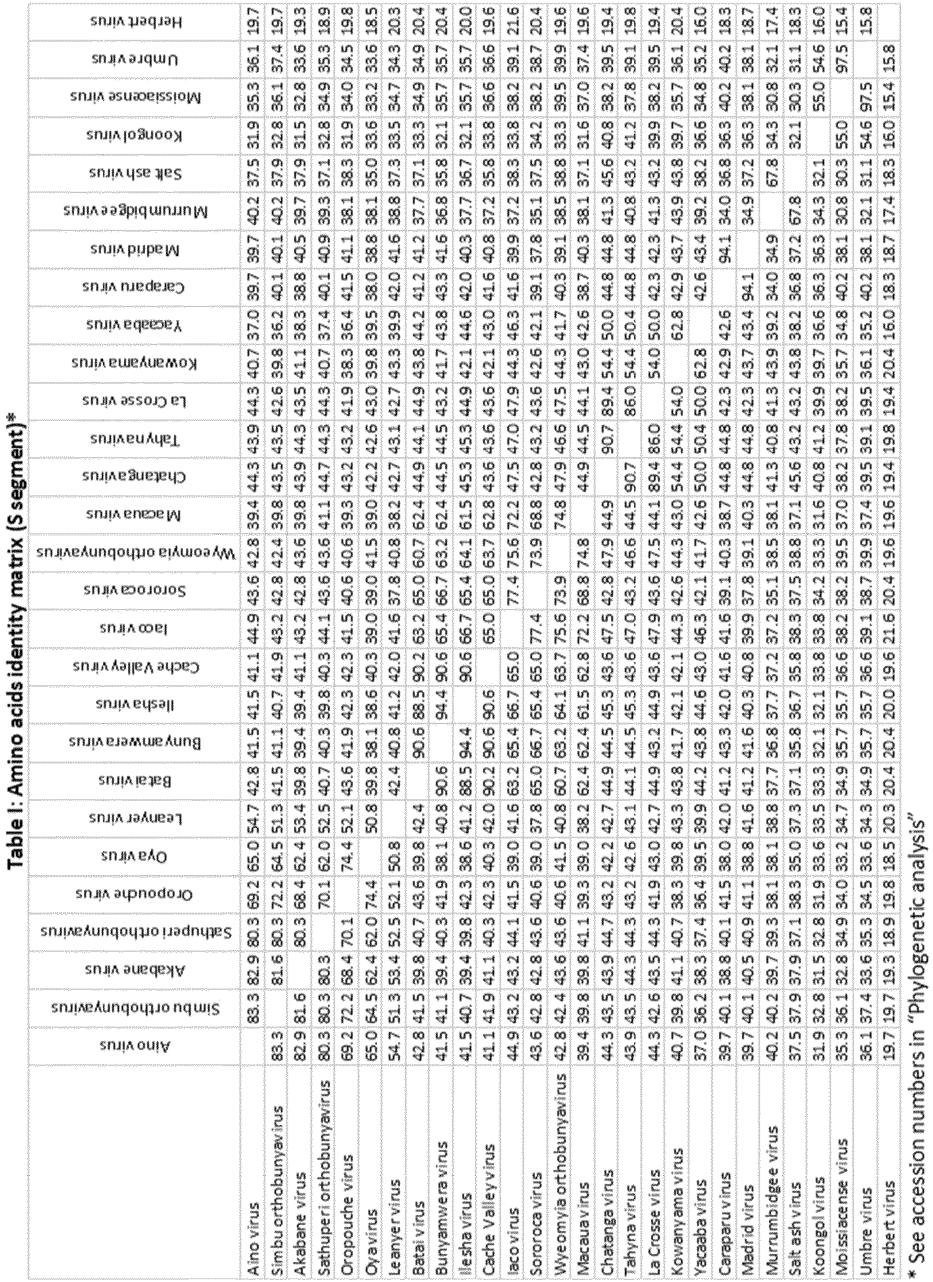

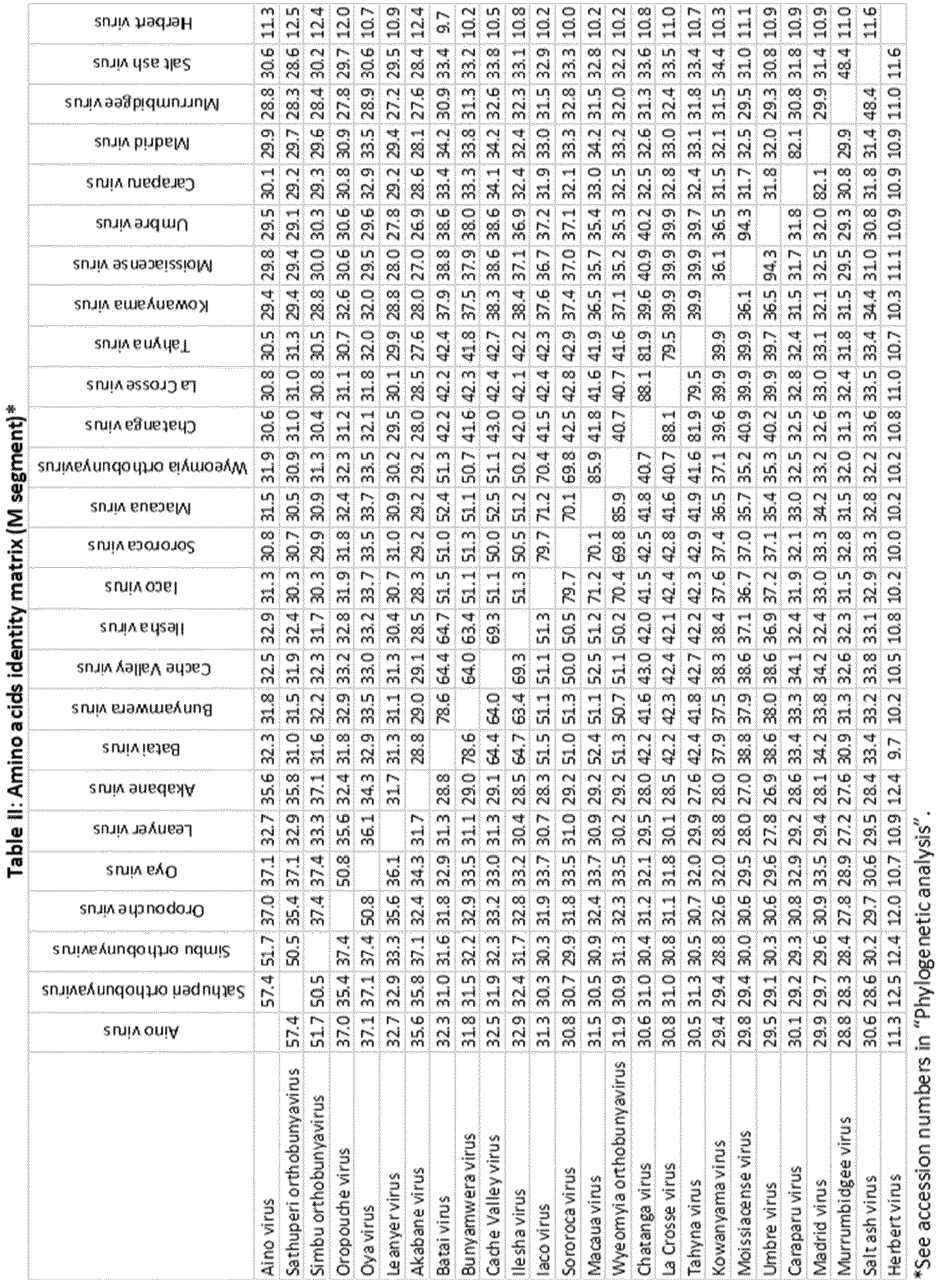

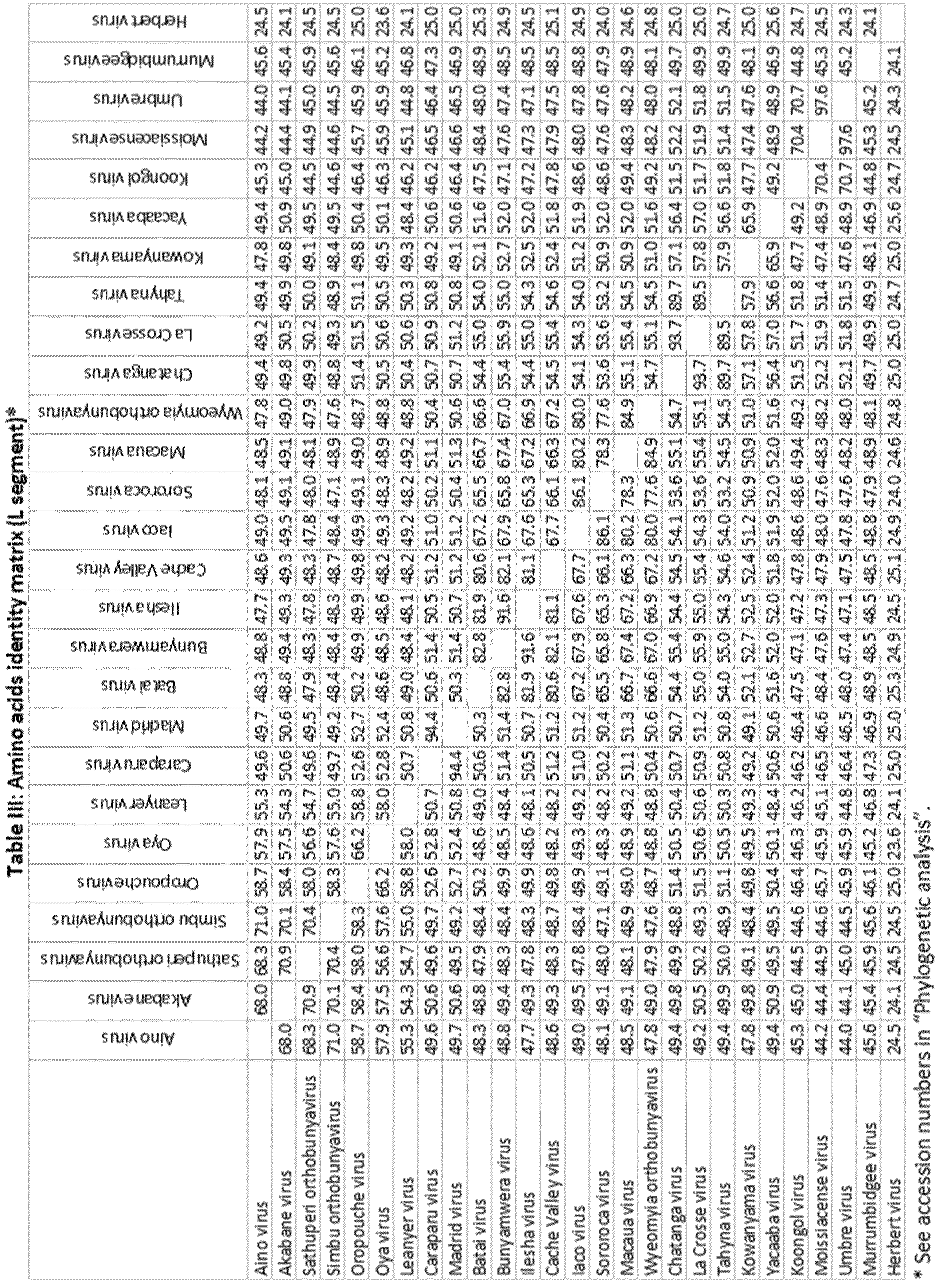

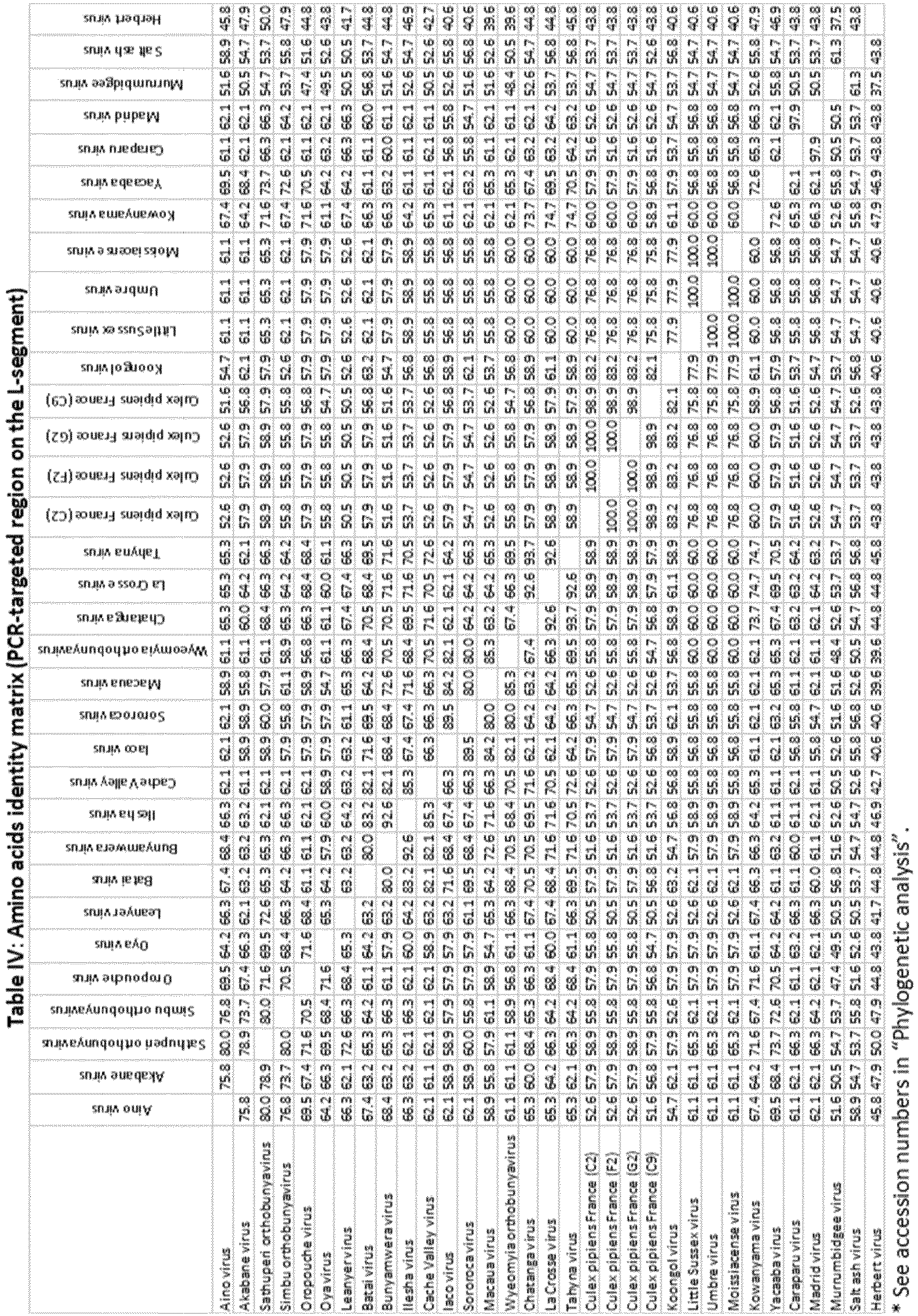

## Claims

1. A method of diagnosis of Moissiacense virus infection in a sample from an individual, comprising determining the presence of at least one viral nucleic acid selected from the group consisting of:
- a first nucleic acid (S segment) comprising a sequence having at least 80% identity with SEQ ID NO: 1 and further including a coding sequence for a Nucleocapsid (N) protein having at least 90% identity with SEQ ID NO: 2, or its complement;
- a second nucleic acid (M segment) comprising a sequence having at least 80% identity with SEQ ID NO: 3 and further including a coding sequence for a polyprotein M having at least 90% identity with SEQ ID NO: 4; or its complement; and
- a third nucleic acid (L segment) comprising a sequence having at least 80% identity with SEQ ID NO: 5 and further including a coding sequence for a polymerase (L) protein having at least 90% identity with SEQ ID NO: 6 including at least 90% identity on the core polymerase domain, or its complement.

2. The method according to claim 1, wherein said Moissiacense virus comprises a S segment sequence of SEQ ID NO: 1 coding for a nucleocapsid (N) protein of SEQ ID NO: 2, a M segment sequence of SEQ ID NO: 3 coding for a polyprotein M of SEQ ID NO: 4 and a L segment sequence of SEQ ID NO: 5 coding for a polymerase (L) protein of SEQ ID NO: 6.

3. The method according to claim 1 or 2, which comprises the detection of at least one specific target sequence of the viral nucleic acid by a process of hybridization with an oligonucleotide probe, amplification with a pair of oligonucleotide primers, sequencing, or a combination thereof.

4. The method according to claim 3, wherein the at least one target sequence is chosen from the sequences SEQ ID NO: 7, 8, 9; the sequences of at least 15 nucleotides from any one of SEQ ID NO: 7 to 9, and the sequences comprising any one of SEQ ID NO: 7 to 9 and up to 40 consecutive nucleotides of 5' and/or 3' flanking sequence from the S, M or L segment sequence.

5. The method according to claim 3 or 4, wherein the oligonucleotide probe or primers are selected from the group consisting of: the sequences of 5 to 100 nucleotides, preferably 15 to 60 nucleotides having 80% to 100% identity with any of SEQ ID NO: 1, 3, 5, and 7 to 9, which are specific for Moissiacense virus; and their complement.

6. The method according to claim 5, wherein the oligonucleotide probe is selected from the group consisting of the sequences SEQ ID NO: 10 to 57 or the pair of oligonucleotide primers is selected from the group consisting of: SEQ ID NO: 10-11, 12-13, 14-15, 16-17, 18-19, 20-21, 22-23, 24-25, 26-27, 28-29, 30-31, 32-33, 34-35, 36-37, 38-39, 40-41, 42-43, 44-45, 46-47, 48-49, 50-51, 52-53, 54-55, and 56-57.

7. A method of diagnosis of Moissiacense virus infection in a sample from an individual, comprising:
- incubating an antigen from Moissiacense virus or antibody thereto with the sample to form a mixture; and
- detecting antigen-antibody complexes in the mixture.

8. The method according to claim 7, wherein the antigen from Moissiacense virus comprises an amino acid sequence selected from the group consisting of:
- a sequence having 90 to 100% with SEQ ID NO: 2 or a fragment thereof;
- a sequence having 90 to 100% identity with SEQ ID NO: 58 or 60 or a fragment thereof such as an extracellular fragment from the sequence SEQ ID NO: 58, preferably chosen from the sequences SEQ ID NO: 61 to 64.

9. The method according to claim 7 or 8, which comprises an immunoprecipitation, ELISA or immunohistochemistry assay.

10. The method according to any one of claims 1 to 9, wherein the sample is brain biopsy, cerebral spinal fluid, whole-blood, plasma or serum.

11. The method according to any one of claims 1 to 10, wherein the same biological sample from the individual is subjected to a method of diagnosis of at least another encephalitis virus such as herpesvirus, enterovirus, polyomavirus, astrovirus, measles virus, mumps virus, or arbovirus.

12. The method according to any one of claims 1 to 11, which is for the diagnosis of encephalitis, in particular in humans, specifically for the differential diagnosis of human encephalitis.

13. A kit for the diagnosis or detection of Moissiacense virus, comprising at least one oligonucleotide probe or primer as defined in claim 5 or 6, an antigen as defined in claim 8 or an antibody thereto, preferably labelled, and eventually further comprising at least one oligonucleotide probe or primer, antigen or antibody thereto for the detection of another encephalitis virus such as herpesvirus, enterovirus, polyomavirus, astrovirus, measles virus, mumps virus, or arbovirus.

14. Use of the kit according to claim 13 for the detection of Moissiacense virus in arthropod vector population for epidemiological survey.

15. An immunogenic or vaccine pharmaceutical composition comprising, as active substance an antigen from Moissiacense virus as defined in claim 8, in association with at least one pharmaceutically acceptable vehicle, adjuvant and/or carrier.

## Patentansprüche

1. Verfahren zur Diagnose von Moissiacense-Virusinfektion in einer Probe eines Individuums umfassend, Bestimmen der Anwesenheit mindestens einer viralen Nukleinsäure ausgewählt aus der Gruppe bestehend aus:
- einer ersten Nukleinsäure (S-Segment) umfassend eine Sequenz mit mindestens 80 % Identität zu SEQ ID NR.: 1 und ferner umfassend eine kodierende Sequenz für ein Nukleocapsid (N) Protein mit mindestens 90 % Identität zu SEQ ID NR.: 2 oder dessen Komplement;
- einer zweiten Nukleinsäure (M-Segment) umfassend eine Sequenz mit mindestens 80 % Identität zu SEQ ID NR.: 3 und ferner umfassend eine kodierende Sequenz für ein Polyprotein M mit mindestens 90 % Identität zu SEQ ID NR.: 4 oder dessen Komplement;
und
- einer dritten Nukleinsäure (L-Segment) umfassend eine Sequenz mit mindestens 80 % Identität zu SEQ ID NR.: 5 und ferner umfassend eine kodierende Sequenz für ein Polymerase (L) Protein mit mindestens 90 % Identität zu SEQ ID NR.: 6 umfassend mindestens 90 % Identität auf der Kernpolymerase Domäne oder dessen Komplement.

2. Verfahren nach Anspruch 1, wobei das Moissiacensevirus eine S-Segment Sequenz von SEQ ID NR.: 1, die für ein Nukleocapsid (N) Protein der SEQ ID NR.: 2 kodiert, eine M-Segment Sequenz der SEQ ID NR.: 3, die für ein Polyprotein M der SEQ ID NR.: 4 kodiert und eine L-Segment Sequenz der SEQ ID NR.: 5, die für ein Polymerase (L) Protein der SEQ ID NR.: 6 kodiert, umfasst.

3. Verfahren nach Anspruch 1 oder 2, welches die Detektion von mindestens einer spezifischen Targetsequenz der viralen Nukleinsäure umfasst, durch ein Verfahren der Hybridisierung mit einer Oligonukleotidsonde, Amplifizierung mit einem Paar von Oligonukleotidprimern, Sequenzieren, oder einer Kombination davon.

4. Verfahren nach Anspruch 3, wobei die mindestens eine Targetsequenz ausgewählt ist aus den Sequenzen SEQ ID NR.: 7, 8, 9; den Sequenzen von mindestens 15 Nukleotiden von einer der SEQ ID NR.: 7 bis 9, und den Sequenzen umfassend eine der SEQ ID NR.: 7 bis 9 und bis zu 40 aufeinanderfolgende Nukleotide der 5' oder 3' flankierenden Sequenz der S, M oder L Segmentsequenz.

5. Verfahren nach Anspruch 3 oder 4, wobei die Oligonukleotidsonde oder die Primer ausgewählt sind aus der Gruppe bestehend aus: den Sequenzen von 5-100 Nukleotiden, bevorzugt 15-60 Nukleotiden, mit 80 %-100 % Identität zu einer der SEQ ID NR.: 1, 3, 5 und 7 bis 9, die spezifisch für das Moissiacensevirus sind, und deren Komplement.

6. Verfahren nach Anspruch 5, wobei die Oligonukleotidsonde ausgewählt ist aus der Gruppe bestehend aus den Sequenzen SEQ ID NR.: 10 bis 57 oder das Paar der Oligonukleotidprimer ausgewählt ist aus der Gruppe bestehend aus: SEQ ID NR.: 10-11, 12-13, 14-15, 16-17, 18-19, 20-21, 22-23, 24-25, 26-27, 28-29, 30-31, 32-33, 34-35, 36-37, 38-39, 40-41, 42-43, 44-45, 46-47, 48-49, 50-51, 52-53, 54-55 und 56-57.

7. Verfahren zur Diagnose einer Moissiacensevirus-Infektion in einer Probe von einem Individuum umfassend:
- Inkubieren eines Antigens eines Moissiacensevirus oder eines Antikörpers dazu mit der Probe um eine Mischung zu bilden; und
- Detektieren von Antigen-Antikörper Komplexen in der Mischung.

8. Verfahren nach Anspruch 7, wobei das Antigen von Moissiacensevirus eine Aminosäuresequenz umfasst, ausgewählt aus der Gruppe bestehend aus:
- einer Sequenz mit 90 bis 100 % Identität zu SEQ ID NR.: 2 oder einem Fragment davon;
- einer Sequenz mit 90 bis 100 % Identität zu SEQ ID NR.: 58 oder 60 oder einem Fragment davon, wie z.B. ein extrazelluläres Fragment der Sequenz SEQ ID NR. 58, bevorzugt ausgewählt aus den Sequenzen SEQ ID NR.: 61 bis 64.

9. Verfahren nach Anspruch 7 oder 8, welches Immunpräzipitation, ELISA oder immunhistochemischen Assay umfasst.

10. Verfahren nach einem der Ansprüche 1-9, wobei die Probe Biopsie aus Hirn, Cerebrospinalflüssigkeit, Vollblut, Plasma oder Serum ist.

11. Verfahren nach einem der Ansprüche 1-10, wobei die gleiche biologische Probe des Individuums einem Verfahren zur Diagnose mindestens eines anderen Encephalitisvirus, wie z.B. Herpesvirus, Enterovirus, Polyomavirus, Astrovirus, Masernvirus, Mumpsvirus oder Arbovirus unterworfen wird.

12. Verfahren nach einem der Ansprüche 1-11, welches für die Diagnose von Encephalitis, insbesondere bei Menschen, insbesondere für die Differentialdiagnose humaner Encephalitis ist.

13. Kit für die Diagnose oder Detektion des Moissiacensevirus, umfassend mindestens eine Oligonukleotidsonde oder einen Primer wie in Anspruch 5 oder 6 definiert, ein Antigen wie in Anspruch 8 definiert oder einen Antikörper dazu, bevorzugt markiert und ggf. ferner umfassend mindestens eine Oligonukleotidsonde oder einen Primer, Antigen oder Antikörper dazu zur Detektion eines anderen Encephalitisvirus, wie z.B. Herpesvirus, Enterovirus, Polyomavirus, Astrovirus, Masernvirus, Mumpsvirus oder Arbovirus.

14. Verwendung des Kits gemäß Anspruch 13 zur Detektion von Moissiacensevirus in arthropoder Vektorpopulation für epidemiologische Untersuchung.

15. Immunogene pharmazeutische Zusammensetzung oder pharmazeutische Vakzin-Zusammensetzung umfassend als aktive Substanz ein Antigen des Moissiacensevirus wie in Anspruch 8 definiert in Verbindung mit mindestens einem pharmazeutisch aktzeptablen Vehikel, Hilfsmittel und/oder Träger.

## Revendications

1. Procédé de diagnostic d'une infection par le virus Moissiacense dans un échantillon provenant d'un individu, comprenant la détermination de la présence d'au moins un acide nucléique viral sélectionné dans le groupe constitué par :
- un premier acide nucléique (segment S) comprenant une séquence ayant une identité d'au moins 80 % avec SEQ ID NO : 1, et comportant en outre une séquence codante pour une protéine de nucléocapside (N) ayant une identité d'au moins 90 % avec SEQ ID NO : 2, ou son complément ;
- un deuxième acide nucléique (segment M) comprenant une séquence ayant une identité d'au moins 80 % avec SEQ ID NO : 3, et comportant en outre une séquence codante pour une polyprotéine M ayant une identité d'au moins 90 % avec SEQ ID NO : 4, ou son complément ; et
- un troisième acide nucléique (segment L) comprenant une séquence ayant une identité d'au moins 80 % avec SEQ ID NO : 5, et comportant en outre une séquence codante pour une protéine polymérase (L) ayant une identité d'au moins 90 % avec SEQ ID NO : 6 comportant une identité d'au moins 90 % sur le domaine polymérase central, ou son complément.

2. Procédé selon la revendication 1, dans lequel ledit virus Moissiacense comprend une séquence de segment S de SEQ ID NO : 1 codant pour une protéine de nucléocapside (N) de SEQ ID NO : 2, une séquence de segment M de SEQ ID NO : 3 codant pour une polyprotéine M de SEQ ID NO : 4 et une séquence de segment L de SEQ ID NO : 5 codant pour une protéine polymérase (L) de SEQ ID NO : 6.

3. Procédé selon la revendication 1 ou 2, qui comprend la détection d'au moins une séquence cible spécifique de l'acide nucléique viral par un processus d'hybridation avec une sonde oligonucléotidique, d'amplification avec une paire d'amorces oligonucléotidiques, de séquençage, ou une combinaison de ceux-ci.

4. Procédé selon la revendication 3, dans lequel l'au moins une séquence cible est choisie parmi les séquences SEQ ID NO : 7, 8, 9 ; les séquences d'au moins 15 nucléotides de l'une quelconque des SEQ ID NO : 7 à 9, et les séquences comprenant l'une quelconque des SEQ ID NO : 7 à 9 et jusqu'à 40 nucléotides consécutifs de séquence flanquante 5' et/ou 3' de la séquence de segment S, M ou L.

5. Procédé selon la revendication 3 ou 4, dans lequel la sonde ou les amorces oligonucléotidiques sont sélectionnées dans le groupe constitué par : les séquences de 5 à 100 nucléotides, de préférence de 15 à 60 nucléotides ayant une identité de 80 % à 100 % avec l'une quelconque des SEQ ID NO : 1, 3, 5, et 7 à 9, qui sont spécifiques du virus Moissiacense ; et leur complément.

6. Procédé selon la revendication 5, dans lequel la sonde oligonucléotidique est sélectionnée dans le groupe constitué par les séquences SEQ ID NO : 10 à 57, ou la paire d'amorces oligonucléotidiques est sélectionnée dans le groupe constitué par : SEQ ID NO : 10-11, 12-13, 14-15, 16-17, 18-19, 20-21, 22-23, 24-25, 26-27, 28-29, 30-31, 32-33, 34-35, 36-37, 38-39, 40-41, 42-43, 44-45, 46-47, 48-49, 50-51, 52-53, 54-55, et 56-57.

7. Procédé de diagnostic d'une infection par le virus Moissiacense dans un échantillon provenant d'un individu, comprenant :
- l'incubation d'un antigène provenant du virus Moissiacense ou d'un anticorps de celui-ci avec l'échantillon pour former un mélange ; et
- la détection de complexes antigène-anticorps dans le mélange.

8. Procédé selon la revendication 7, dans lequel l'antigène provenant du virus Moissiacense comprend une séquence d'acides aminés sélectionnée dans le groupe constitué par :
- une séquence ayant 90 à 100 % d'identité avec SEQ ID NO : 2 ou un fragment de celle-ci ;
- une séquence ayant 90 à 100 % d'identité avec SEQ ID NO : 58 ou 60 ou un fragment de celle-ci, tel qu'un fragment extracellulaire de la séquence SEQ ID NO : 58, de préférence choisi parmi les séquences SEQ ID NO : 61 à 64.

9. Procédé selon la revendication 7 ou 8, qui comprend un test d'immunoprécipitation, ELISA ou d'immunohistochimie.

10. Procédé selon l'une quelconque des revendications 1 à 9, dans lequel l'échantillon est une biopsie de cerveau, du liquide céphalo-rachidien, du sang total, du plasma ou du sérum.

11. Procédé selon l'une quelconque des revendications 1 à 10, dans lequel le même échantillon biologique provenant de l'individu est soumis à un procédé de diagnostic d'au moins un autre virus d'encéphalite tel que l'herpèsvirus, l'entérovirus, le polyomavirus, l'astrovirus, le virus de la rougeole, le virus des oreillons, ou l'arbovirus.

12. Procédé selon l'une quelconque des revendications 1 à 11, qui est destiné au diagnostic de l'encéphalite, en particulier chez l'homme, spécifiquement pour le diagnostic différentiel de l'encéphalite humaine.

13. Kit pour le diagnostic ou la détection du virus Moissiacense, comprenant au moins une sonde ou amorce oligonucléotidique selon la revendication 5 ou 6, un antigène selon la revendication 8 ou un anticorps de celui-ci, de préférence marqué, et éventuellement comprenant en outre au moins une sonde ou amorce oligonucléotidique, un antigène ou un anticorps de celui-ci pour la détection d'un autre virus d'encéphalite tel que l'herpèsvirus, l'entérovirus, le polyomavirus, l'astrovirus, le virus de la rougeole, le virus des oreillons, ou l'arbovirus.

14. Utilisation du kit selon la revendication 13 pour la détection du virus Moissiacense dans une population de vecteurs arthropodes pour une étude épidémiologique.

15. Composition pharmaceutique immunogène ou vaccinale comprenant, en tant que substance active, un antigène provenant du virus Moissiacense tel que défini dans la revendication 8, en association avec au moins un véhicule, adjuvant et/ou support pharmaceutiquement acceptable.
